# EUROPÄISCHE PATENTSCHRIFT

(11) **EP 0 968 175 B1**
(45) Veröffentlichungstag und Bekanntmachung des Hinweises auf die Patenterteilung: **12.12.2001**
(21) Anmeldenummer: 98916989.1
(22) Anmeldetag: 18.03.1998
(51) Int. Cl.: C07C 217/94, C07D 209/86, C09K 11/06, H05B 33/14, H01L 51/30

(54) **SPIROVERBINDUNGEN UND DEREN VERWENDUNG**
SPIRO COMPOUNDS AND THE USE THEREOF
COMPOSES SPIRO ET LEUR UTILISATION

(30) Priorität: 20.03.1997 DE 19711714
(43) Veröffentlichungstag der Anmeldung: 05.01.2000
(73) Patentinhaber: Celanese Ventures GmbH, 65926 Frankfurt am Main (DE)
(72) Erfinder: SALBECK, Josef, D-65779 Kelkheim (DE); LUPO, Donald, D-60316 Frankfurt am Main (DE)
(86) Internationale Anmeldenummer: EP9801559
(87) Internationale Veröffentlichungsnummer: WO9842655

(56) Entgegenhaltungen:
- EP-A- 0 333 641
- EP-A- 0 676 461
- EP-A- 0 718 858
- WO-A-97/10617

## Beschreibung

Aufgrund des weltweit steigenden Bedarfs an elektrischer Energie und der begrenzten Vorräte an Kohle, Öl und Gas, die bei ihrer Verbrennung zudem das Treibhausgas CO₂ freisetzen, hat die Erzeugung von elektrischem Strom aus Sonnenlicht in den letzten Jahre erhöhtes Interesse gefunden.

In der EP-A 0 333 641 ist eine photoelektrochemische Zelle beschrieben, die dadurch gekennzeichnet ist, daß sie einen nanoporösen, d. h. eine extrem aufgerauhte und damit vergrößerte Oberfläche aufweisenden Metalloxid-Halbleiter enthält. Der Ladungstransport zwischen Halbleiter/Chromophor-Schicht und Gegenelektrode erfolgt in dieser Zelle durch eine Elektrolytlösung. Obwohl mit solchen Zellen gute Ergebnisse erzielt werden, ist das Eigenschaftsprofil einer solchen Vorrichtung noch deutlich verbesserungsfähig.

Aus der EP-A 0 718 858 ist eine solche Zelle mit einem flüssigkristallinen Ladungstransportmaterial anstelle eines Elektrolyten bekannt. Die erreichten scheinbaren Quantenausbeuten sind aber noch verbesserungsbedürftig.

Es wurde nun überraschend gefunden, daß sich bestimmte Derivate des Spirobifluorens in hervorragender Weise als Ladungstransportmaterial für photovoltaische Zellen eignen.

Einzelne strukturell andersartige Spriobifluorenderivate sind beispielsweise in US-A 5,026,894, J.M. Tour et al. J. Am. Chem. Soc. 112 (1990) 5662 und J.M. Tour et al. Polym. Prepr. (1990) 408 als Verknüpfungselemente für polymere, organische Halbleiter beschrieben und als Materialien für molekulare Elektronik vorgeschlagen.

In der EP-A 0 676 461 ist die Verwendung von Spiroverbindungen der folgenden allgemeinen Formel, 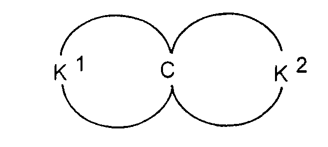 wobei
K¹ und K² unabhängig voneinander konjugierte Systeme bedeuten, in Elektrolumineszenzvorrichtungen, beschreiben.

Eine Anwendung in photovoltaischen Zellen läßt sich daraus nicht herleiten.

Ein Gegenstand der Erfindung sind daher Spiroverbindungen der allgemeinen Formel (I), 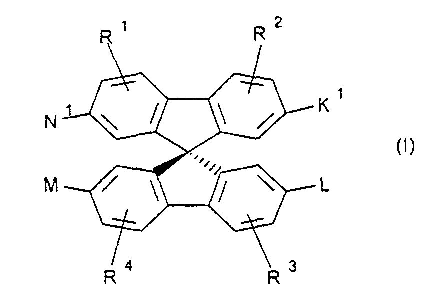 wobei die Symbole folgende Bedeutungen haben:
K¹, L, M, N¹, R¹, R², R³, R⁴ sind gleich oder verschieden und bedeuten
   a) Wasserstoff, -NO₂, -CN, -F oder -Cl,
   b) einen geradkettigen oder verzweigten Alkylrest mit 1 bis 20 C-Atomen, wobei
      b1) eine oder mehrere nicht benachbarte CH₂-Gruppen durch -O-, -S-, -CO-O-, -O-CO-, -O-CO-O-, NR⁵ oder -Si(CH₃)₂- ersetzt sein können und/oder
      b2) eine oder mehrere CH₂-Gruppen durch -CH=CH-, -C≡C-, 1,4-Phenylen, 1,4-Cyclohexylen oder 1,3-Cyclopentylen ersetzt sein können und/oder
      b3) ein oder mehrere H-Atome durch F und/oder Cl ersetzt sein können und/oder
   c) eine der folgenden Gruppen: 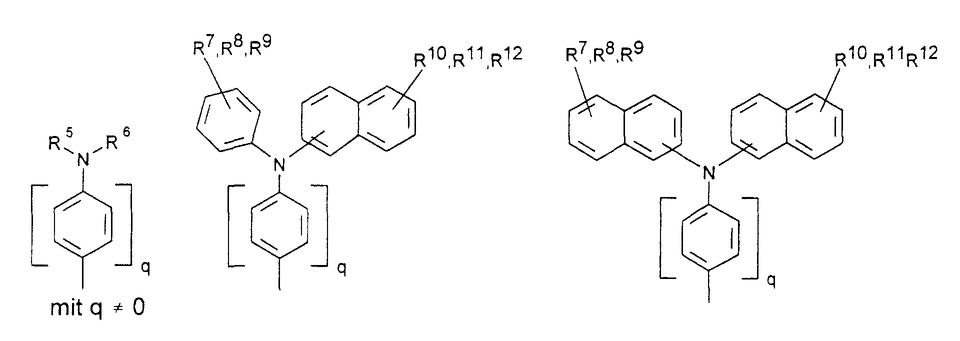 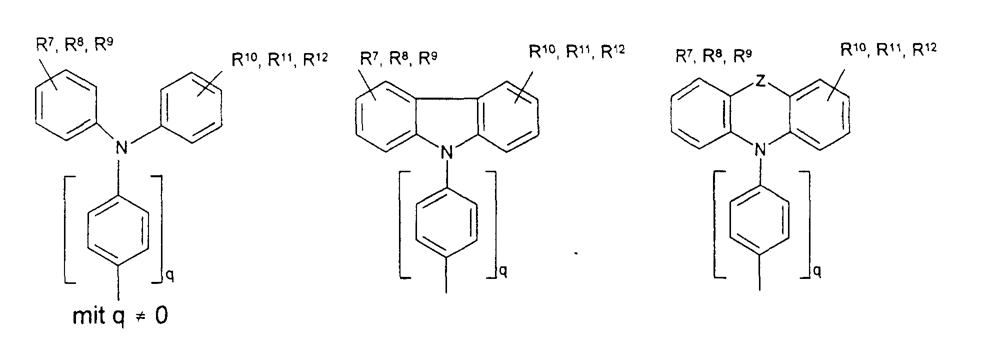 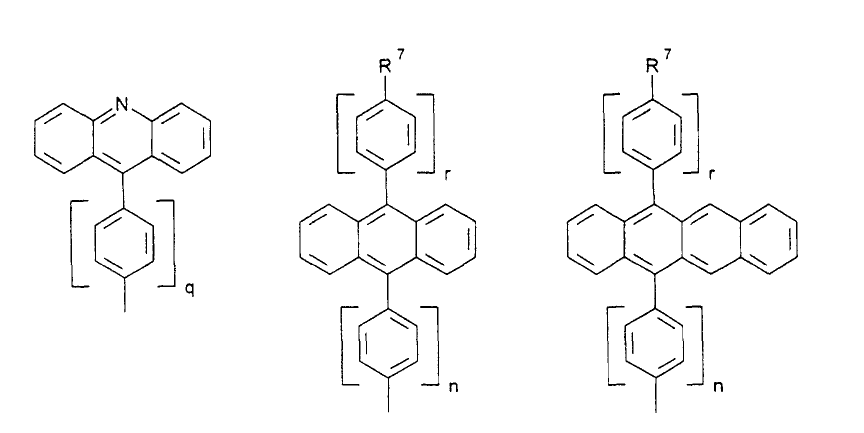 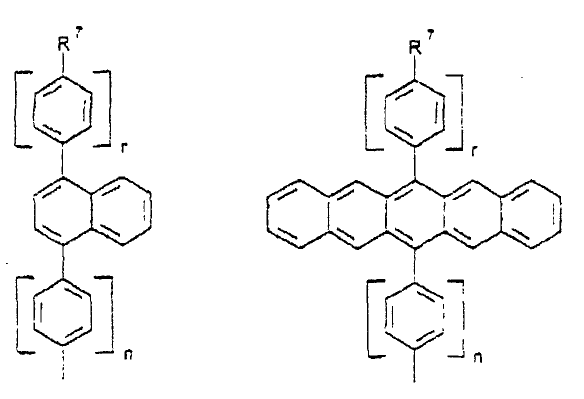 oder
   d) eine der folgenden Gruppen; 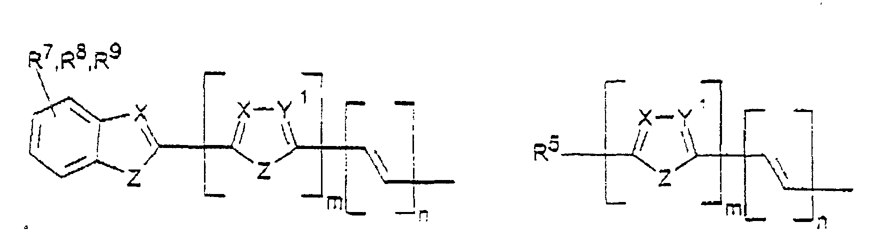 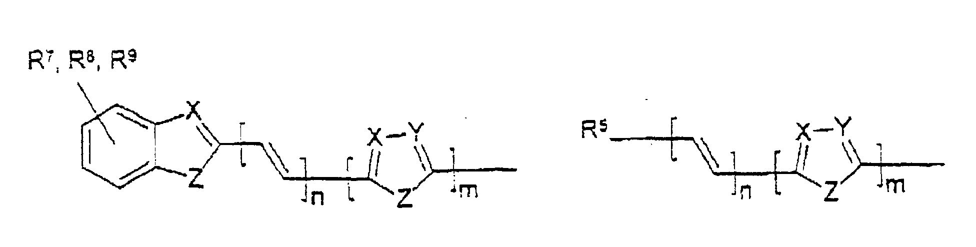 mit der Maßgabe, daß mindestens einer, vorzugsweise mindestens zwei, der Reste K¹, L, M, N¹, R¹, R², R³, R⁴ eine der unter c) aufgeführten Gruppen ist;
X, Y¹ sind jeweils gleich oder verschieden =CR⁷- oder =N-;
Z ist -O-, -S-, -NR⁵-, -CRR-, -CR=CR- oder -CR=N-; R⁵, R⁶ sind jeweils gleich oder verschieden
a) Wasserstoff
b) ein geradkettiger oder verzweigter Alkylrest mit 1 bis 20 C-Atomen, wobei
   b1) eine oder mehrere nicht benachbarte und nicht an Stickstoff gebundene CH₂-Gruppen durch -O-, -S-, -CO-O-, -O-CO-, -O-CO-O- oder -Si(CH₃)₂ ersetzt sein können und/oder
   b2) eine oder mehrere CH₂-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
   b3) ein oder mehrere H-Atome durch F und/oder Cl ersetzt sein können und/oder
   b4) R⁵ und R⁶ zusammen auch einen Ring bilden können;
c) Phenyl, Biphenyl, 1-Naphthyl, 2-Naphthyl, 2-Thienyl, 2-Furanyl;
   R⁷, R⁸, R⁹, R¹⁰, R¹¹, R¹² sind gleich oder verschieden

a) Wasserstoff, -CN, -F, NO₂ oder -Cl
b) ein geradkettiger oder verzweigter Alkylrest mit 1 bis 20 C-Atomen, wobei
   b1) eine oder mehrere nicht benachbarte CH₂-Gruppen durch -O-, -S-, -CO-O-, -O-CO-, -O-CO-O-, -NR⁵ oder -Si(CH₃)₂- ersetzt sein können und/oder
   b2) eine oder mehrere CH₂-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
   b3) ein oder mehrere H-Atome durch F und/oder Cl ersetzt sein können;
c) Phenyl, Biphenyl, 1-Naphthyl, 2-Naphthyl, 2-Thienyl, 2-Furanyl, -O-Phenyl, -O-Biphenyl, -O-1-Naphthyl, -O-2-Naphthyl, -O-2-Thienyl, -O-2-Furanyl,
m, n, p, q, r sind jeweils gleich oder verschieden 0, 1, 2, 3, 4, 5 oder 6, vorzugsweise 0, 1, 2, 3, 4, 5 oder 6, vorzugsweise 0, 1, 2, 3, 4, besonders bevorzugt 0, 1, 2 oder 3.

Die Verbindungen der Formel (II) sind vorzugsweise amorph und zeichnen sich durch hohe Glasübergangstemperaturen aus.

Bevorzugt sind Spirobifluorenderivate der Formeln (II) a-c 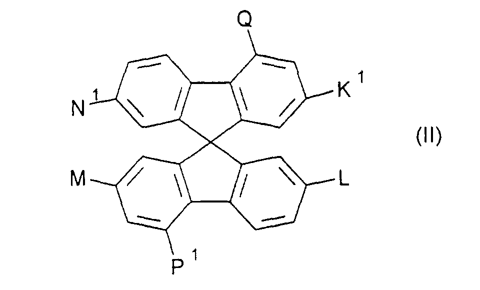 wobei die Symbole folgende Bedeutungen haben:
II.a) K¹ = L = M = N¹ und ist aus der Gruppe: 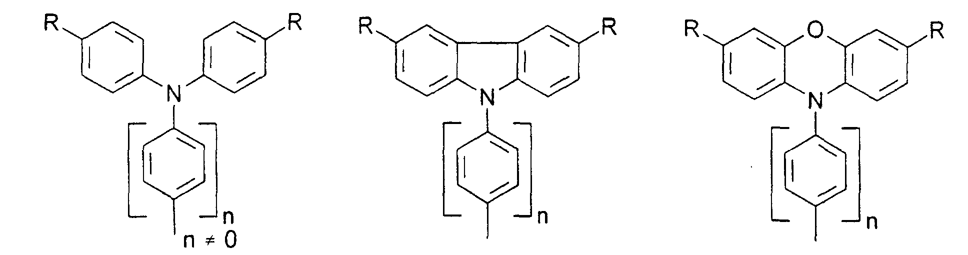 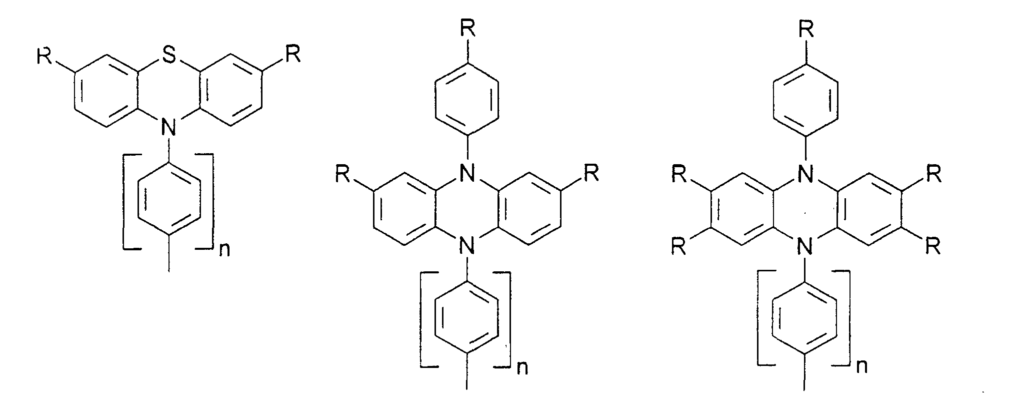 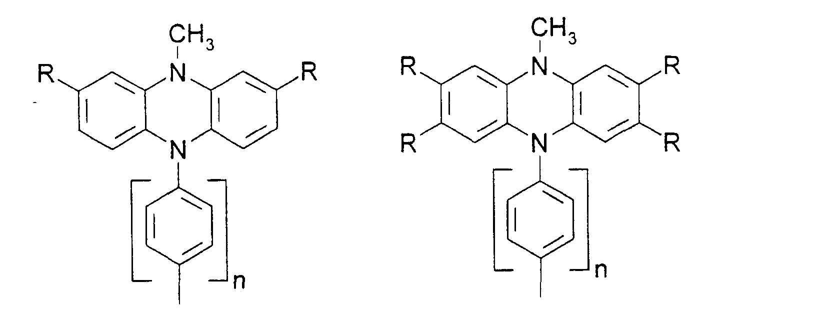 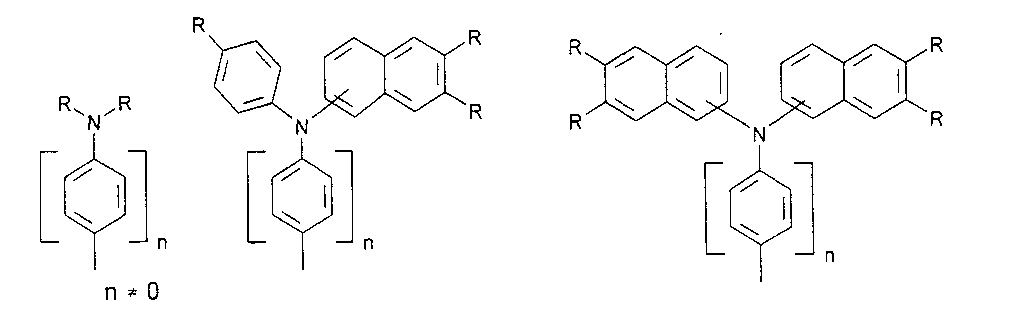 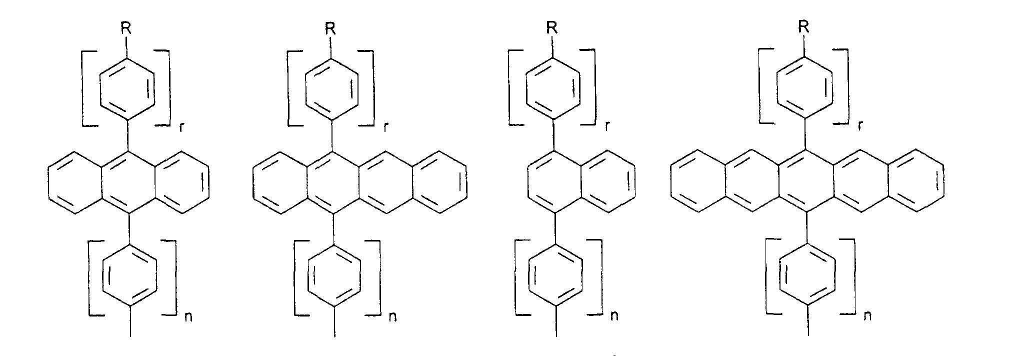
   R gleich oder verschieden H, Alkyl, -O-Alkyl, -S-Alkyl, mit jeweils 1 bis 20 C-Atomen, vorzugsweise 1 bis 4 C-Atomen, Phenyl, Biphenyl, 1-Naphthyl, 2-Naphthyl, 2-Thienyl, 2-Furanyl, -O-Phenyl, -O-Biphenyl, -O-1-Naphthyl, -O-2-Naphthyl, -O-2-Thienyl, -O-2-Furanyl, CN, NR₂, wobei -O-Alkyl/Aryl, -S-Alkyl/Aryl, CN, NR₂ nicht an Stickstoff gebunden sein darf;
   n = 0,1,2,3,4.
   und Q, P¹ sind gleich oder verschieden und aus der Gruppe H, COOR, CH₂OR, 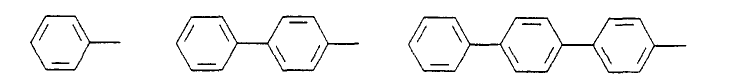 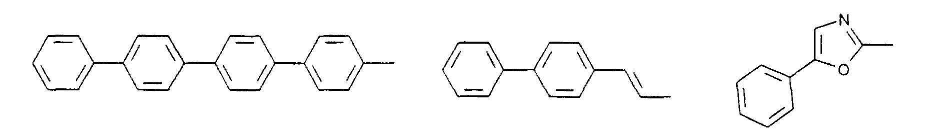 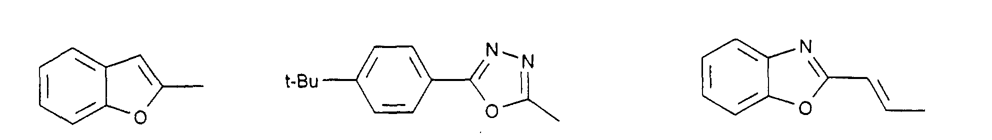 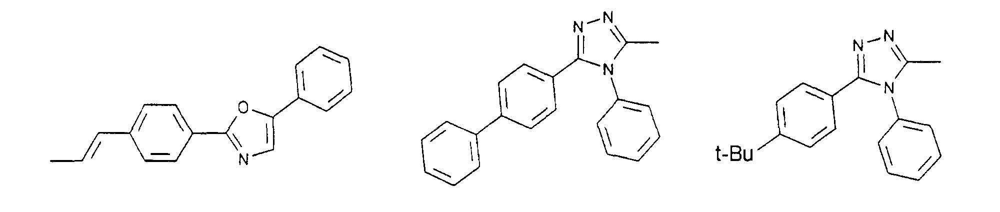 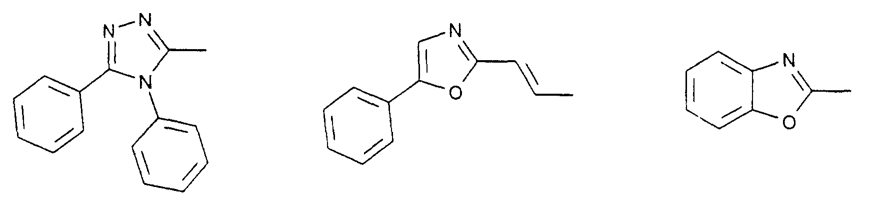 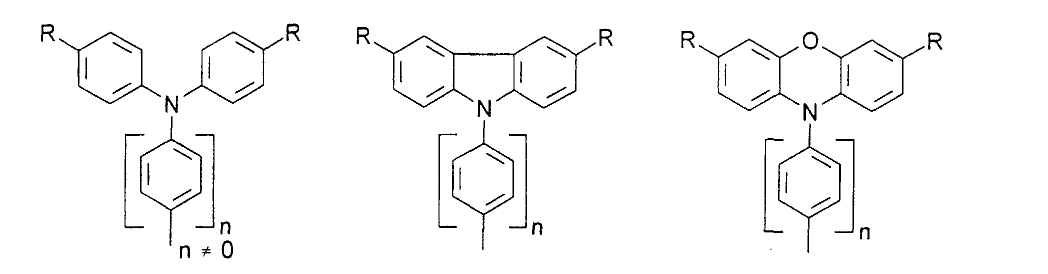 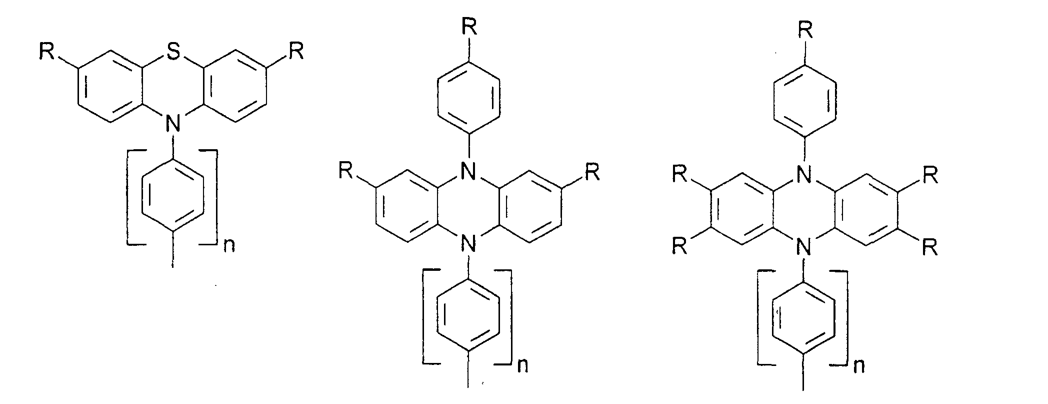 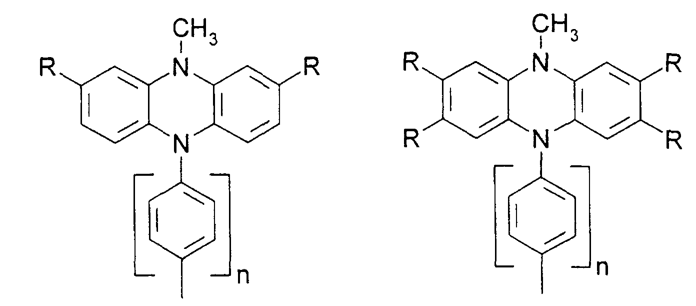 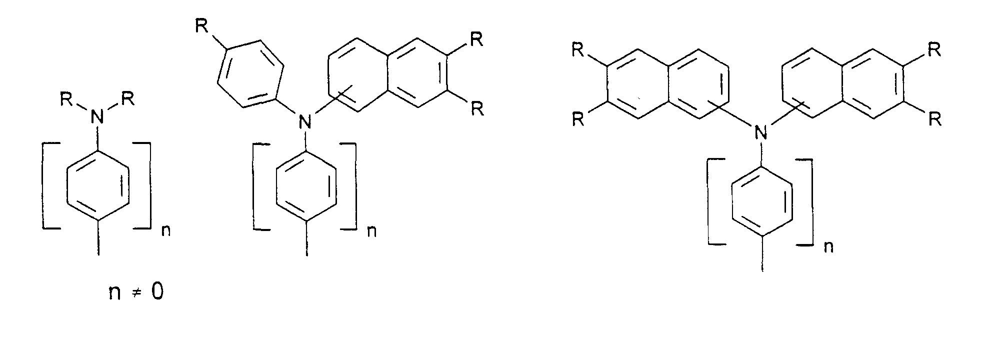 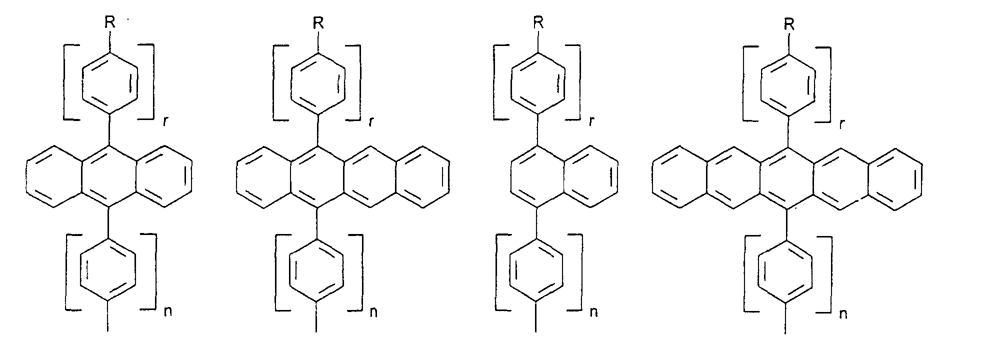 wobei die Symbole und Indizes die oben angegebenen Bedeutungen haben
II.b) K¹ = N¹ und ist aus der Gruppe 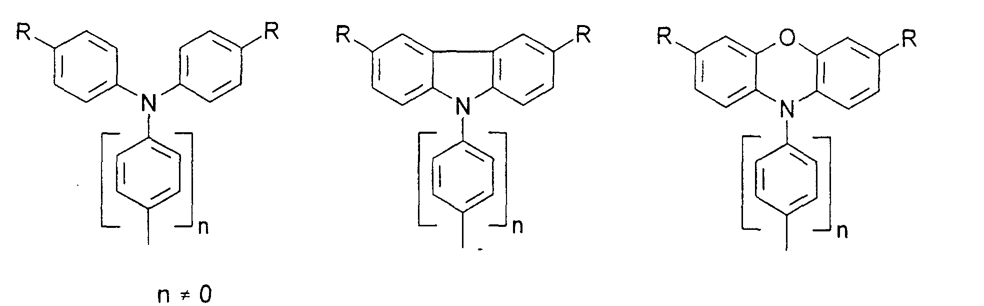 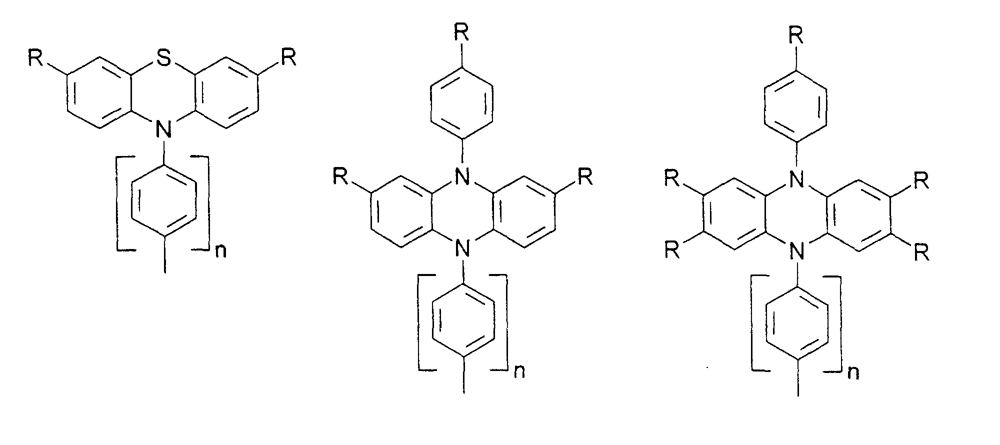 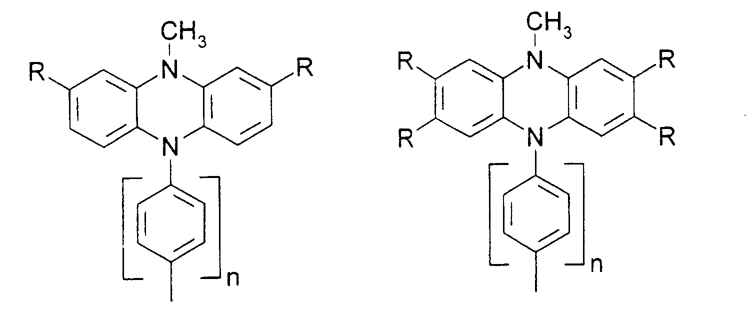 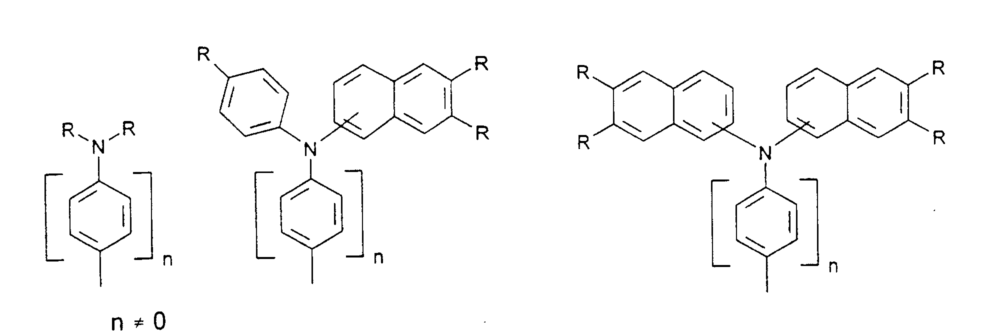 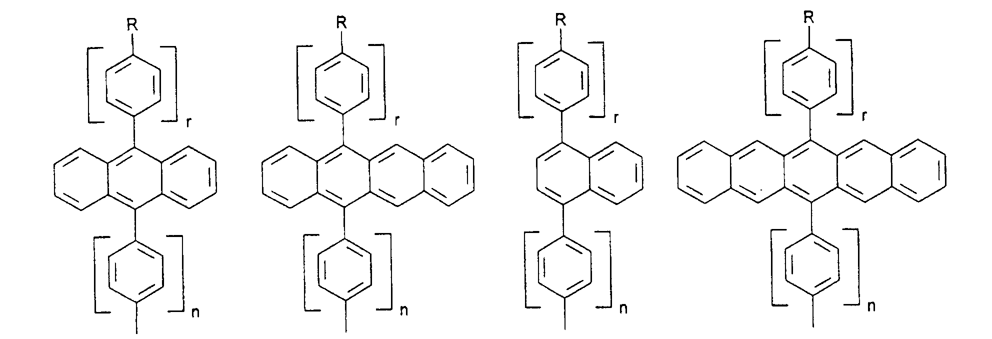 und L = M und ist aus der Gruppe
   H, COOR, CH₂OR, 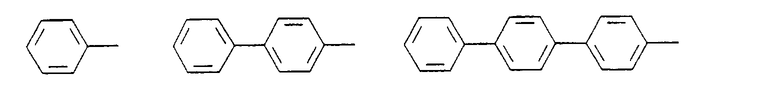 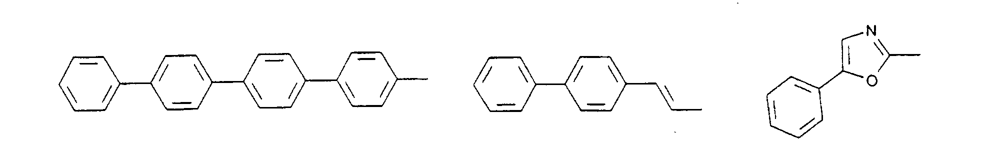 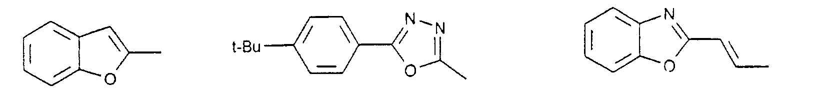 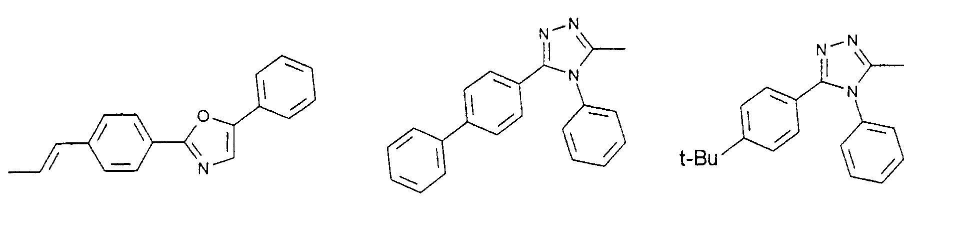 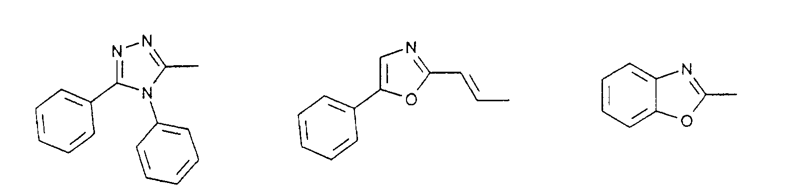 und Q, P¹ sind gleich oder verschieden aus der Gruppe 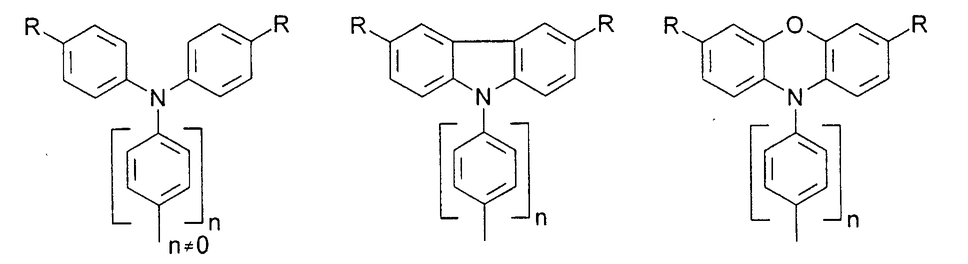 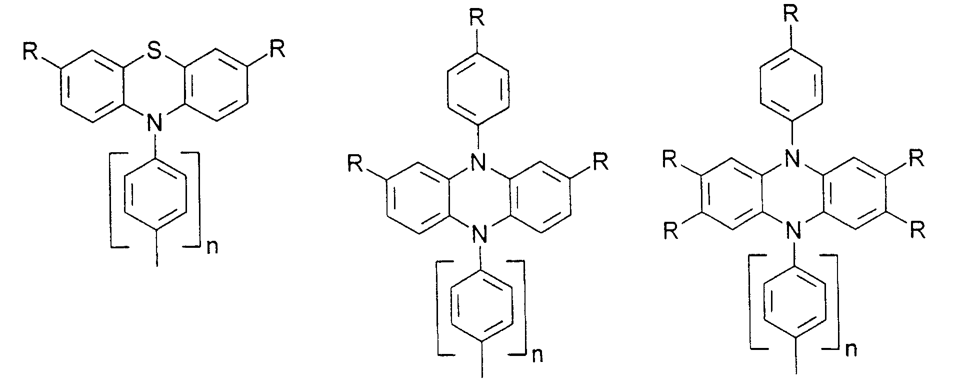 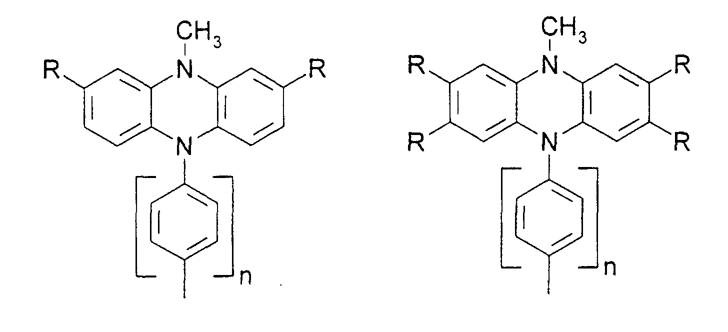 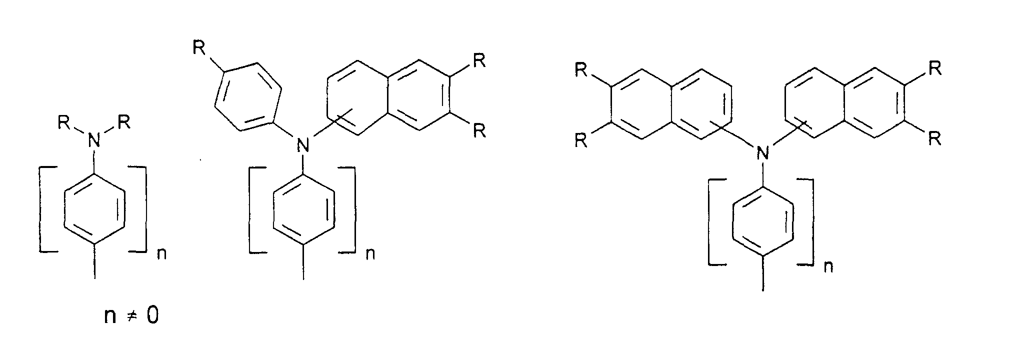 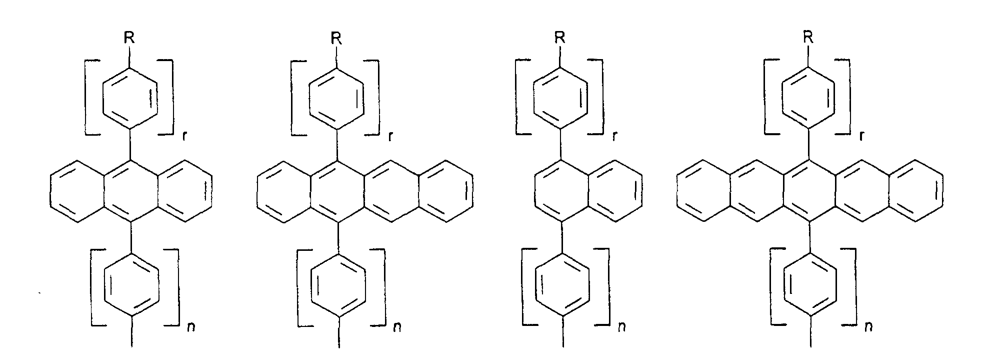 H, COOR, CH₂OR, 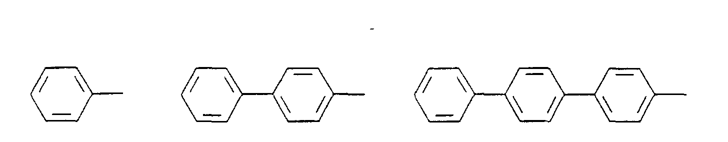 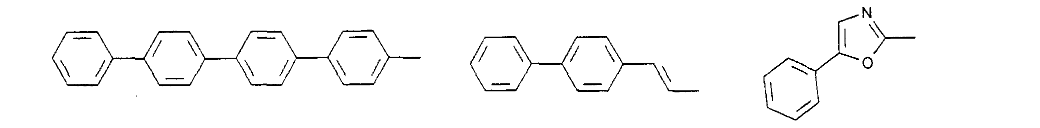 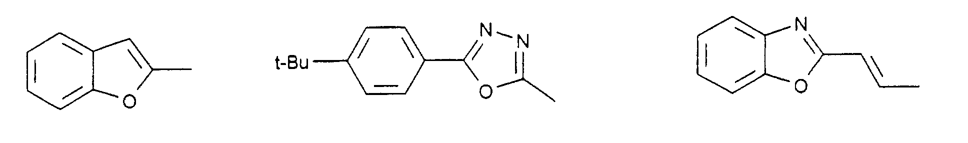 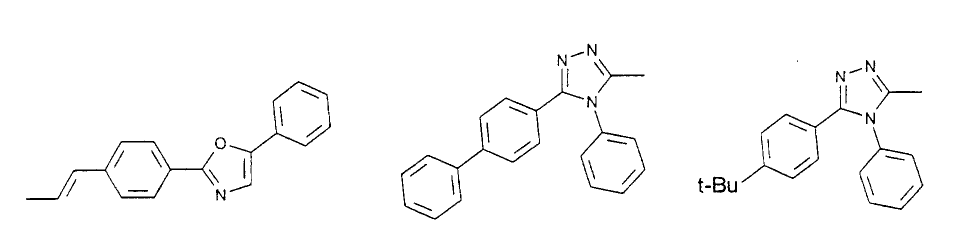 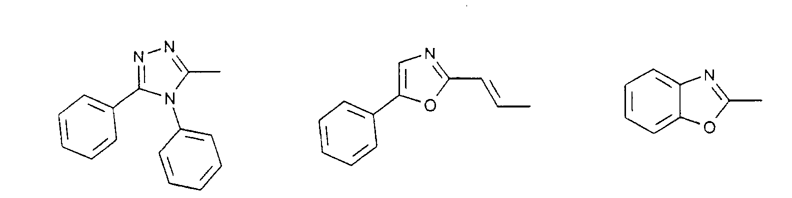 wobei die Symbole und Indizes die oben angegebenen Bedeutungen haben;
llc) K¹ = M und ist aus der Gruppe 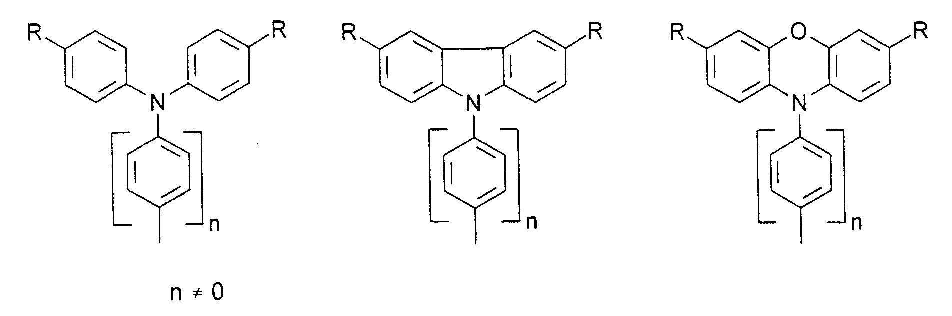 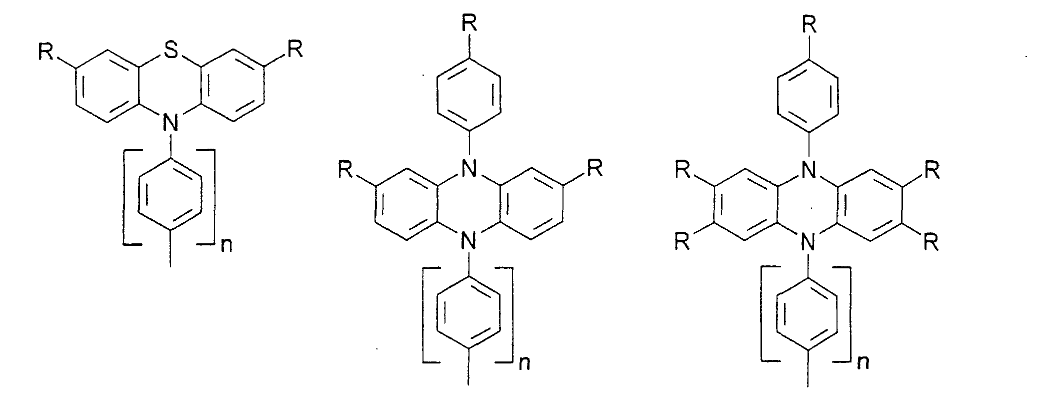 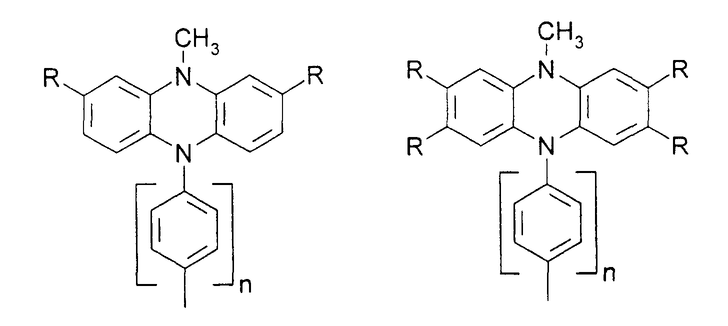 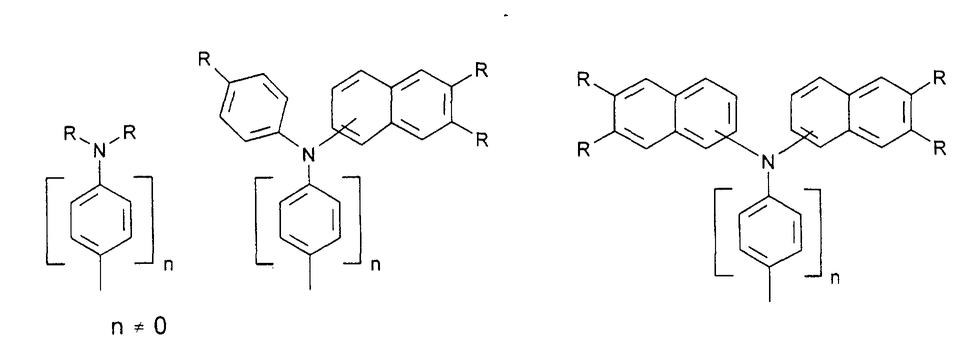 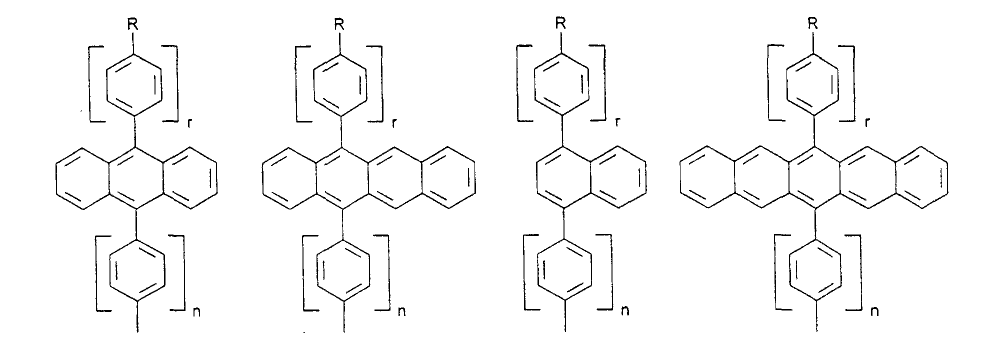 und M = N¹ ist und aus der Gruppe
   H, COOR, CH₂OR, 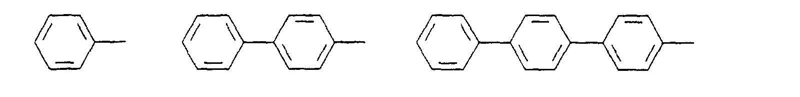 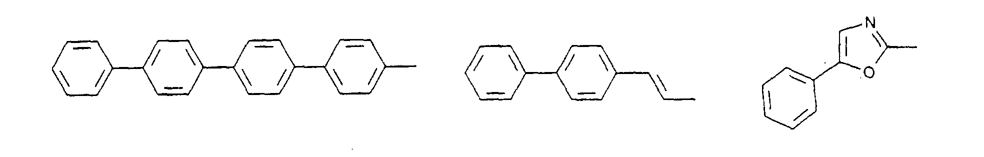 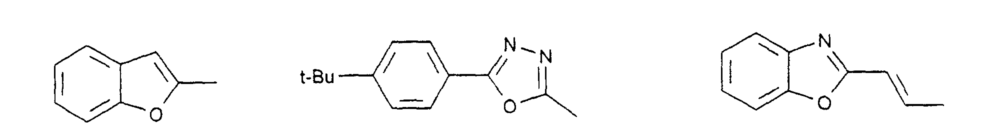 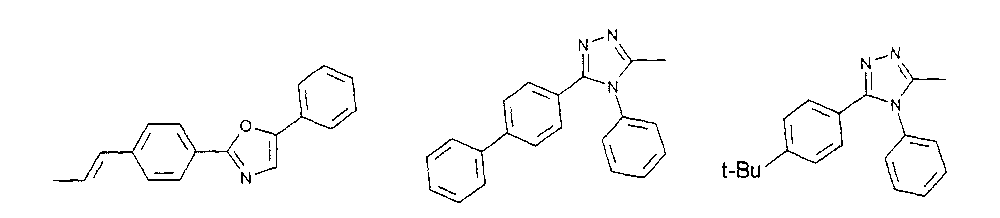 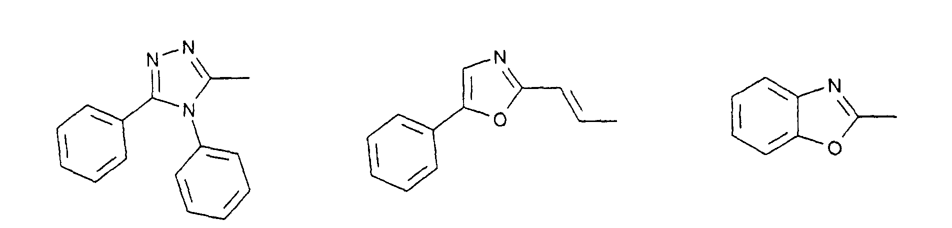 und Q, P¹ sind gleich oder verschieden aus der Gruppe 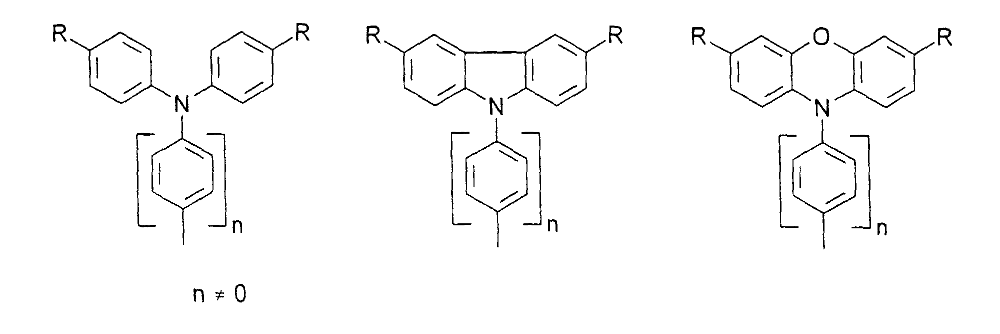 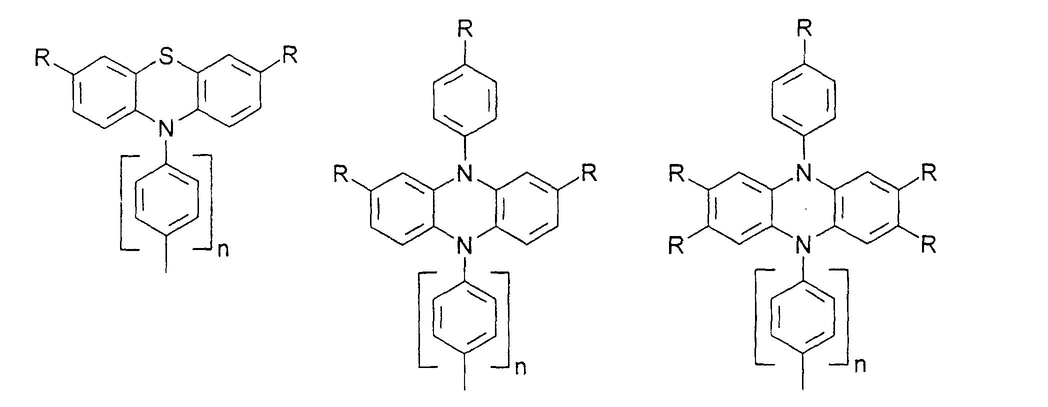 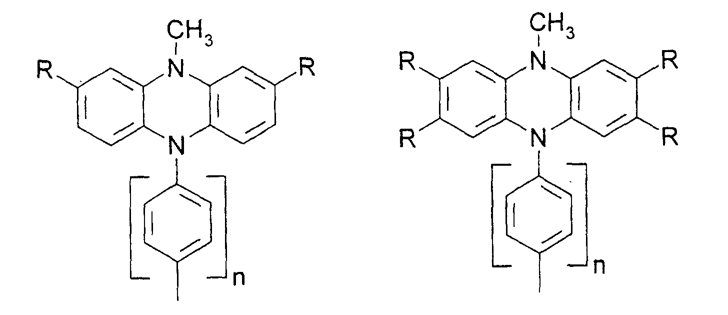 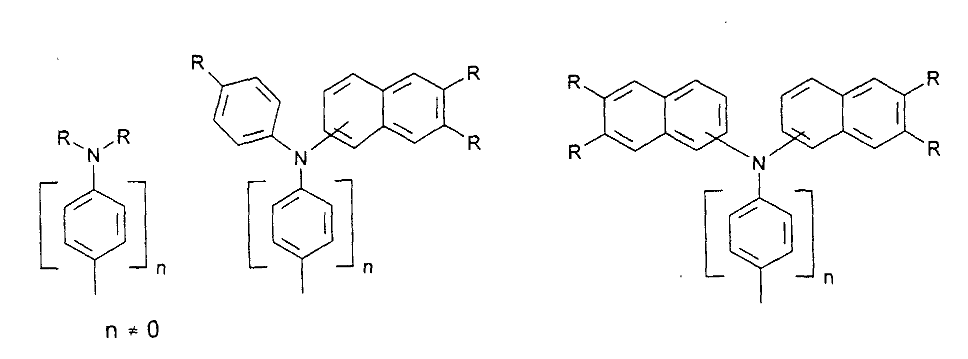 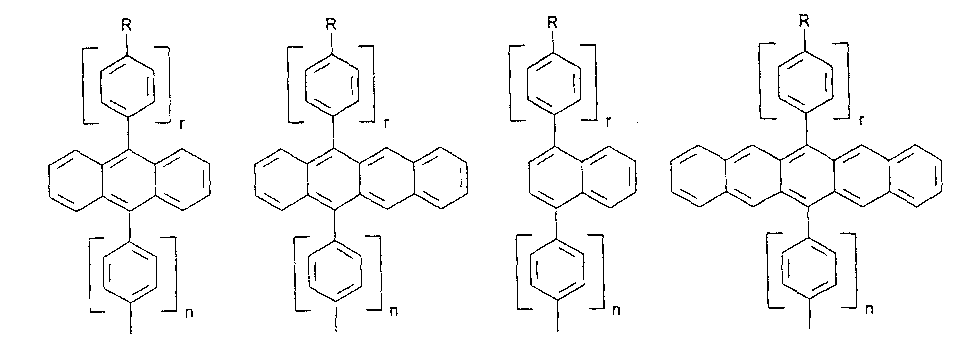 H, COOR, CH₂OR, 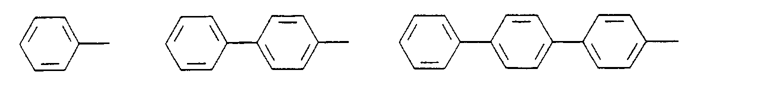 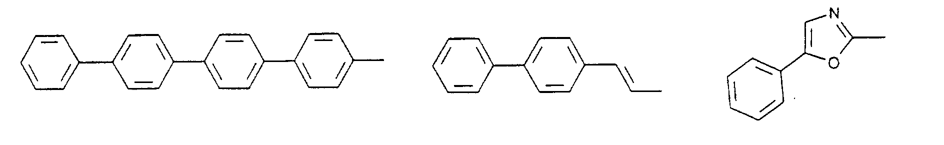 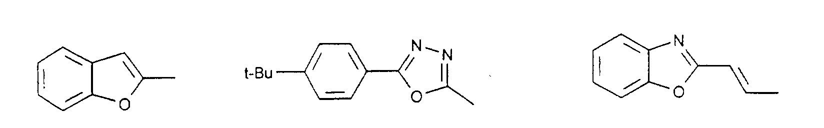 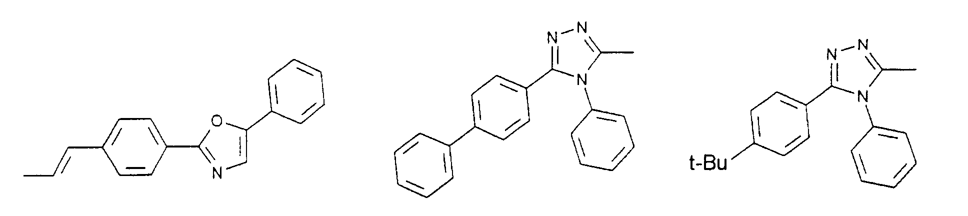 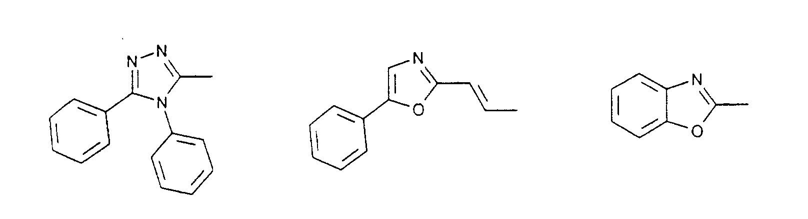 wobei die Symbole und Indizes die oben angegebenen Bedeutungen haben.

Insbesondere bevorzugt sind folgende Verbindungen der Formel (II):
   llaa) K¹ = L = M = N¹ und ist aus der Gruppe: 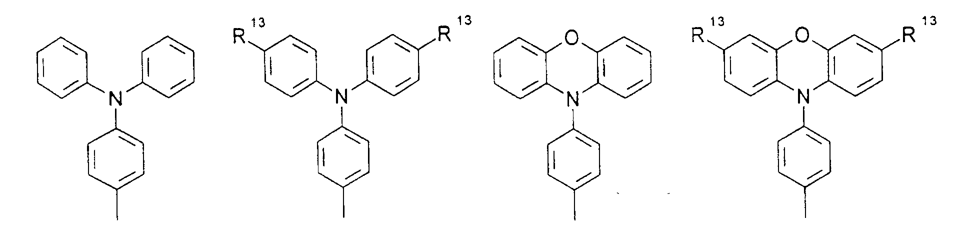 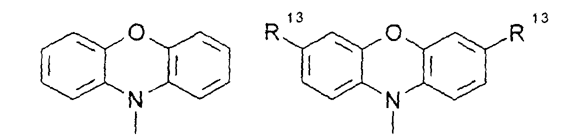 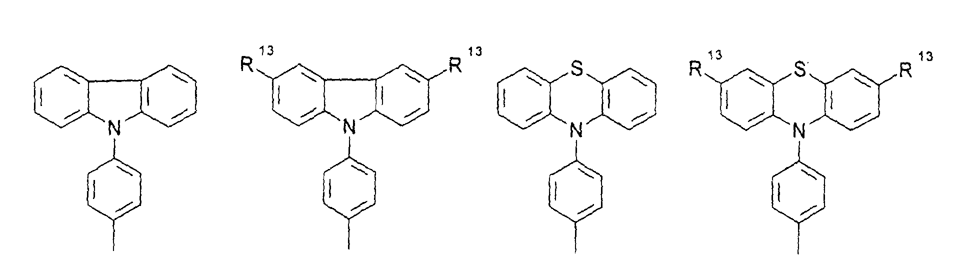 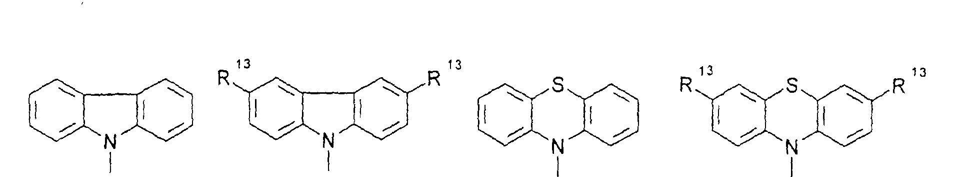 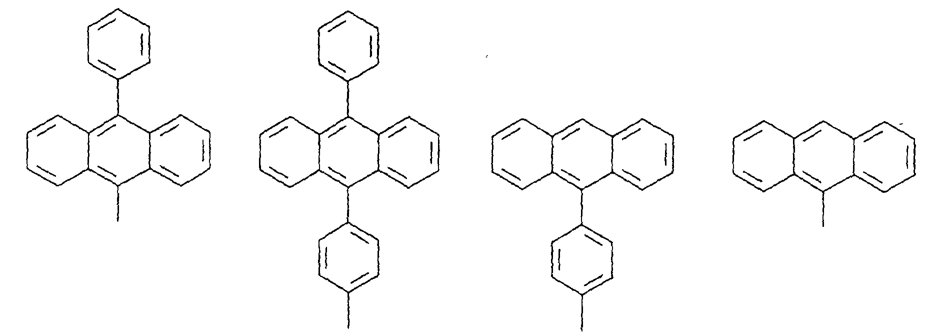 wobei R¹³ -O-CH₃, -O-C₂H₅, -S-CH₃, -S-C₂H₅, vorzugsweise -O-CH₃, -S-CH₃, besonders bevorzugt -O-CH₃, bedeutet;
      und Q = P¹ und ist aus der Gruppe
      H, COOR¹⁴, CH₂OR¹⁴, 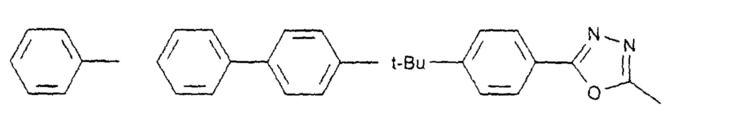 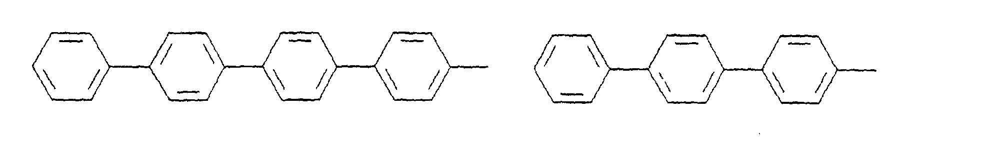 wobei R¹⁴ eine geradkettige oder verzweigte Alkylgruppe mit 1 bis 12, vorzugsweise 1 bis 4 C-Atomen ist;
   II.ba) K¹ = L = M = N¹ = Q = P¹ und ist aus der Gruppe 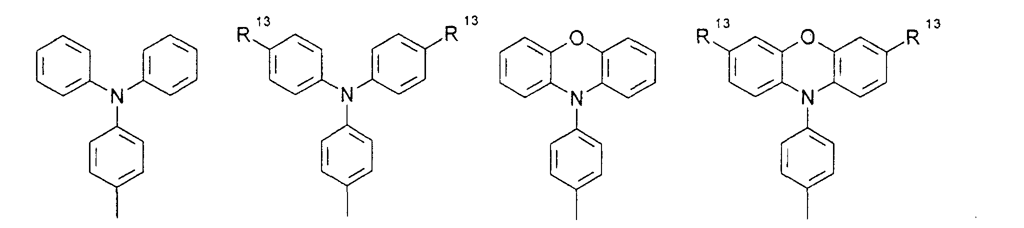 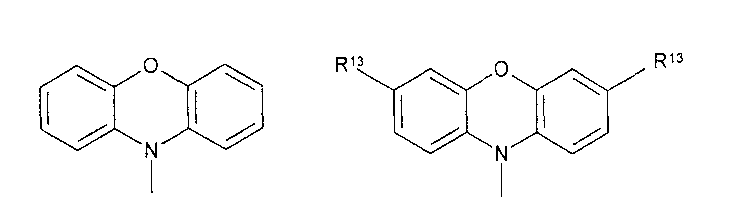 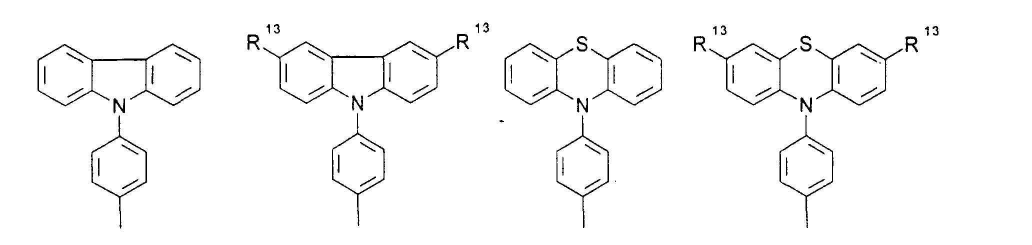 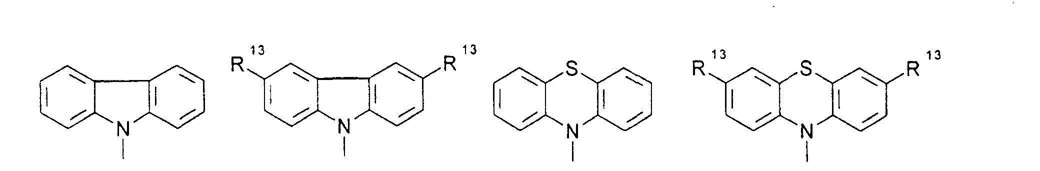 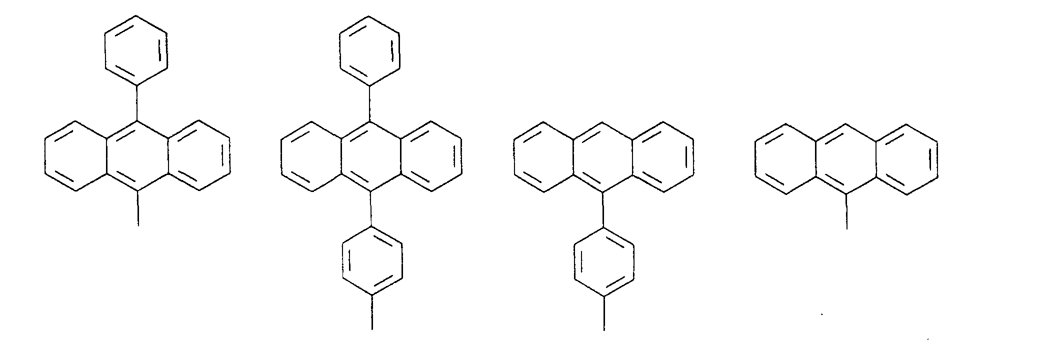 wobei R¹³ die oben angegebenen Bedeutungen hat;
   II.ca) K¹ = L = M = N¹ und ist aus der Gruppe 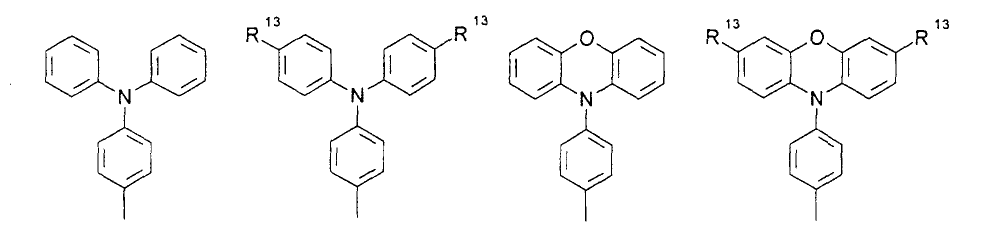 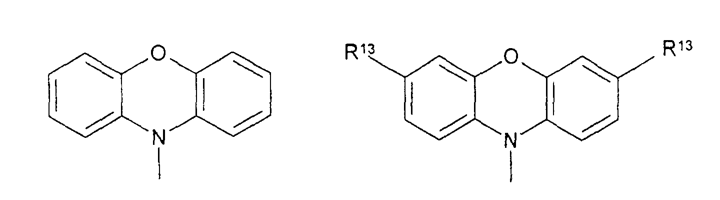 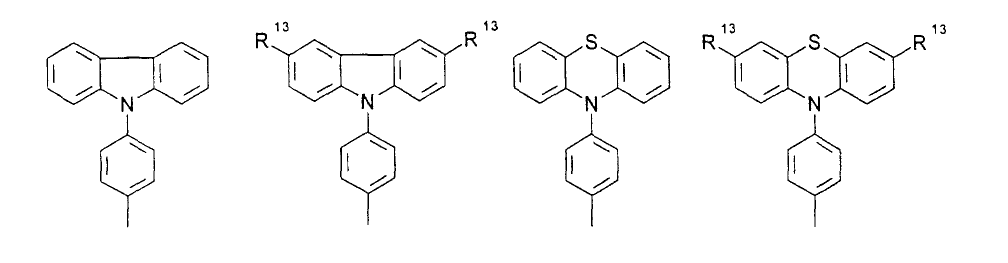 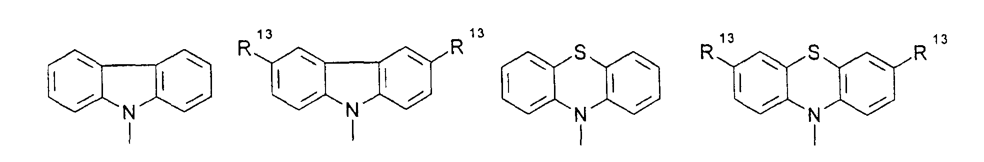 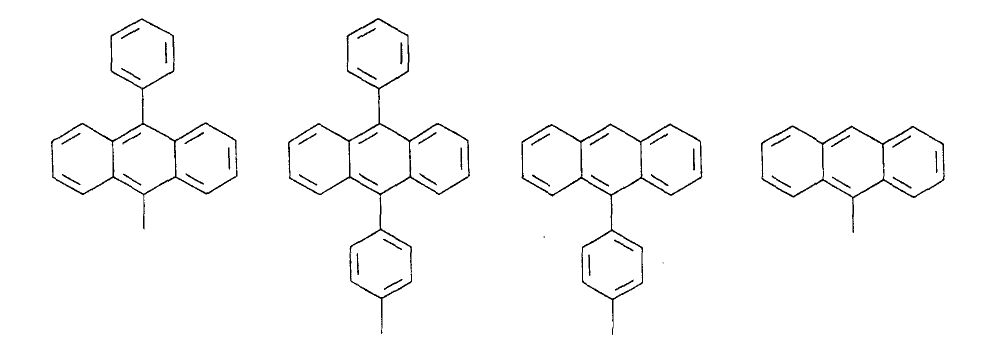 und Q = H und P¹ ist aus der Gruppe
      H, COOR¹⁴, CH₂OR¹⁴, 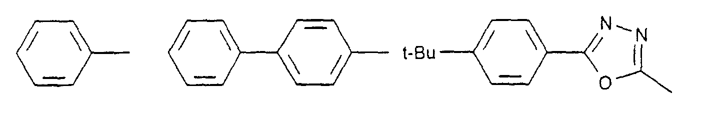 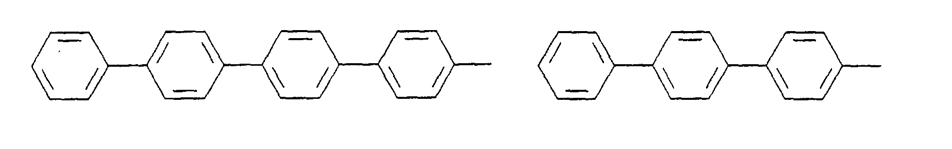
wobei R¹³, R¹⁴ die oben angegebenen Bedeutungen haben.

Die Herstellung der erfindungsgemäßen Spiroverbindungen erfolgt nach an sich literaturbekannten Methoden, wie sie in Standardwerken zur Organischen Synthese, z.B. Houben-Weyl, Methoden der Organischen Chemie, Georg-Thieme-Verlag, Stuttgart und in den entsprechenden Bänden der Serie "The Chemistry of Heterocyclic Compounds" von A. Weissberger und E.C. Taylor (Herausgeber) beschrieben werden.

Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch gemacht werden.

Verbindungen der Formel (I) werden beispielsweise ausgehend vom 9,9'-Spirobifluoren erhalten, dessen Synthese z.B. von R. G. Clarkson, M. Gomberg, J. Am. Chem. Soc. 1030, 52, 2881, beschrieben ist.

Die Herstellung von Verbindungen der Formel (lla) kann beispielsweise ausgehend von einer Tetrahalogenierung in den Positionen 2,2',7,7' des 9,9'-Spirobifluorens und anschließender Substitutionsreaktion erfolgen (siehe z.B. US 5,026,894) oder über eine Tetraacetylierung der Positionen 2,2',7,7' des 9,9'-Spirobifluorens mit anschließender C-C-Verknüpfung nach Umwandlung der Acetylgruppen in Aldehydgruppen oder Heterocyclenaufbau nach Umwandlung der Acetylgruppen in Carbonsäuregruppen erfolgen.

Die Herstellung von Verbindungen der Formel (IIb) kann beispielsweise analog zu denen der Formel (lla) erfolgen, wobei die stöchiometrischen Verhältnisse bei der Umsetzung so gewählt werden, daß die Positionen 2,2' bzw. 7,7' funktionalisiert werden (siehe z.B. J. H. Weisburger, E. K. Weisburger, F. E. Ray, J. Am. Chem. Soc. 1959, 72 4253; F. K. Sutcliffe, H. M. Shahidi, D. Paterson, J. Soc. Dyers Colour 1978, 94, 306 und G. Haas, V. Prelog, Helv. Chim. Acta 1969, 52 1202).

Die Herstellung von Verbindungen der Formel (llc) kann beispielsweise über eine Dibromierung in 2,2' Stellung und anschließende Diacetylierung in 7,7' Stellung des 9,9'-Spirobifluorens mit anschließender Umsetzung analog zu den Verbindungen (llc) erfolgen.

Verbindungen der Formeln (II) mit K¹, L, Q, P¹ = H und M = N¹ oder Q, P¹ = H, K¹ = L und M = N¹ sind beispielsweise durch Wahl geeignet substituierter Ausgangsverbindungen beim Aufbau des Spirobifluorens herstellbar, z.B. kann 2,7-Dibromspirobifluoren aus 2,7-Dibromfluorenon und 2,7-Dicarbethoxy-9,9'-spirobifluoren durch Einsatz von 2,7-Dicarbethoxyfluorenon aufgebaut werden. Die freien 2',7'-Positionen des Spirobifluorens können dann unabhängig weiter substituiert werden.

Für die Synthese der Gruppen K¹, L, M, N¹, P¹, R¹, R², R³, R⁴ sei beispielsweise verwiesen auf DE-A 23 44 732, 24 50 088, 24 29 093, 25 02 904, 26 36 684, 27 01 591 und 27 52 975 für Verbindungen mit 1,4-Phenylen-Gruppen; DE-A 26 41 724 für Verbinoungen mit Pyrimidin-2,5-diyl-Gruppen; DE-A 40 26 223 und EP-A 0 391 203 für Verbindungen mit Pyridin-2,5-diyl-Gruppen; DE-A 32 31 462 für Verbindungen mit Pyridazin-3,6-diyl-Gruppen; N. Miyaura, T. Yanagi und A. Suzuki in Synthetic Communications 1981, 11, 513 bis 519, DE-A-39 30 663; M. J. Sharp, W. Cheng, V. Snieckus, Tetrahedron Letters 1987, 28 , 5093; G. W. Gray, J. Chem. Soc. Perkin Trans II 1989, 2041 und Mol. Cryst. Liq. Cryst. 1989, 172, 165; Mol. Cryst. Liq. Cryst. 1991, 204, 43 und 91; EP-A 0 449 015; WO 89/12039; WO 89/03821; EP-A 0 354 434 für die direkte Verknüpfung von Aromaten und Heteroaromaten.

Die Herstellung disubstituierter Pyridine, disubstituierter Pyrazine, disubstituierter Pyrimidine und disubstituierter Pyridazine findet sich beispielsweise in den entsprechenden Bänden der Serie "The Chemistry of Heterocyclic Compounds" von A. Weissberger und E. C. Taylor (Herausgeber).

Aminogruppen enthaltende Verbindungen der Formel (I) können über Variaten der Ullmann-Reaktion (J. March, Adv. Org. Chem., 4. Aufl., S. 665, John Wiley & Sons, New York 1992) aufgebaut werden, wie sie bsp. in Chem. Lett. 1989, S.1145; Mol. Cryst. Liq. Cryst. 1994, 242, 127 und insbesondere bei J. Salbeck et al., 213^{th} ACS National Meeting, San Francisco 1997, Book of Abstracts S 199, beschrieben ist. Weiterhin möglich ist ein aus US 5,576,460 bekanntes Verfahren. Bevorzugt ist die Herstellung solcher Verbindungen nach einem in der deutschen Patentanmeldung 19738860.4 mit dem Titel "Verfahren zur Herstellung von Aryloligoaminen" offenbarten Verfahren. Auf diese Anmeldung wird ausdrücklich Bezug genommen, sie gilt durch Zitat als Bestandteil der Beschreibung.

Die erfindungsgemäßen Spiroverbindungen der Formel (I) eignen sich als Ladungstransportmaterialien, vorzugsweise für photovoltaische Zellen.

Gegenstand der Erfindung ist daher auch die Verwendung von Spiroverbindungen der Formel (I) als Ladungstransportmaterial, insbesondere für photovoltaische Zellen.

Weiterhin Gegenstand der Erfindung ist eine photovoltaische Zelle mit einer Ladungstransportschicht, die eine oder mehrere, vorzugsweise eine, Spiroverbindung der Formel (I) enthält, vorzugsweise daraus besteht.

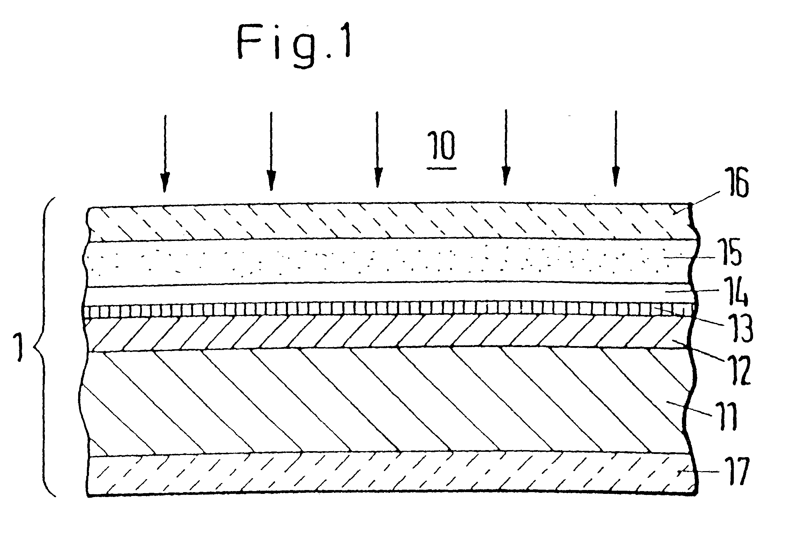

In Fig. 1 ist eine bevorzugte Ausführungsform einer solchen Zelle 1 dargestellt (nicht maßstabsgetreu).
Auf einem leitenden Träger 11, der als Elektrode bzw. Kontakt dienen kann und beispielsweise aus einem Metall oder Indium-Zinn-Oxid (ITO) besteht, ist als lichtabsorbierende Schicht ein Halbleiter 12 aufgebracht, der vorzugsweise eine Oberfläche mit einem Rauheitsfaktor > 1 hat. Vorzugsweise enthält die erfindungsgemäße Zelle auf der Oberfläche des Halbleiters einen Chromophor, hier als Chromophorschicht 13 dargestellt. Die Bezeichnung lichtabsorbierende Schicht umfaßt im Sinne der Erfindung sowohl eine Halbleiterschicht als auch eine Kombination Halbleiter/Chromophor, auch wenn die eigentliche Absorption in diesem Fall nahezu ausschließlich durch den Chromophor erfolgt. Daran schließt sich die Ladungstransportschicht 14 an, die erfindungsgemäß eine Spiroverbindung der Formel (I) enthält. Sie wird auf der einen Seite durch die Gegenelektrode 15 begrenzt, die beispielsweise aus einem leitfähigen Glas, leitfähig beschichtetem Kunststoff, aus Metall oder einem anderen leitfähigen, vorzugsweise lichtdurchlässigen Material, bestehen kann. Die Zelle 1 ist vorzugsweise oben und unten durch je eine isolierende Schicht 16 bzw. 17 abgeschlossen. Sie kann einen, in der Figur nicht gezeigten, seitlichen Abschluß enthalten, beispielsweise einen Rahmen aus einem elektrisch isolierenden Material, wie Kunststoff oder Glas. Durch die Verwendung eines Lochleitermaterials anstelle des flüssigen Elektrolyten ist solch ein seitlicher Rahmen aber nicht grundsätzlich notwendig. Zumindest eine Seite der Zelle muß für das in elektrische Energie zu wandelnde Licht durchlässig sein, so daß dieses zu dem Chromophor bzw. dem Halbleiter gelangen kann.
Die Zelle enthält darüber hinaus in der Figur nicht gezeigte Vorrichtungen zur Abnahme des von der Zelle erzeugten elektrischen Stroms.

Die erfindungsgemäße photovoltarische Zelle enthält als lichtabsorbierende Schicht vorzugsweise einen Halbleiter, der vorzugsweise eine sehr große Bandlücke, besonders bevorzugt mindestens 3,0 eV, ganz besonders bevorzugt über 3,0 eV, aufweist.
Damit eignen sich vorzugsweise Metalloxid-Halbleiter, insbesondere die Oxide der Übergangsmetalle, sowie der Elemente der dritten Hauptgruppe und der vierten, fünften und sechsten Nebengruppe (des periodischen Systems der Elemente) von Titan, Zirkon, Hafnium, Strontium, Zink, Indium, Yttrium, Lanthan, Vanadium, Niob, Tantal, Chrom, Molybdän, Wolfram, aber auch Oxide von Zink, Eisen, Nickel oder Silber, Perovskite, wie SrTiO₃, CaTiO₃, oder Oxide von anderen Metallen der zweiten und dritten Hauptgruppe oder Mischoxide oder Oxidgemische dieser Metalle. Es kann aber auch jedes andere Metalloxid mit Halbleitereigenschaften und großem Energieabstand (Bandlücke) zwischen Valenzband und Leitungband verwendet werden.
Besonders bevorzugt als Halbleitermaterial ist Titandioxid.

Der Halbleiter weist vorzugsweise einen Rauheitsfaktor von größer als 1, besonders bevorzugt von größer als 20, ganz besonders von größer als 150 auf.
Der Rauheitsfaktor ist definiert als das Verhältnis einer wirklichen/effektiven Oberfläche zur Fläche der Projektion dieser Oberfläche eines Körpers, in diesem Fall also der Oberfläche des Halbleiters.

Der Rauheitsfaktor kann z.B.durch gravimetrische Adsorptionsmethoden bestimmt werden, wie z.B. in F. Kohlrausch, Praktische Physik, Band 1, S. 397 (Stuttgart: B.G. Teubner, 1985) beschrieben wird. Im allgemeinen beträgt die Größe der Poren 5-200 nm, vorzugsweise 10-50 nm.

Ein Verfahren zum Herstellen von polykristallinen Metalloxid-Halbleiterschichten mit dem Sol-Gel-Verfahren (beschrieben in Einzelheiten z. B. in Stalder und Augustynski, J. Electrochem. Soc. 1979, 126, 2007), wo beim Verfahrensschritt der Hydrolyse des Metall-Alkoholats die prozentuale relative Feuchtigkeit der Umgebungsatmosphäre in einem Bereich von 30 % bis 80 % liegen kann und innerhalb von ± 5 %, vorzugsweise ± 1 %, konstant gehalten wird, ergibt Metalloxid-Halbleiterschichten, mit denen in erfindungsgemäßen photovoltaischen Zellen eine besonders hohe elektrische Ausbeute erzielt werden kann.
Die rauhe Oberfläche mit polykristalliner Struktur bietet eine um den Rauheitsfaktor größere Fläche für eine, vorzugsweise, monomolekulare Oberflächenschicht eines Chromophors. Damit wird erreicht, daß das auf eine Fläche bestimmter Größe einfallende Licht mit bedeutend höherer Ausbeute in elektrische Energie gewandelt wird. Der Halbleiter kann als für den einfallenden Lichtstrom durchsichtig betrachtet werden. Licht wird aber teilweise auf der Oberfläche reflektiert und gelangt z. T. auf benachbarte Oberflächen. Das in den Halbleiter eindringende und nicht absorbierte bzw. gewandelte Licht trifft z. T, direkt und zu einem anderen Teil indirekt und zu einem weiteren Teil indirekt, nach Totalreflexion an der Oberfläche, auf der Austrittsseite auf Chromophormoleküle, womit es gelingt, eine bedeutend höhere Lichtausbeute zu erzielen.

Die Empfindlichkeit, d. h. die photoelektronische Ausbeute für sichtbares Licht, also auch für Sonnenlicht, kann also erhöht werden, indem auf der Oberfläche des Halbleiters sog. Chromophore, auch Sensibilatoren oder Dyes genannt, als Ladungsträger chemisch an- oder eingelagert (chemisorbiert) werden. Die beiden Funktionen der Lichtabsorption und der Ladungsträger-Trennung sind bei diesen photoelektronischen Systemen getrennt. Die Lichtabsorption wird vom Chromophor im Oberflächenbereich übernommen, und die Trennung der Ladungsträger erfolgt an der Grenzschicht Halbleiter/Chromophor.
Verschiedene Chromophore haben unterschiedliche spektrale Empfindlichkeiten. Die Wahl des Chromophors kann somit der spektralen Zusammensetzung des Lichts der Lichtquelle angepaßt werden, um die Ausbeute möglichst zu vergrößern. Als Chromophore, d. h. Sensibilisatoren, eigenen sich insbesondere die Komplexe von Übergangsmetallen vom Typ Metall (L₃), Metall (L₂) von Ruthenium und Osmium (z. B. Ruthenium-tris-(2,2'-bipyridyl-4,4'-dicarbonsäure) und deren Salze, Ruthenium cis diaqua bipyridyl Komplexe, wie Ruthenium cis-diaqua bis(2,2'bipyridyl-4,4'dicarboxylate) sowie Porphyrine (z. B. Zink tetra (4-carboxyphenyl) Porphyrin) und Cyanide (z. B. Eisen-Hexacyanid-Komplexe) und Phthalocyanine.
Die Chromophore können im Bereich der Oberfläche des Metalloxyd-Halbleiters chemisorbiert, adsorbiert oder sonstwie fest angelagert sein. Günstige Resultate wurden beispielsweise mit Chromophoren erzielt, die mit Carbonsäure- oder Phosphonsäure-Liganden an die Oberfläche des Metalloxyd-Halbleiters gebunden sind.

Geeignete Chromophore sind beispielsweise in Chem. Rev. 1995, 49-68 beschrieben.

Besonders bevorzugt sind Ruthenium-tris-(2,2'-bipyridyl-4,4'-dicarbonsäure), deren Salze und die Chromophore (VIII) und (IX), 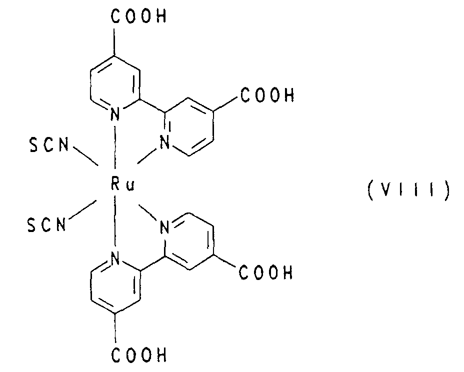 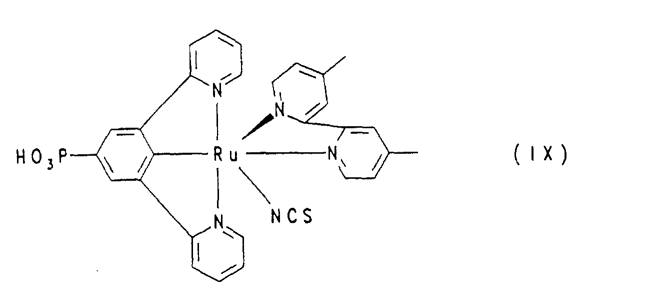 deren Synthese und Eigenschaften in J. Chem. Soc. Chem. Comm. 1995, 65 beschrieben sind.

Das Aufbringen des Chromophors, beispielsweise Ruthenium-tris-(2,2'-bipyridyl-4,4'-dicarbonsäure) oder ein Salz davon, erfolgt z. B. durch Eintauchen des Substrats mit der Oxidschicht in eine Lösung, beispielsweise eine wäßrige oder alkoholische, vorzugsweise ethanolische, während etwa einer Stunde. Die Konzentration der Lösung ist vorzugsweise 0,1 bis 10 molar, besonders bevorzugt 1 bis 5 molar, beispielsweise 2 molar. Andere Chromophore lassen sich nach analogen Verfahren auf Titanoxid oder andere Metalloxid-Halbleiter aufbringen.

Als Elektrode 15 eignen sich stabile, metallisch leitende Substanzen, z. B. Au, Ag, Pt, Cu, oder andere Metalle. Es können aber auch, für einige Anwendungen vorzugsweise lichtdurchlässige leitfähige Substanzen, wie dotierte Metalloxide, z. B. Indium-Zinn-Oxid, Sb-dotiertes Zinnoxid oder Al-dotiertes Zinkoxid, verwendet werden. Dabei kann die Austrittsarbeit des verwendeten Elektrodenmaterials vorzugsweise an das lonisationspotential des verwendeten Lochtransportmaterials angepaßt werden.

Die Elektrode kann, wie in der EP-A 0 333 641 beschrieben, auf ein transparentes Substat, z. B. Glas, aufgebracht werden und mit der Lochtransportschicht verbunden sein. Vorzugsweise kann sie in der erfindungsgemäßen Zelle durch physikalische Abscheidemethoden, z. B. Aufdampfen oder Zerstäuben (Sputtern), direkt auf die Lochtransportschicht aufgebracht werden, ohne daß eine zweite Glasplatte notwendig ist. Dieses Verfahren ist in Anwendungen zu bevorzugen, bei denen das Gewicht der Zelle vermindert werden soll.

Falls gewünscht kann die Zelle z. B. mit einem Kleber oder einer Folie versiegelt werden.

Eine erfindungsgemäße photovoltaische Zelle hat im allgemeinen eine Dicke von 5 bis 20 mm (mit Substrat).
Sie kann zur Vermeidung von Reflexionsverlusten mit einer ein-, zwei- oder mehrlagigen Antireflexschicht versehen sein.

Spiroverbindungen der Formel (I) eigenen sich weiterhin als Elektrolumineszenzmaterialien.

Als Elektrolumineszenzmaterialien im Sinne der Erfindung gelten Materialien, die als aktive Schicht in einer Elektrolumineszenzvorrichtung Verwendung finden. Aktive Schicht bedeutet, daß die Schicht befähigt ist, bei Anlegen eines elektrischen Feldes Licht abzustrahlen (lichtemittierende Schicht) und/oder daß sie die Injektion und/oder den Transport der positiven und/oder negativen Ladungen verbessert (Ladungsinjektions- oder Ladungstransportschicht).

Gegenstand der Erfindung ist daher auch die Verwendung einer Spiroverbindung der Formel (I) als Elektrolumineszenzmaterial, d.h. die Verwendung als aktive Schicht in einer Elektrolumineszenzvorrichtung.

Um als Elektrolumineszenzmaterialien Verwendung zu finden, werden die Spiroverbindungen der Formel (I) im allgemeinen nach bekannten, dem Fachmann geläufigen Methoden, wie Eintauchen (Dipping) oder Spincoating, in Form eines Films auf ein Substrat aufgebracht.

Gegenstand der Erfindung ist weiterhin eine Elektrolumineszenzvorrichtung mit einer oder mehreren aktiven Schichten, wobei mindestens eine dieser aktiven Schichten ein oder mehrere erfindungsgemäße Spiroverbindungen der Formel (I) enthält. Die aktive Schicht kann beispielsweise eine lichtemittierende Schicht und/oder eine Transportschicht und/oder eine Ladungsinjektionsschicht sein.

Der allgemeine Aufbau solcher Elektrolumineszenzvorrichtungen ist beispielsweise in US 4,539,507 und US 5,151,629 beschrieben. Polymere enthaltende Elektrolumineszenzvorrichtungen sind beispielsweise in WO-A 90/13148 oder EP-A 0 443 861 beschrieben.

Sie enthalten üblicheiweise eine elektrolumineszierende Schicht zwischen einer Kathode und einer Anode, wobei mindestens eine der Elektroden transparent ist. Zusätzlich können zwischen der elektrolumineszierenden Schicht und der Kathode eine oder mehrere Elektroneninjektions- und/oder Elektronentransportschichten eingebracht sein und/oder zwischen der elektrolumineszierenden Schicht und der Anode eine oder mehrere Lochinjektions- und/oder Lochtransportschichten eingebracht sein. Als Kathode dienen vorzugsweise Metalle oder metallische Legierungen, z.B. Ca, Mg, Al, In, Mg/Ag. Als Anode dienen beispielsweise Metalle, z.B. Au, oder andere metallisch leitende Stoffe, wie Oxide, z.B. ITO (Indiumoxid/Zinnoxid) auf einem transparentem Substrat, z.B. aus Glas oder einem transparenten Polymer.

Im Betrieb wird die Kathode auf negatives Potential gegenüber der Anode gesetzt. Dabei werden Elektronen von der Kathode in die Elektroneninjektionsschicht/ Elektronentransportschicht oder direkt in die lichtemittierende Schicht injiziert. Gleichzeitig werden Löcher von der Anode in die Lochinjektionsschicht/ Lochtransportschicht oder direkt in die lichtemittierende Schicht injiziert.

Die injizierten Ladungsträger bewegen sich unter dem Einfluß der angelegten Spannung durch die aktiven Schichten aufeinander zu. Dies führt an der Grenzfläche zwischen Ladungstransportschicht und lichtemittierender Schicht oder innerhalb der lichtemittierenden Schicht zu Elektronen/Loch-Paaren, die unter Aussendung von Licht rekombinieren.
Die Farbe des emittierten Lichtes kann durch die als lichtemittierende Schicht verwendeten Materialien variiert werden.

Elektrolumineszenzvorrichtungen finden z.B. Anwendung als selbstleuchtende Anzeigeelemente, wie Kontrollampen, alphanumerische Displays, Hinweisschilder, und in optoelektronischen Kopplern.

Auf den Inhalt der prioritätsbegründenden deutschen Patentanmeldung 19711714.7 mit dem Titel "Spiroverbindungen und deren Werwendung", sowie auf den Inhalt der Zusammenfassung der vorliegenden Anmeldung wird hiermit ausdrücklich Bezug genommen, beide gelten durch Zitat als Bestandteil dieser Anmeldung.

Die Erfindung wird durch die Beispiele näher erläutert, ohne sie dadurch beschränken zu wollen.

### Beispiele

### A. Ausgangsverbindungen

a) 9,9'-Spirobifluoren
   6,3 g Magnesiumspäne und 50 mg Anthracen wurden in 120 ml trockenem Diethylether in einem 1 l Dreihalskoben mit Rückflußkühler unter Argon vorgelegt und das Magnesium 15 min mit Ultraschall aktiviert. 62 g 2-Brombiphenyl wurden in 60 ml trockenem Diethylether gelöst. Etwa 10 ml dieser Lösung wurden dem vorgelegten Magnesium zugegeben, um die Grignard-Reaktion zu starten.
   Nach dem Anspringen der Reaktion wurde unter weiterer Ultrabeschallung die 2-Brombiphenyl-Lösung so zugetropft, daß die Lösung gelinde am Rückfluß siedete. Nach beendeter Zugabe wurde die Reaktionsmischung eine weitere Stunde am Rückfluß unter Ultraschall gekocht.
   48,8 g 9-Fluorenon wurden in 400 ml trockenem Diethylether gelöst und unter weiterer Ultrabeschallung der Grignard-Lösung zugetropft. Nach beendeter Zugabe wurde weitere 2 h gekocht. Der nach Abkühlung der Reaktionsmischung ausgefallene gelbe Magnesiumkomplex des 9-(2-Biphenyl)-9-fluorenols wurde abgesaugt und mit wenig Ether gewaschen. Der Magnesiumkomplex wird in 800 ml Eiswasser hydrolysiert, welches 40 g Ammoniumchlorid enthielt. Nach 60 min Rühren wurde das gebildete 9-(2-Biphenyl)-9-fluorenol abgesaugt, mit Wasser gewaschen und trocken gesaugt.
   Das getrocknete 9-(2-Biphenyl)-9-fluorenol wurde dann in 500 ml Eisessig in der Hitze gelöst. Zu dieser Lösung wurden 0,5 ml HCI konz. gegeben. Man ließ die Lösung einige Minuten kochen und fällte das gebildete 9,9'-Spirobifluoren aus der heißen Lösung mit Wasser (Wasserzugabe bis Trübung einsetzte). Nach Abkühlung wurde das Produkt abgesaugt und mit Wasser gewaschen. Das getrocknete Produkt wurde zur weiteren Reinigung aus Ethanol umkristallisiert. Man erhielt 66 g (80 %, bez. auf 2-Brombiphenyl) 9,9'-Spirobifluoren als farblose Kristalle Smp. 198°C.
b) 2,2'-Dibrom-9,9'-spirobifluoren (F. K. Sutcliffe, H. M. Shahidi, D. Patterson, J. Soc. Dyers Colour 94 (1978) 306)
   3,26 g (10,3 mmol) 9,9'-Spirobifluoren wurden in 30 ml Methylenchlorid gelöst und mit 5 mg FeCl₃ (wasserfrei) als Katalysator versetzt. Der Reaktionskolben wurde vor Lichtzutritt geschützt. 1,12 ml (21,8 mmol) Brom in 5 ml Methylenchlorid wurden innerhalb von 30 min unter Rühren zugetropft. Nach 24 h wurde die resultierende braune Lösung mit gesättigter NaHCO₃-Lösung und Wasser gewaschen, um überschüssiges Brom zu entfernen. Die organische Phase wurde nach dem Trocknen über Na₂SO₄ einrotiert. Der weiße Rückstand wurde aus Methanol umkristallisiert, man erhielt 3,45 g (70 %) der Dibromverbindung als farblose Kristalle, Smp. 240°C.
c) 2,7-Dibromo-9,9'-spirobifluoren
   Ein Grignardreagenz bereitet aus 0.72 g (30 mmol) Magnesiumspänen und 5.1 ml (30 mmol) 2-Brombiphenyl in 15 ml Diethylether wurde im Verlauf von 2 h, unter Rühren (im Ultraschallbad) zu einer siedenden Suspension von 10.0 g (29.6 mmol) 2,7-Dibrom-9-fluorenon in 100 ml trockenem Diethylether getropft. Nach beendeter Zugabe wurde 3 Stunden weiter gekocht. Nach Abkühlung über Nacht wurde der ausgefallene Niederschlag abgesaugt und mit kaltem Ether gewaschen. Der abgesaugte Magnesiumkomplex wurde in einer Lösung von 15 g Ammoniumchlorid in 250 ml Eiswasser hydrolisiert. Nach 1 h wurde das gebildete 9-(2-Biphenylyl)-2,7-dibromo-9-fluorenol abgesaugt, mit Wasser gewaschen und trockengesaugt. Das getrocknete Fluorenol wurde für die Ringschlußreaktion in 100 ml Eisessig, nach Zugabe von 3 Tropfen HCI conc. 6 Stunden gekocht. Man ließ über Nacht kristallisieren, saugte das gebildete Produkt ab und wusch mit Eisessig und Wasser.
   Ausbeute: 11g ( 77%) 2,7-Dibromo-9,9'-spirobifluoren. Zur weiteren Reinigung wurde aus THF umkristallisiert.
   ¹H-NMR (CDCl₃, ppm): 6.73 (d, J= 7.63 Hz, 2 H, H-1',8'); 6.84 (d, J = 1.83 Hz, 2 H, H-1,8); 7.15 (td, J = 7.63, 1.22 Hz., 2 H, H-2',7'); 7.41 (td, J = 7.63, 1.22 Hz, 2 H, H-3',6'); 7.48 (dd, J = 8.24, 1.83 Hz, 2 H, H-3,6); 7.67 (d, J = 8.24; 2 H; H-4,5); 7.85 (d, J = 7.63, 2 H, H-4',5').
d) 2,2',7,7'-Tetrabrom-9,9'-spirobifluoren
   Zu einer Lösung von 3,16 g (10,0 mmol) 9,9'-Spirobifluoren in 30 ml Methylenchlorid wurden 80 mg (0,5 mmol) wasserfreies FeCl₃ gegeben und 2,1 ml (41 mmol) Brom in 5 ml Methylenchlorid über 10 min hinweg zugetropft. Die Lösung wurde 6 h rückflußgekocht. Beim Abkühlen fiel das Produkt aus. Der Niederschlag wurde abgesaugt und mit wenig kaltem Methylenchlorid gewaschen. Nach dem Trocknen erhielt man 6,0 g (95 %) der Tetrabromverbindung als weißen Feststoff.
e) 2-Bromo-9,9'-spirobifluoren und 2,2',7-Tribrom-9,9'-spirobifluoren sind bei veränderter Stöchiometrie auf analoge Weise herstellbar.
f) 2,2',4,4',7,7'-Hexabrom-9,9'-spirobifluoren In einem 250 ml 250 ml Dreihalskolben mit Trockenrohr und Tropftrichter wurden unter Lichtausschluß zu einer Lösung von 6.32 g 9,9'-Spirobifluoren in 40 ml Methylenchlorid (mit einer Spatelspitze wasserfreiem FeCl₃) unter Rühren bei RT 6.43 ml Brom in 10 ml Methylenchlorid innerhalb von 10 min. zugetropft. Nach 1 h wurde der entstandene weiße Niederschlag abgesaugt. Feststoff und Mutterlauge wurden getrennt voneinander aufgearbeitet. Der Feststoff wurde in CH₂Cl₂ gelöst und wie die Mutterlauge mit ges. Na₂SO₃-Lösung, ges. NaHCO₃ -Lösung und Wasser gewaschen, über Na₂SO₄ getrocknet, eingeengt. Aus der Feststofffraktion wurden 10,0 g reines, farbloses 2,2',4,4',7,7'-Hexabrom-9,9'-spirobifluoren isoliert, aus der Muttelauge konnten noch 5,7 g schwach gefärbtes Produkt isoliert werden. Die Gesamtausbeute war nahezu quantitativ.
g) 2,2',7,7'-Tetrajod-9,9'-spirobifluoren
   In einem 100 ml Zweihalskolben mit Rückflußkühler und Trockenrohr wurden 3,16 g (10 mmol) 9,9'-Spirobifluoren, gelöst in 30 ml Chloroform, bei Raumtemperatur mit 5,8 g (22,8 mmol) lod versetzt und anschließend 10,75g (25 mmol) Bis-(trifluoracetoxy)-jodbenzol zugegeben. Das Reaktionsgemisch erwärmte sich auf ca 40° unter Bildung eines hellen Niederschlags. Nach 1,5 h wurde das bereits ausgefallene Produkt abgesaugt und mit Chloroform nachgewaschen und getrocknet. Die Chloroformlösungen werden vereinigt, und nacheinander mit gesättigter Natriumsulfitlösung, gesättigter Natriumkarbonatlösung und Wasser gewaschen. Nach dem Trocknen über Natriumsulfat wurde eingeengt und eine zweite Produktfraktion erhalten. Beide Produktfraktionen wurden vereinigt, in Aceton aufgekocht und nach Abkühlung abgesaugt. Man erhielt 2,2',7,7'-Tetrajod-9,9'-spirobifluoren als feinkristallines farbloses Pulver mit 8,1 g in nahezu quantitativer Ausbeute. ¹H-NMR (CDCl₃, ppm): 6.98 (d, J = 1,48 Hz, 4 H, H-1,1',8,8'); 7.54 (dd, J = 7.88, 1.48 Hz, 4 H, H-3,3',6,6'); 7.72 (d, J = 7.88 Hz, 4 H, H-4,4',5,5').
h) 9,9'-Spirobifluoren-2,2'-dicarbonsäure
   aus 2,2'-Dibrom-9,9'-spirobifluoren über 2,2'-Dicyano-9,9'-spirobifluoren. 1,19 g 2,2'-Dibromo-9,9'-spirobifluoren und 0,54 g CuCN wurden in 5 ml DMF 6 h zum Rückfluß erhitzt. Die erhaltene braune Mischung wurde in eine Mischung aus 3 g FeCl₃ (hydrat.) und 1,5 ml konz. HCI in 20 ml Wasser gegossen. Die Mischung wurde 30 min bei 60 bis 70°C gehalten, um den Cu-Komplex zu zerstören. Die heiße wäßrige Lösung wurde zweimal mit Toluol extrahiert. Die organischen Phasen wurden dann mit verdünnter HCI, Wasser und 10 %iger NaOH gewaschen. Die organische Phase wurde filtriert und eingeengt. Der erhaltene gelbe Rückstand wurde aus Methanol umkristallisiert, man erhielt 0,72 g (80 %) 2,2'-Dicyano-9,9'-spirobifluoren als schwach gelbliche Kristalle (Schmelzbereich 215 bis 245°C). 3 g 2,2'-Dicyano-9,9'-spirobifluoren wurden mit 25 ml 30 %iger NaOH und 30 ml Ethanol für 6 h unter Rückfluß erhitzt. Das Dinatriumsalz der Spirobifluorendicarbonsäure fiel als gelber Niederschlag aus, der abfiltriert und in 25 %iger HCI erhitzt wurde, um die freie Säure zu gewinnen. Die Spirobifluorendicarbonsäure wurde aus Eisessig umkristallisiert. Man erhielt 2,2 g (66,6 %) weiße Kristalle (Smp. 376°C, IR-Bande 1685 cm⁻¹ C=O).
i-k) 9,9'-Spirobifluoren-2,2',7,7'-tetracarbonsäure ist aus 2,2',7,7'-Tetrabrom-9,9'-spirobifluoren in analoger Weise herstellbar, ebenso sind 9,9'-Spirobifluoren-2,2',7,-triacarbonsäure aus 2,2',7,-Triabrom-9,9'-spirobifluoren und 9,9'-Spirobifluoren-2,2',4,4',7,7'-hexacarbonsäure aus 2,2',4,4 ,7,7'-Hexabrom-9,9'-spirobifluoren in analoger Weise darstellbar.
l) 9,9'-Spirobifluoren-2,2'-dicarbonsäure
   aus 9,9'-Spirofluoren über 2,2'-Diacetyl-9,9'-spirobifluoren (G. Haas, V. Prelog, Helv. Chim. Acta 52 (1969) 1202; V. Prelog, D. Bedekovic, Helv. Chim. Acta 62 (1979) 2285) Eine Lösung von 3,17 g 9,9'-Spirobifluoren in 30 ml abs. Schwefelkohlenstoff wurde nach Zugabe von 9,0 g feingepulvertem, wasserfreien AlCl₃ während 10 min tropfenweise unter Rühren mit 1,58 g Acetylchlorid in 5 ml abs. Schwefelkohlenstoff versetzt und 1 Stunde unter Rückfluß gekocht. Die unter vermindertem Druck zur Trockene eingedampfte Mischung wurde bei 0°C mit 100 g Eis und 50 ml 2n Salzsäure versetzt. Nach üblicher Aufarbeitung wurde das Rohprodukt chromatographisch mit Benzol/Essigester (10:1) an Kieselgel getrennt. Man erhielt 3,62 g (89 %) 2,2'-Diacetyl-9,9'-spirobifluoren (umkristallisiert aus Chloroform/Essigester, Smp. 255 bis 257°C) und 204 mg 2-Acetyl-9,9'-spirobifluoren (umkrist. aus Chloroform/Benzol, Smp. 225°C). [Daneben konnte bei der Chromatographie auch 2,2',7-Triacetyl-9,9'-spirobifluoren (Smp. 258 bis 260°C) und 2,2',7,7'-Tetraacetyl-9,9'-spirobifluoren (Smp. > 300°C) isoliert werden, umkristallisiert aus Essigester/Hexan].
   Zu einer Lösung von 6,0 g Natriumhydroxid in 30 ml Wasser wurden bei 0°C unter Rühren zuerst 7,2 g Brom und dann eine Lösung von 3,0 g 2,2'-Diacetyl-9,9'-spirobifluoren in wenig Dioxan zugetropft. Nach weiterem 1 stündigem Rühren bei Raumtemperatur wurde die klare gelbe Lösung mit 1 g Natriumhydrogensulfit, gelöst in 20 ml Wasser, versetzt. Nach Ansäuern mit konz. HCI wurde das ausgefallene farblose Produkt abfiltriert und mit wenig Wasser gewaschen. Umkristallisation aus Ethanol lieferte 9,9'-Spirobifluoren-2,2'-dicarbonsäure als wasserklare Prismen (Smp 352°C).
m) Synthese von 2,7-Dicarbethoxy-9,9'-spirobifluoren
   Ein Grignardreagenz bereitet aus 0.97 g (40 mmol) Magnesiumspänen und 9.32g (6.8 ml, 40 mmol) 2-Brombiphenyl in 50 ml trockenem Diethylether wurde im Verlauf von 2 h zu einer siedenden Lösung von 13 g (40 mmol) 2,7-Dicarbethoxy-9-fluorenon in 100 ml trockenem Diethylether getropft. Nach beendeter Zugabe wurde 3 Stunden weiter gekocht. Nach Abkühlung über Nacht wurde der ausgefallene Niederschlag abgesaugt und mit kaltem Ether gewaschen. Der abgesaugte Magnesiumkomplex wurde in einer Lösung von 15 g Ammoniumchlorid in 250 ml Eiswasser hydrolisiert. Nach 1 h wurde das gebildete 9-(2-Biphenylyl)-2,7-dicarbethoxy-9-fluorenol abgesaugt, mit Wasser gewaschen und trockengesaugt. Das getrocknete Fluorenol wurde für die Ringschlußreaktion in 100 ml Eisessig, nach Zugabe von 3 Tropfen HCI conc. 6 Stunden gekocht. Man ließ über Nacht kristallisieren, saugt das gebildete Produkt ab und wusch mit Eisessig und Wasser. Ausbeute: 15,1 g (82%) 2,7-Dicarbethoxy-9,9'-spirobifluoren. Zur weiteren Reinigung wurde aus Ethanol umkristallisiert.
   ¹H-NMR (CDCl₃, ppm): 1.30 (t, J = Hz, 6 H, Ester-CH₃); 4.27 (q, J = Hz, 4 H, Ester-CH₂); 6.68 (dt, J = 7.63, 0.92 Hz, 2 H, H-1', 8'); 7.11 (td, J = 7.48, 1.22 Hz, 2H, H-2', 7'); 7.40 (td, J = 7.48, 1.22 Hz, 4 H, H-1, 8, 3', 6'); 7.89 (dt, J = 7.63, 0.92 Hz, 2 H, H-4', 5'); 7.94 (dd, J = 7.93, 0.6 Hz, 2 H, H-4, 5); 8.12 (dd, J = 7.93, 1.53 Hz, 2 H, H-3, 6).
n) Spirobifluoren-4-carbonsäure
   In einem 2 L Kolben wurden 100 g Fluorenon in 1000 ml trockenem Xylol gelöst und unter Rühren mit 200 g trockenem NaOH versetzt. Anschließend wurde 70 h am Rückfluß gekocht. Nach Abkühlen wurde abgesaugt und der Rückstand mit ca. 2 l Wasser versetzt. Die wäßrige Suspension wurde abgesaugt und der Niederschlag nochmals mit Toluol aufgeschlämmt, abgesaugt und getrocknet. Diese Zwischenstufe wurde in einem 500 ml Zweihalskolben mit Rückflußkühler und Trockenrohr mit ca. 5 ml Eisessig pro 1 g unter Refluxieren gelöst, 20 ml konz. HCI zugegeben und 5 h am Rückfluß gekocht. Die Reaktionsmischung wurde auf ca. das halbe Volumen eingeengt und anschließend auf das vierfache Volumen H₂O gegossen. Der angefallene Niederschlag wurde abgesaugt, gut mit H₂O gewaschen und getrocknet. Zur weiteren Reinigung wurde bei RT in alkalischer MeOH/Wasser (10:1) Mischung gelöst und mit Toluol ausgeschüttelt. Die MeOH/Wasser-Phase wurde abgetrennt und nach dem Ansäuern mit Salzsäure in Wasser gegossen. Die ausgefallene Spirobifluoren-4-carbonsäure wurde abgesaugt, mit Wasser gewaschen und getrocknet. Ausbeute 35 g.
o) 2',7'-Dibrom-9,9'-spirobifluoren-4-carbonsäure
   60 g 9,9'-Spirobifluoren-4-carbonsäure wurden in 600 ml Chloroform in der Hitze gelöst, und mit 1 Spsp Eisen(III)-chlorid versetzt. Über einen Tropftricher wurden 60 ml Brom in 60 ml CHCl₃ innerhalb 30 min. zugetropft und weitere 90 min refluxiert. Nach Abkühlung wurde der ausgefallenen Niederschlag abgesaugt und gewaschen, das Filtrat mit Na₂SO₃-Lösung und NaHCO₃-Lösung und Wasser gewaschen. Nach Aufarbeitung und Reinigung analog zur Hexabromierung isolierte man 74,3 g ( 85%) 2',7'-Dibrom-9,9'-spirobifluoren-4-carbonsäure als weißes Pulver.
p) 2,2'-Bis(brommethyl)-9,9'-spirobifluoren
   aus 2,2'-Dicarboxy-9,9'-spirobifluoren über 9,9'-Spirobifluoren-2,2'-dimethanol (V. Prelog, D. Bedekovicc, Helv. Chim. Acta 62 (1979) 2285) Bei Raumtemperatur wurden 10 g einer 70 %igen Lösung von Natrium-dihydro-bis(2-methoxyethoxy)-aluminat (Fluka) in Benzol langsam zu einer Suspension von 2,0 g 2,2'-Dicarboxy-9,9'-spirobifluoren (freie Carbonsäure) in 20 ml Benzol zugetropft. Nach 2 h Kochen unter Rückfluß, wobei sich die Carbonsäure auflöste, wurde das überschüssige Reduktionsmittel bei 10°C mit Wasser zersetzt, das Gemisch mit konz. HCI angesäuert und mit Chloroform ausgeschüttelt.
   Die mit Wasser gewaschene und über Magnesiumsulfat getrocknete organische Phase wurde eingedampft und der Rückstand aus Benzol umkristallisiert. Man erhielt 1,57 g 9,9'-Spirobifluoren-2,2'-dimethanol (Smp. 254 bis 255°C).
   Zu einer Lösung von 13,5 g 9,9'-Spirofluoren-2,2'-dimethanol in 400 ml Benzol wurden 91,5 g einer 33 %igen Lösung von Bromwasserstoff in Eisessig zugetropft und das Gemisch 7 h unter Rückfluß gekocht. Danach wurde mit 200 ml Wasser versetzt und die mit Wasser gewaschene und über Magnesiumsulfat getrocknete organische Phase eingedampft. Die Chromatograhie an Kieselgel mit Benzol lieferte 11,7 g 2,2'-Bis(brommethyl)-9,9'-spirobifluoren als farblose Plättchen (Smp. 175 bis 177°C).
q) 9,9'-Spirobifluoren-2,2'-dicarbaldehyd und
r) 2'-Hydroxymethyl-9,9'-spirobifluoren-2-carbaldehyd Eine Lösung von 380 mg 9,9'-Spirobifluoren-2,2'-dimethanol in 15 ml Toluol wurde mit 5 g Chrom(VI)oxid auf Graphit (Seloxcette, Alpha Inorganics) versetzt und 48 h unter Stickstoff am Rückfluß gekocht. Dann wurde durch eine Glasfilternutsche abgenutscht und das Filtrat eingedampft. Chromatographie an Kieselgel mit Chloroform und Kristallisation aus Methylenchlorid/Ether lieferte 152 mg 9,9'-Spirobifluoren-2,2'-dicarbaldehyd (Smp. > 300°C) und
   204 mg 2'-Hydroxymethyl-9,9'-spirobifluoren-2-carbaldehyd (Smp. 262 bis 263°C).
s) 2,2'-Diamino-9,9'-spirobifluoren
   Eine Mischung aus 150 ml konz. HNO₃ und 150 ml Eisessig wurden zu einer kochenden Lösung von 15,1 g 9,9'-Spirobifluoren in 500 ml Eisessig über einen Zeitraum von 30 min zugetropft anschließend wurde die Lösung 75 min weiter refluxiert. Nach Abkühlung und Stehenlassen der Lösung für 1 h wurde das gleiche Volumen Wasser zugesetzt, wobei das Produkt ausgefiel. Nach dem Absaugen erhielt man 18,5 g gelbe Kristalle (Smp. 220 bis 224°C) von 2,2'-Dinitro-9,9'-Spirobifluoren. Umkristallisation aus 250 ml Eisessig ergibt 12,7 g hellgelbe Kristallnadeln (Smp. 245 bis 249°C, analytisch rein 249 bis 250°C).

Eine Mischung aus 4,0 ml Dinitro-spriobifluoren und 4,0 g Eisenpulver wurde in 100 ml Ethanol unter Rückfluß erhitzt, während 15 ml konz. HCI über einen Zeitraum von 30 min zugetropft wurden. Nach weiteren 30 min Rückflußkochen wurde überschüssiges Eisen abfiltriert. Das grüne Filtrat wurde in eine Lösung aus 400 ml Wasser, 15 ml konz. NH₄OH und 20 g Na,K-Tartrat gegeben. Das weiße Diamin wurde von der dunkelgrünen Lösung des Eisenkomplexes abfiltriert. Das Diamin wurde zur Reinigung in verdünnter HCI gelöst und bei Raumtemperatur mit Aktivkohle (Darco) gerührt und abfiltriert. Die filtrierte Lösung wurde unter Rühren (KPG-Rührer) tropfenweise mit NH₄OH neutralisiert und das ausgefallene Produkt abgesaugt. Man erhielt 3,5 g weißes 2,2'-Diamino-9,9'-spirobifluoren, das aus Ethanol umkristallisiert wurde (Smp. 243°C).

### B. Synthesebeispiel

a) 2,2',7,7'-Tetra-(N-carbazolyl)-9,9'-spirobifluoren
   In einem 100 ml-Dreihalskolben mit Rückflußkühler und Stickstoffventil wurden 5,01 g (6.11 mmol) 2,2',7,7'-Tetraiod-spirobifluoren mit 5,30 g (31,7 mmol) Carbazol, 1,52 g (23,9 mmol) aktiviertem Kupfer, 6,60 g (47,8 mmol) Kaliumcarbonat und 499 mg (1,89 mmol) 18-crown-6 in 25 ml o-Xylol unter Rückfluß (144 °C) 80 Stunden erhitzt. Die Reaktionsmischung nahm im Laufe der Reaktion eine grünliche Färbung an. Nach Abkühlung wurde abgesaugt, und der Rückstand mit 30 ml Wasser 20 min aufgekocht. Nach Abkühlung wurde die wäßrige Suspension abgesaugt, und das Produkt aus dem festen Rückstand unter Zugabe von 3 Tropfen Hydrazinhydrat in 140 ml Chloroform gelöst. Man ließ 15 min refluxieren, filtrierte ab und wusch mit 5 ml Chloroform nach. Aus der einrotierten Chloroformlösung isolierte man 5,50 g Rohprodukt. Aus dem Filtrat der Reaktionslösung wurde eine weitere Fraktion (0,890 g) isoliert, dazu wurde zur Trockene eingeengt und der Rückstand mit 5 ml Aceton aufgerührt, abgesaugt und mit Aceton nachgewaschen. Das Rohprodukt wurde zur Entfernung von verbliebenem Carbazol mit Toluol erhitzt und anschließend abgesaugt. Gesamtausbeute: 4 g (67 %) 2,2',7,7'-Tetra-(N-carbazolyl)-9,9'-spirobifluoren. Zur weiteren Reinigung wurde aus Chloroform/Methanol umkristallisiert.

## Patentansprüche

1. Spiroverbindung der Formel (I), 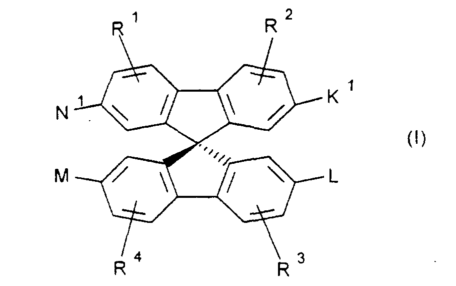 wobei die Symbole folgende Bedeutungen haben:
K¹, L, M, N¹, R¹, R², R³, R⁴ sind gleich oder verschieden und bedeuten
a) Wasserstoff, -NO₂, -CN, -F oder -Cl,
b) einen geradkettigen oder verzweigten Alkylrest mit 1 bis 20 C-Atomen, wobei
b1) eine oder mehrere nicht benachbarte CH₂-Gruppen durch -O-, -S-, -CO-O-, -O-CO-, -O-CO-O-, NR⁵ oder -Si(CH₃)₂- ersetzt sein können und/oder
b2) eine oder mehrere CH₂-Gruppen durch -CH=CH-, -C≡C-, 1,4-Phenylen, 1,4-Cyclohexylen oder 1,3-Cyclopentylen ersetzt sein können und/oder
b3) ein oder mehrere H-Atome durch F und/oder Cl ersetzt sein können und/oder
c) eine der folgenden Gruppen: 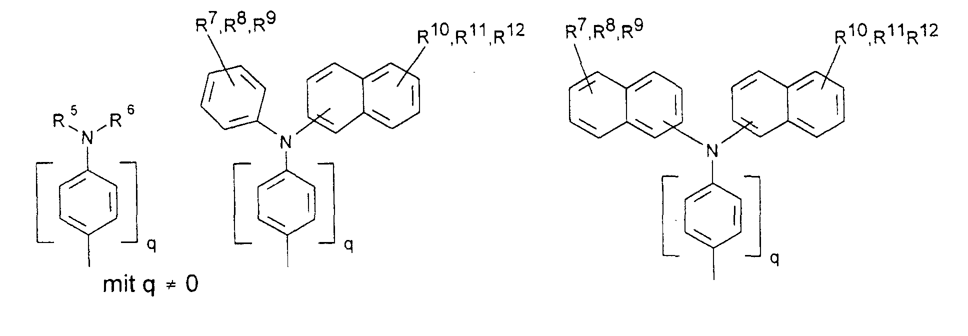 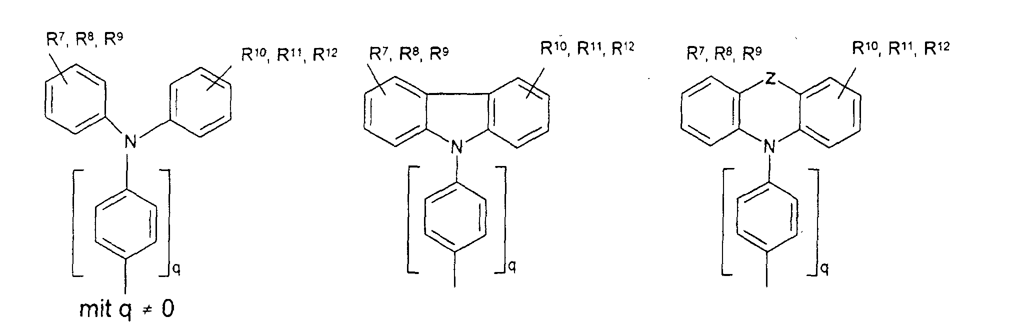 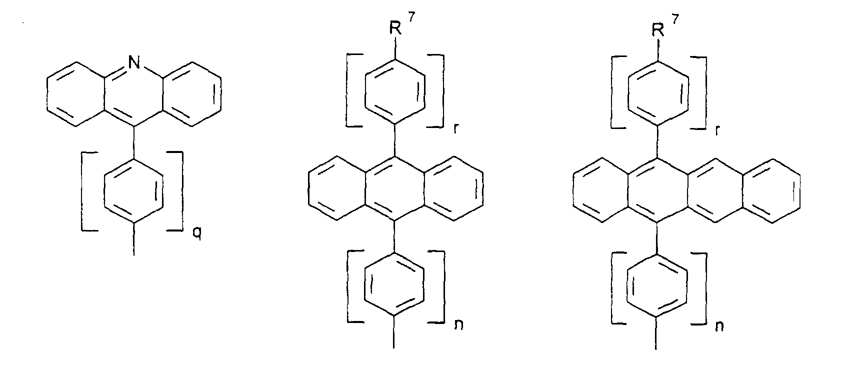 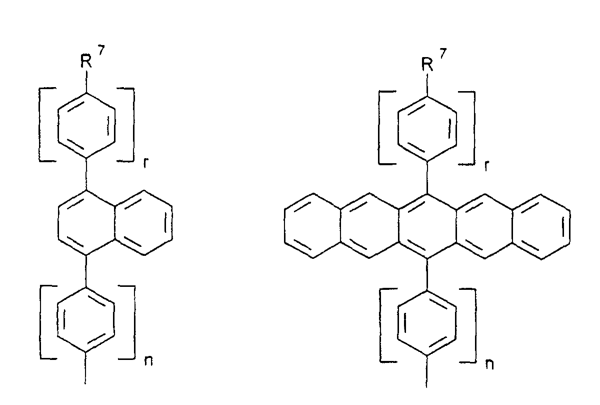 oder
d) eine der folgenden Gruppen: 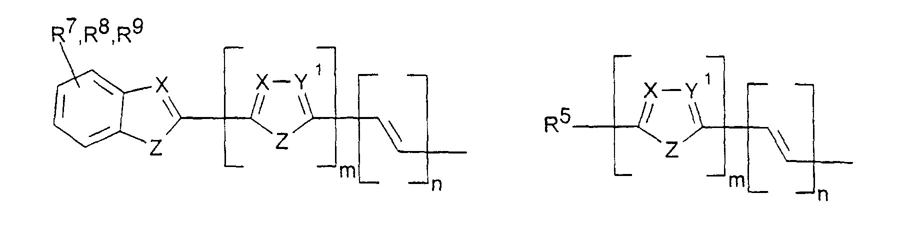 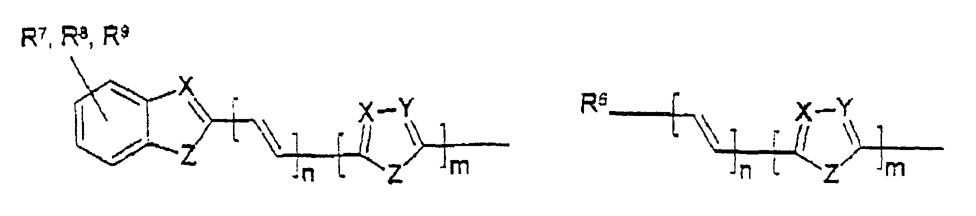 mit der Maßgabe, daß mindestens einer der Reste K¹, L, M, N¹, R¹, R², R³, R⁴ eine der unter c) aufgeführten Gruppen ist;
X, Y¹ sind jeweils gleich oder verschieden =CR⁷- oder =N-;
Z ist -O-, -S-, -NR⁵-, -CRR-, -CR=CR- oder -CR=N-;
R⁵, R⁶ sind jeweils gleich oder verschieden
a) Wasserstoff
b) ein geradkettiger oder verzweigter Alkylrest mit 1 bis 20 C-Atomen, wobei
b1) eine oder mehrere nicht benachbarte und nicht an Stickstoff gebundene CH₂-Gruppen durch -O-, -S-, -CO-O-, -O-CO-, -O-CO-O- oder -Si(CH₃)₂ ersetzt sein können und/oder
b2) eine oder mehrere CH₂-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
b3) ein oder mehrere H-Atome durch F und/oder Cl ersetzt sein können und/oder
b4) R⁵ und R⁶ zusammen auch einen Ring bilden können;
c) Phenyl, Biphenyl, 1-Naphthyl, 2-Naphthyl, 2-Thienyl, 2-Furanyl;
R⁷, R⁸, R⁹, R¹⁰, R¹¹, R¹² sind gleich oder verschieden
a) Wasserstoff, -CN, -F, NO₂ oder -Cl
b) ein geradkettiger oder verzweigter Alkylrest mit 1 bis 20 C-Atomen, wobei
b1) eine oder mehrere nicht benachbarte CH₂-Gruppen durch -O-, -S-, -CO-O-, -O-CO-, -O-CO-O-, -NR⁵ oder -Si(CH₃)₂- ersetzt sein können und/oder
b2) eine oder mehrere CH₂-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
b3) ein oder mehrere H-Atome durch F und/oder Cl ersetzt sein können;
c) Phenyl, Biphenyl, 1-Naphthyl, 2-Naphthyl, 2-Thienyl, 2-Furanyl, -O-Phenyl, -O-Biphenyl, -O-1-Naphthyl, -O-2-Naphthyl, -O-2-Thienyl, -O-2-Furanyl,
m, n, p, q, r sind jeweils gleich oder verschieden 0, 1, 2, 3, 4, 5 oder 6.

2. Spiroverbindung gemäß Anspruch 1, **dadurch gekennzeichnet, daß** es sich um eine Spirobifluorenderivat der Formeln (II) a-c handelt 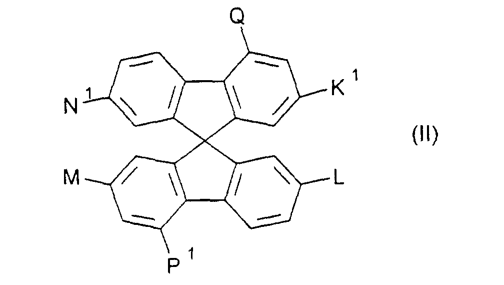 wobei die Symbole folgende Bedeutungen haben:
II.a) K¹ = L = M = N¹ und ist aus der Gruppe: 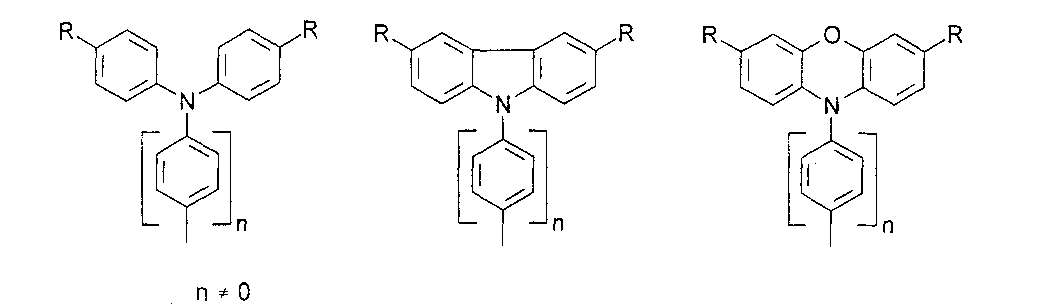 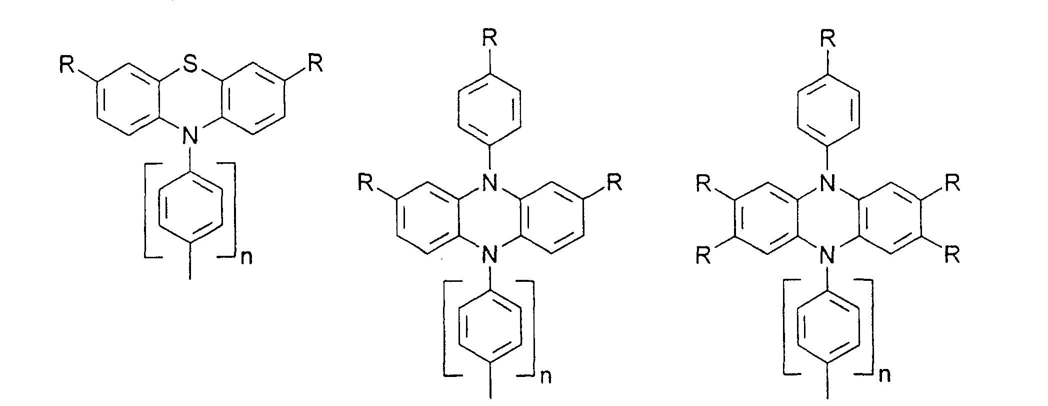 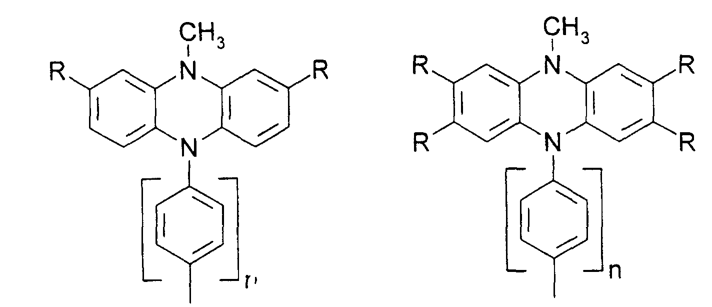 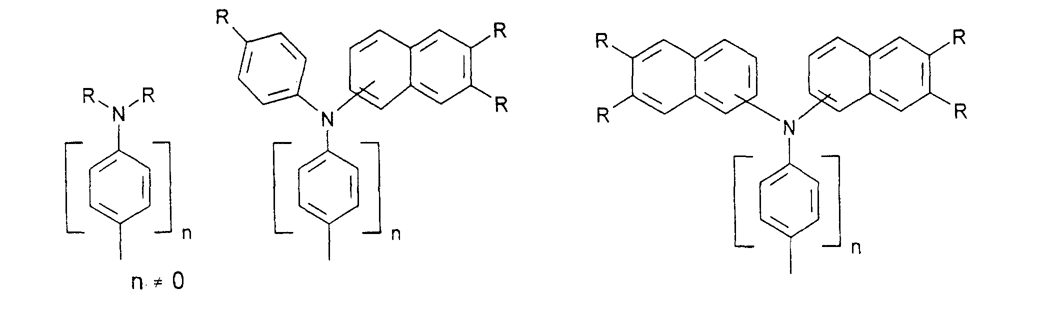 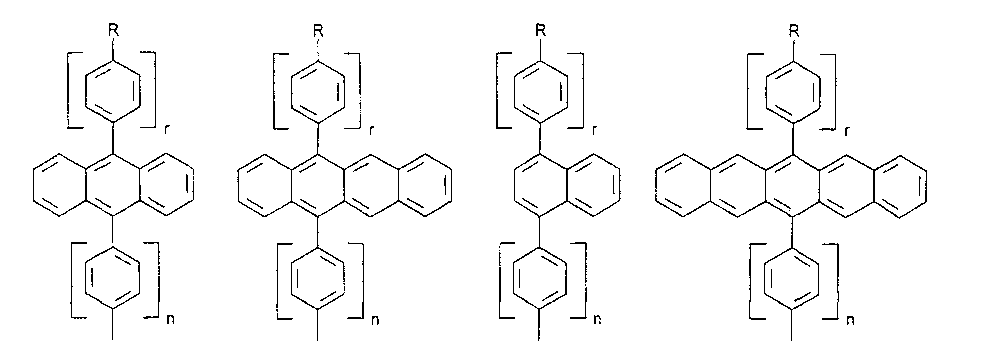
R gleich oder verschieden H, Alkyl, -O-Alkyl, -S-Alkyl, mit jeweils 1 bis 20 C-Atomen, vorzugsweise 1 bis 4 C-Atomen, Phenyl, Biphenyl, 1-Naphthyl, 2-Naphthyl, 2-Thienyl, 2-Furanyl, -O-Phenyl, -O-Biphenyl, -O-1-Naphthyl, -O-2-Naphthyl, -O-2-Thienyl, -O-2-Furanyl, CN, NR₂, wobei -O-Alkyl/Aryl, -S-Alkyl/Aryl, CN, NR₂ nicht an Stickstoff gebunden sein darf;
n = 0,1,2,3,4;
und Q, P¹ sind gleich oder verschieden und aus der Gruppe
H, COOR, CH₂OR, 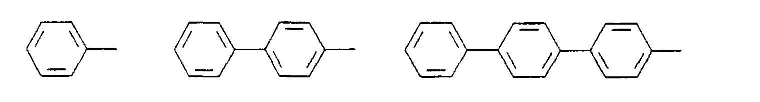 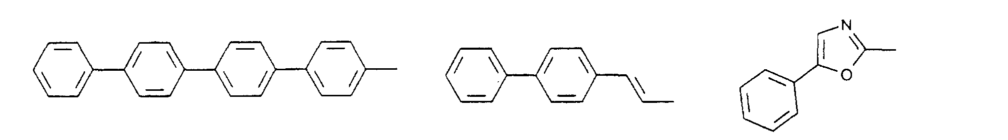 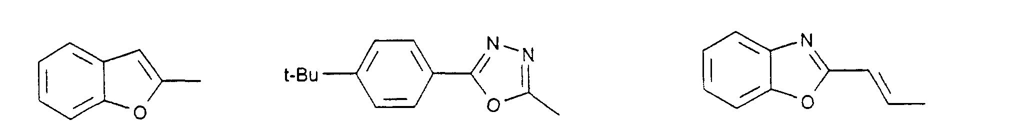 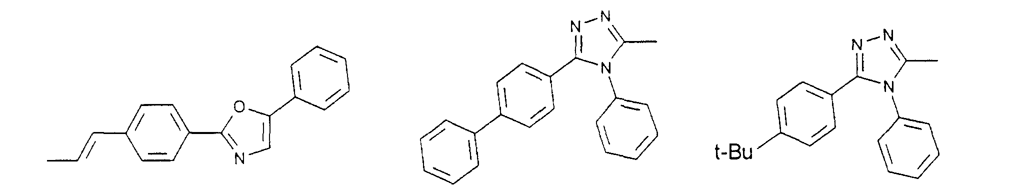 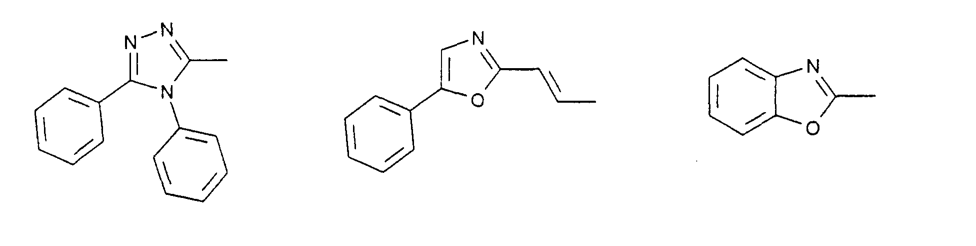 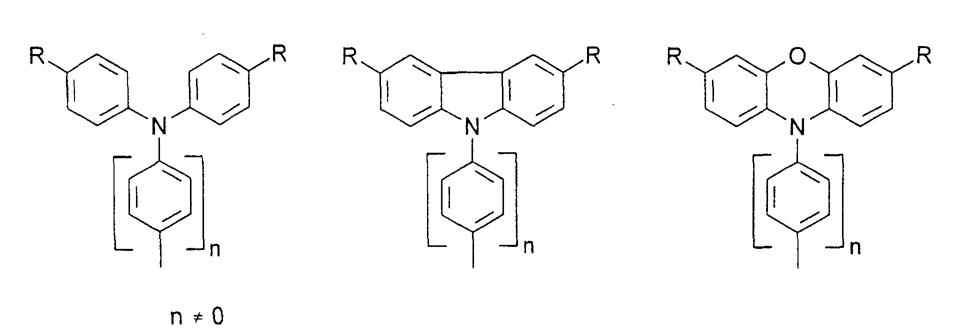 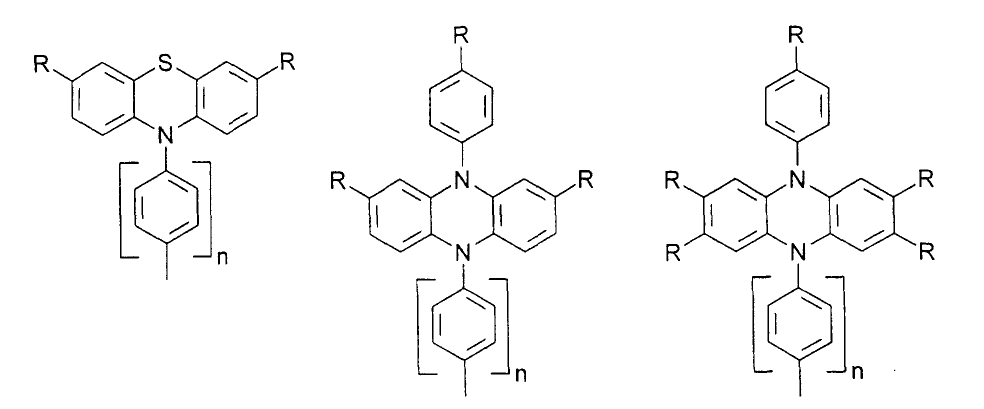 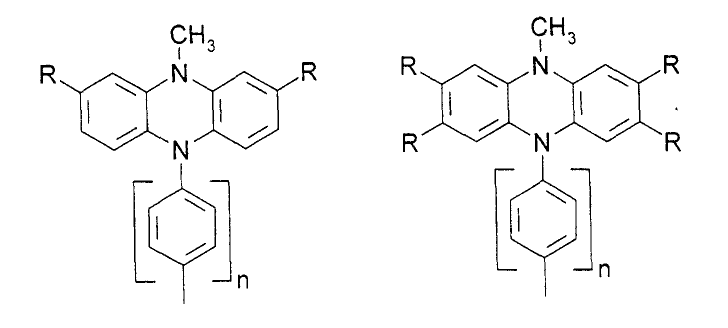 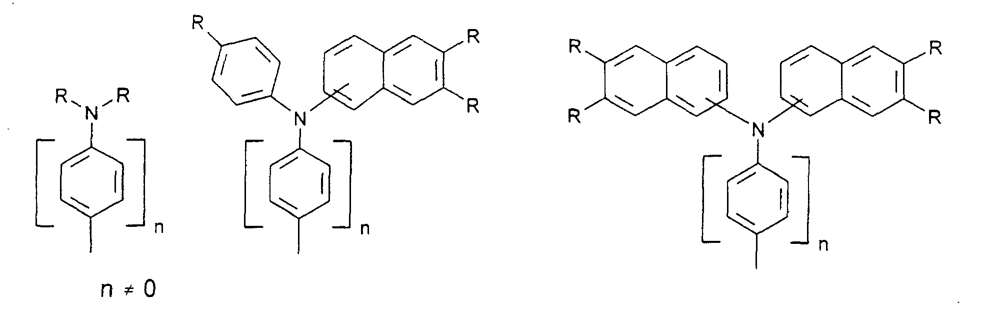 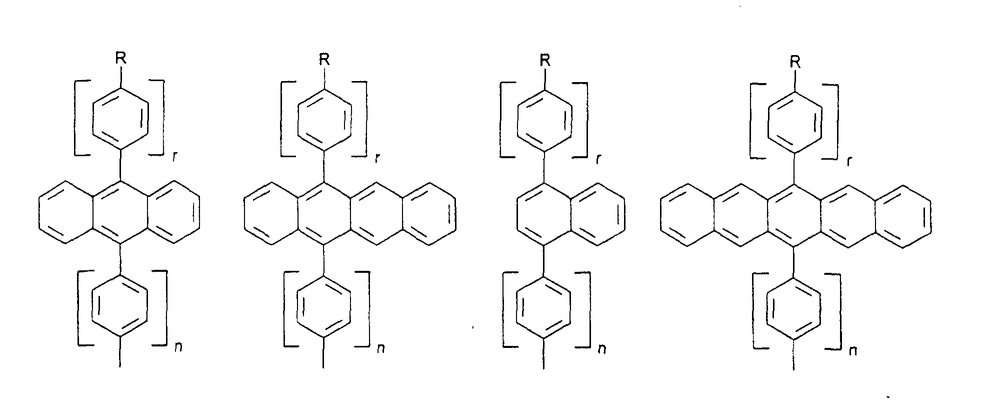 wobei die Symbole und Indizes die oben angegebenen Bedeutungen haben
II.b) K¹ = N¹ und ist aus der Gruppe 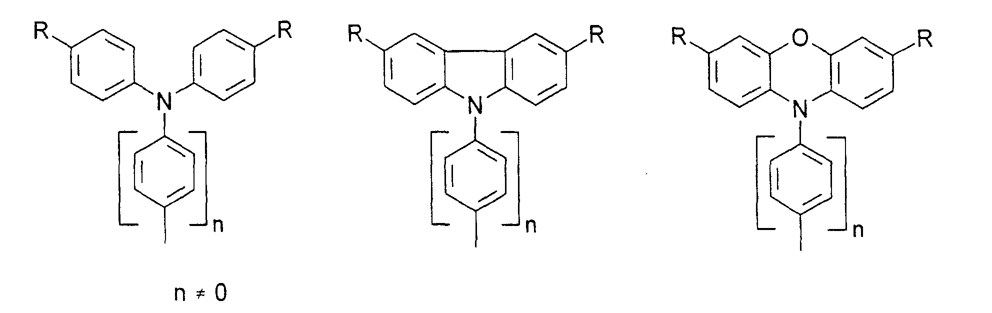 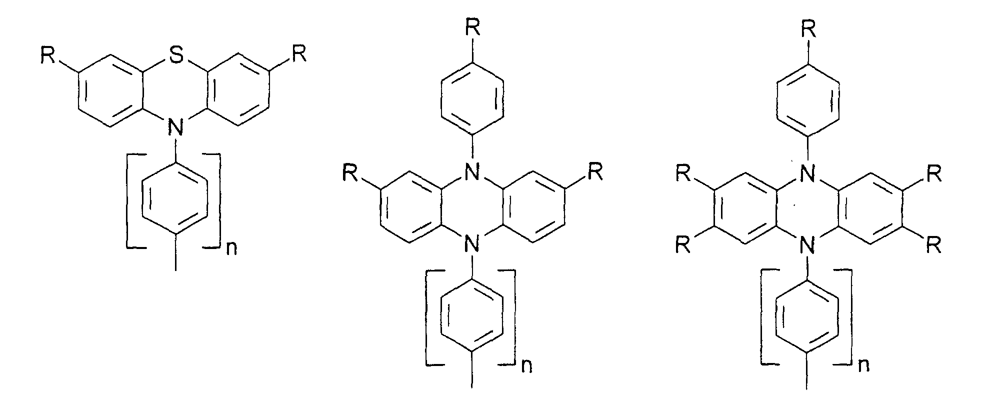 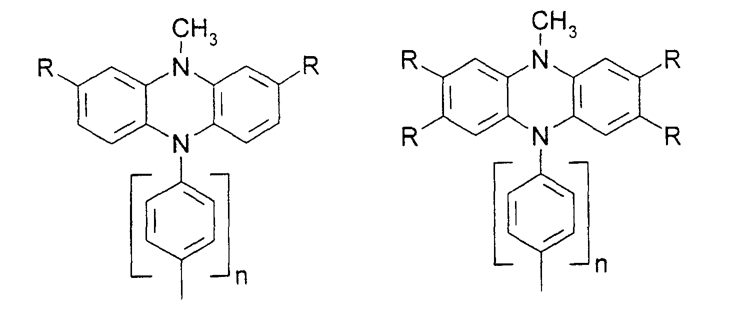 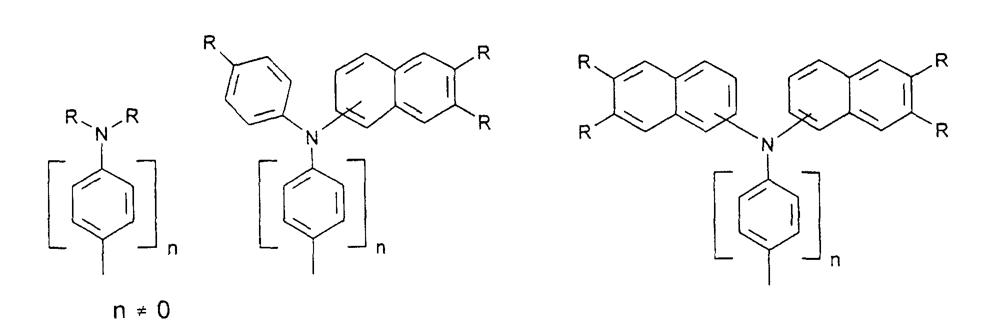 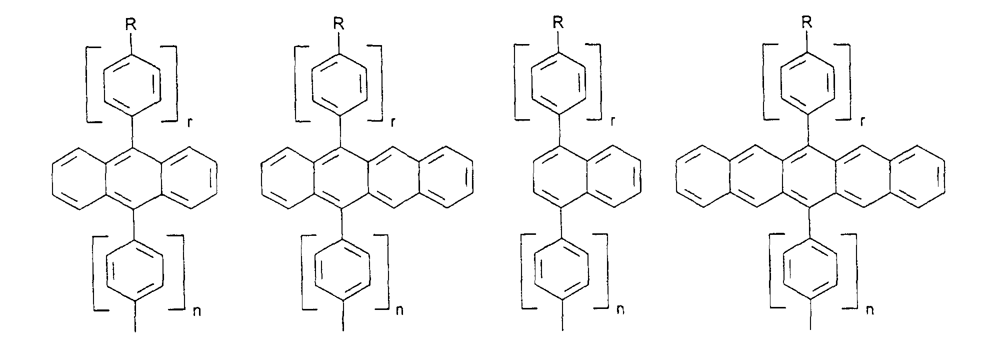 und L = M und ist aus der Gruppe
H, COOR, CH₂OR, 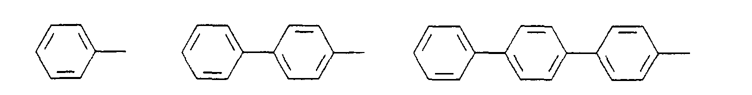 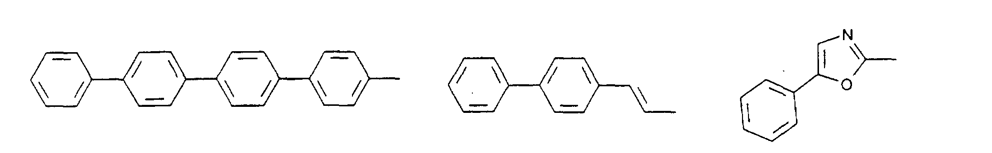 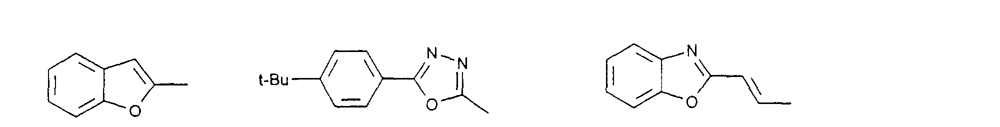 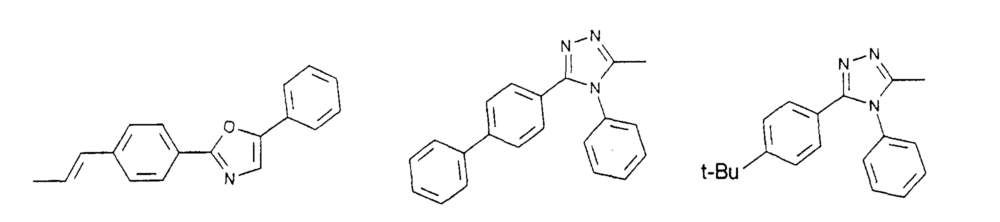 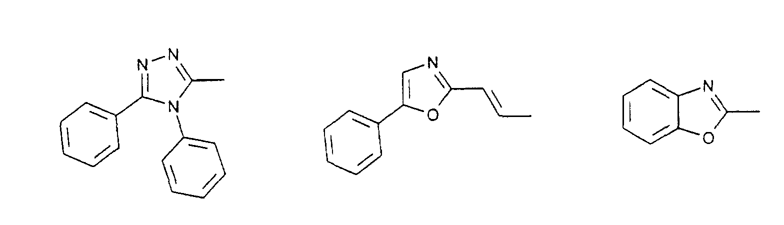 und Q, P¹ sind gleich oder verschieden aus der Gruppe 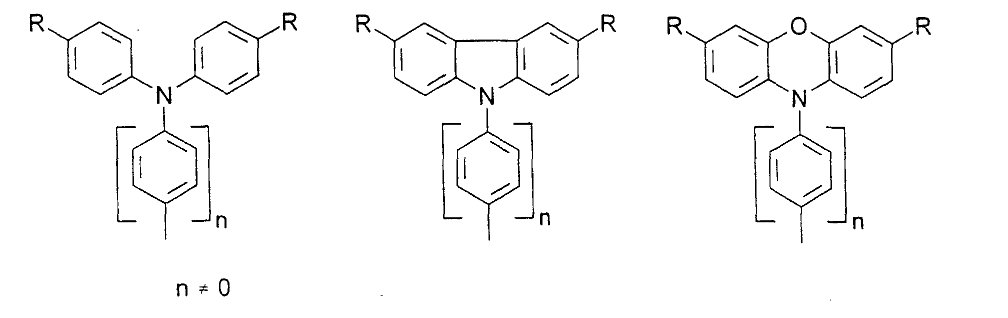 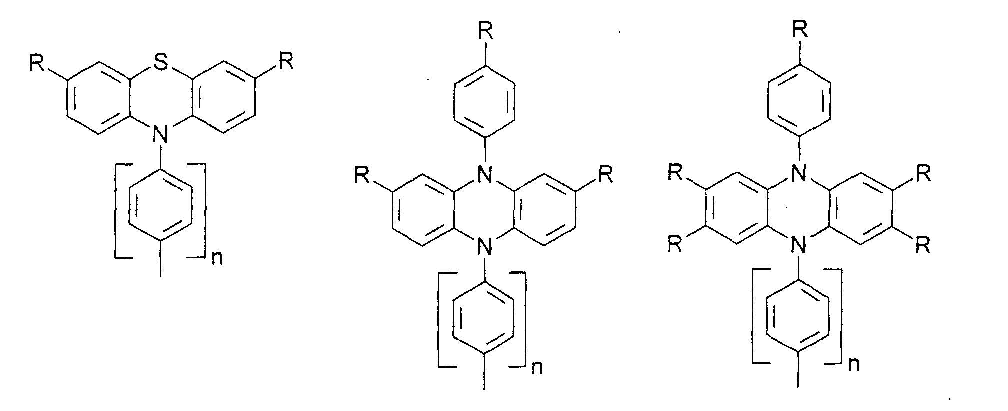 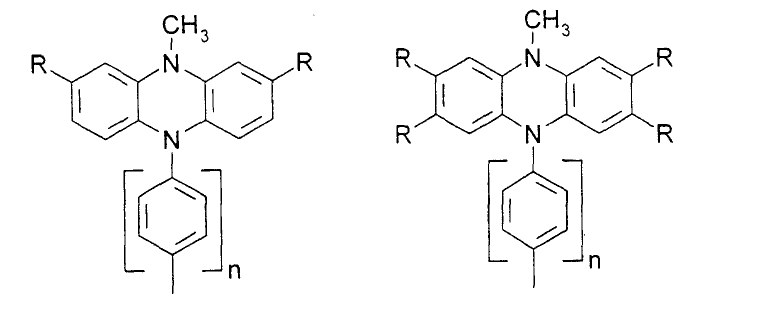 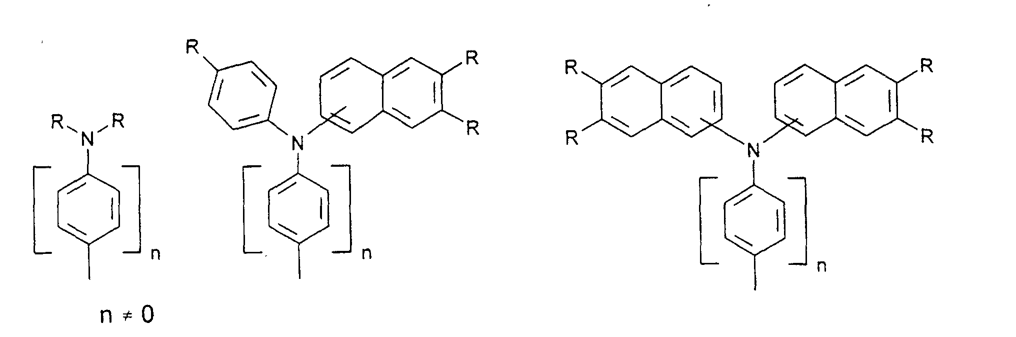 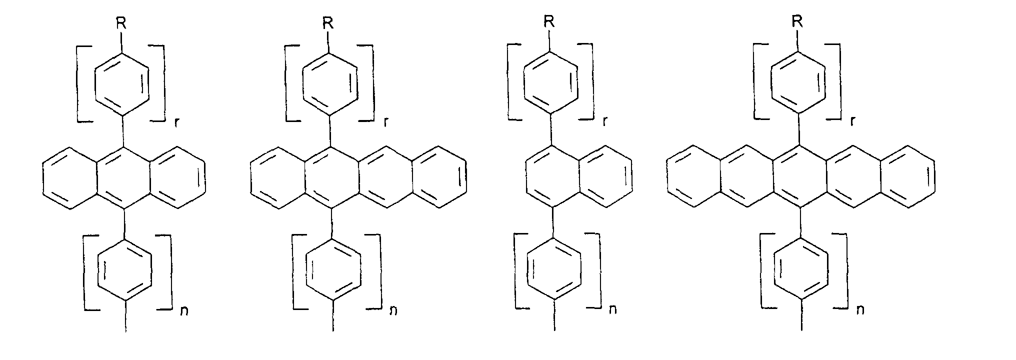 H, COOR, CH₂OR, 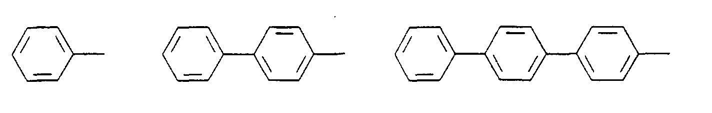 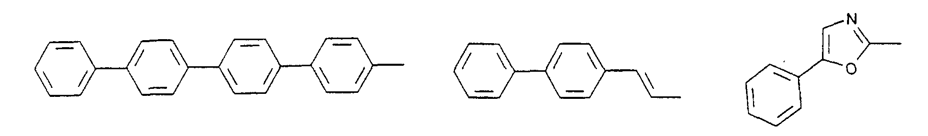 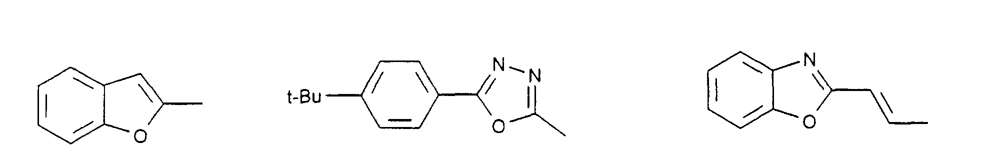 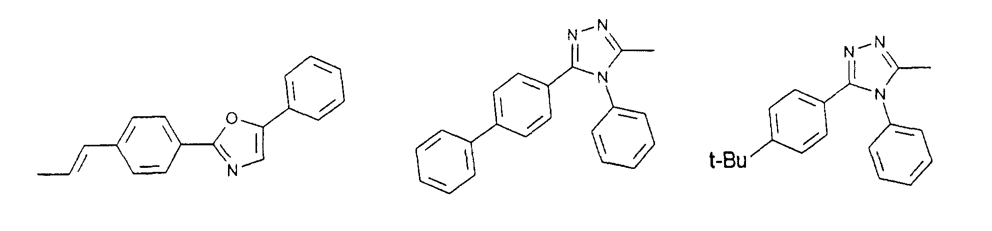 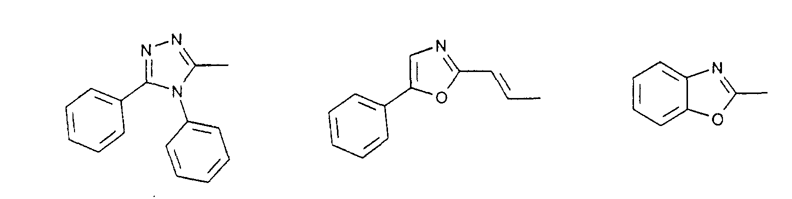 wobei die Symbole und Indizes die oben angegebenen Bedeutungen haben,
llc) K¹ = M und ist aus der Gruppe 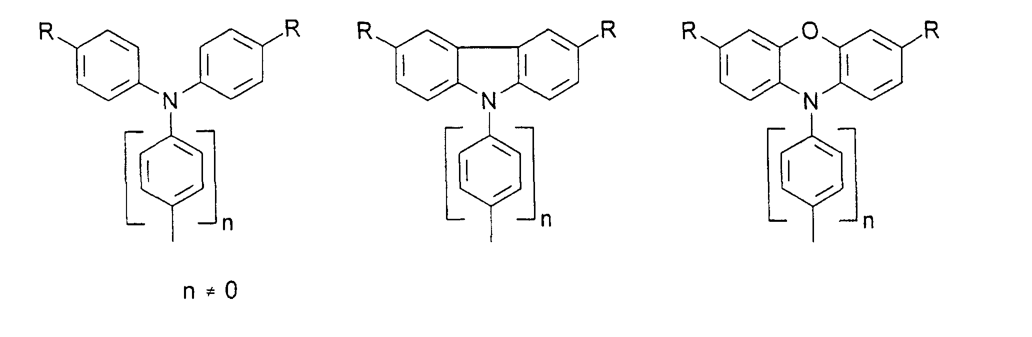 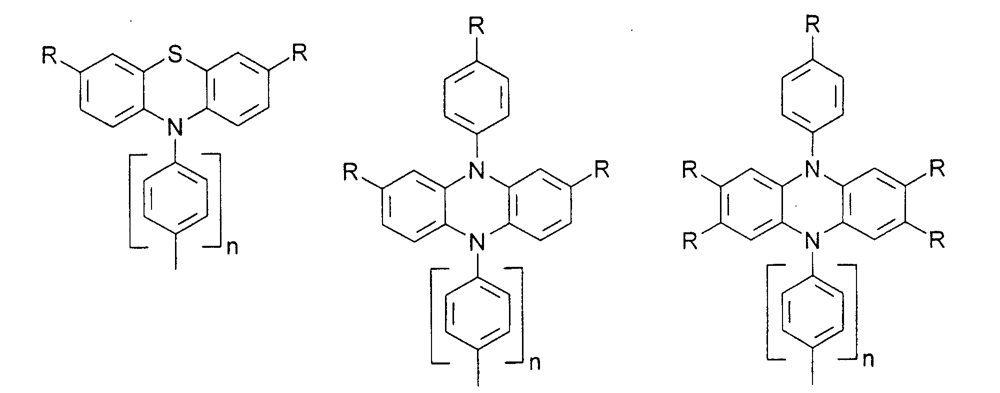 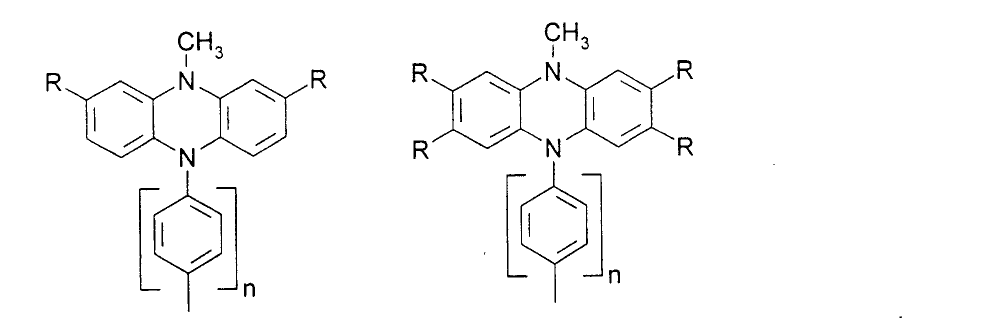 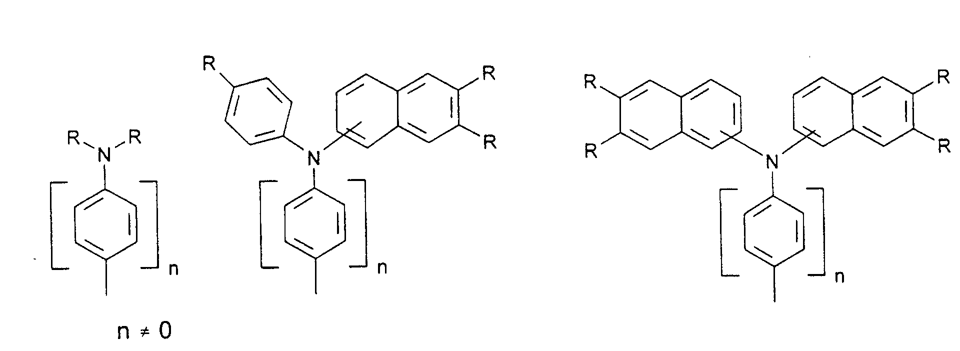 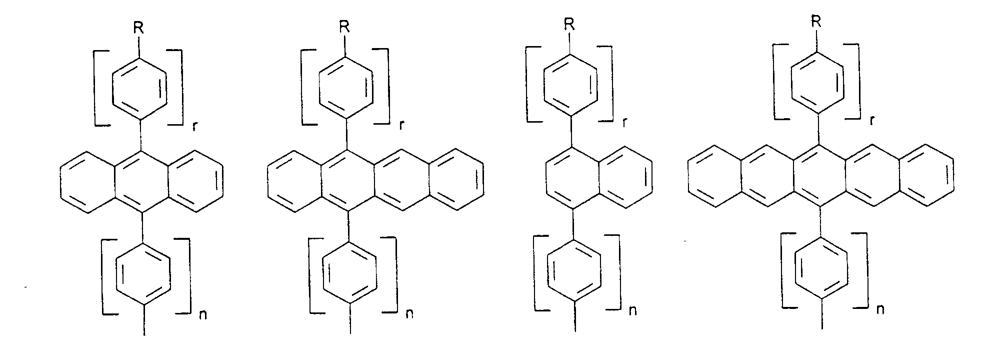 und M = N¹ ist und aus der Gruppe
H, COOR, CH₂OR, 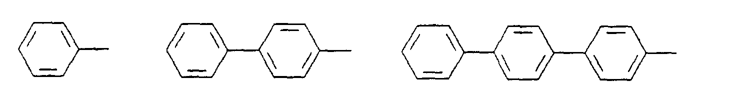 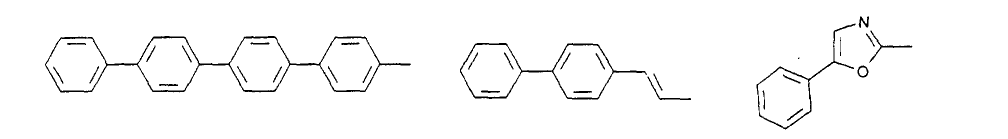 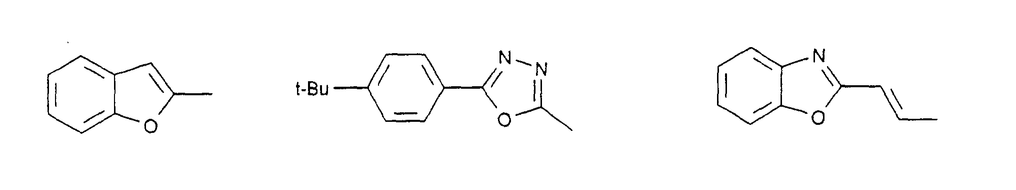 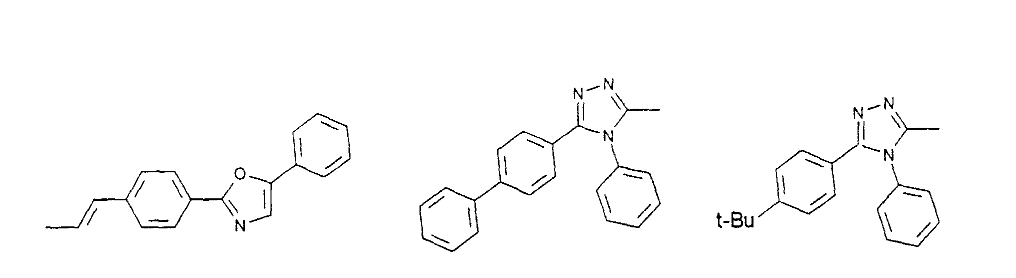 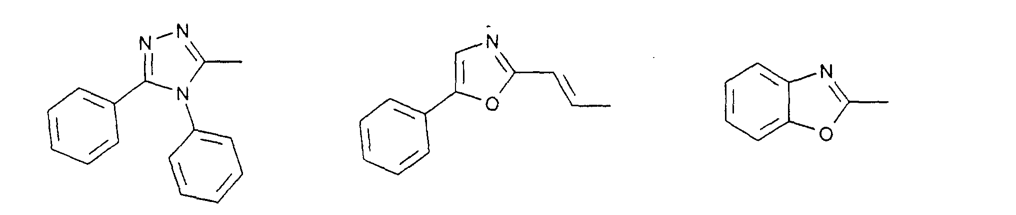 und Q, P¹ sind gleich oder verschieden aus der Gruppe 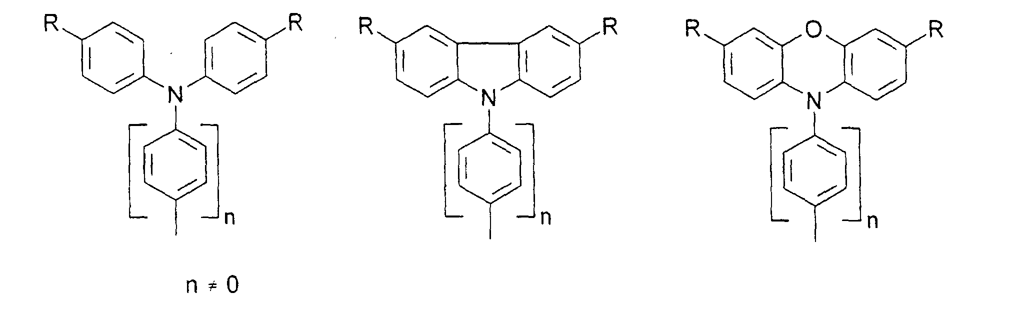 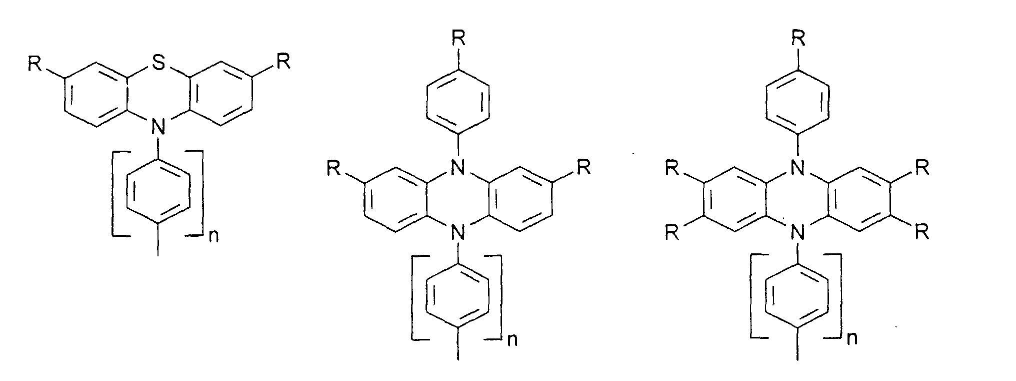 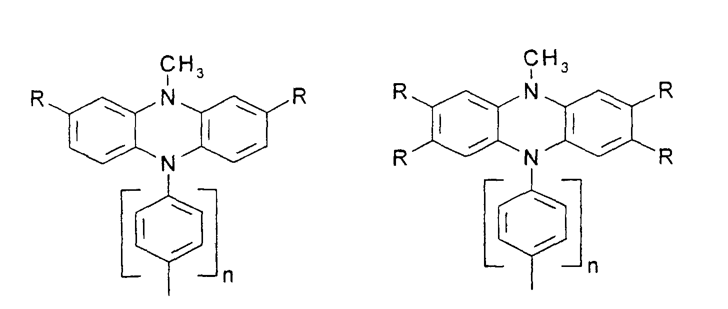 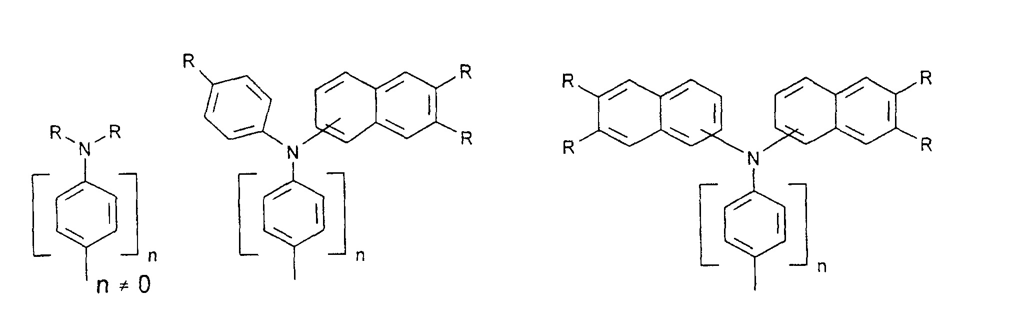 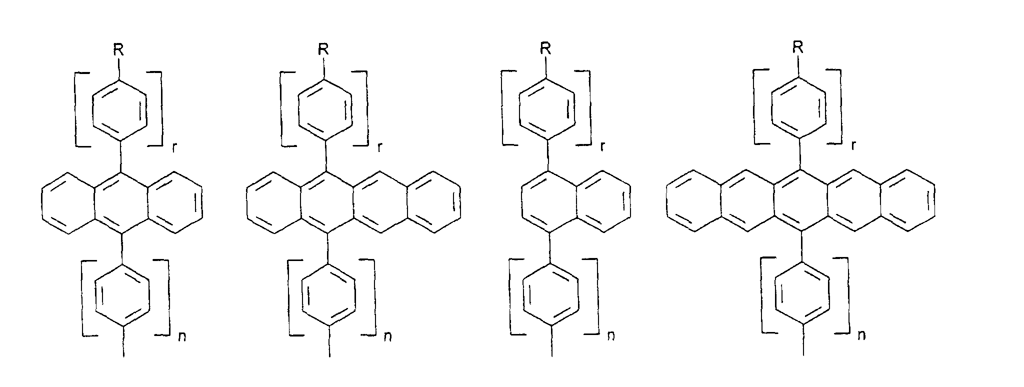 H, COOR, CH₂OR, 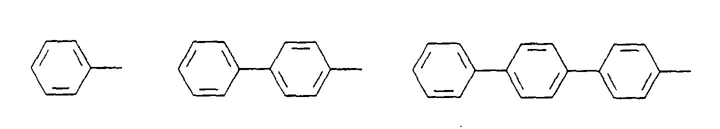 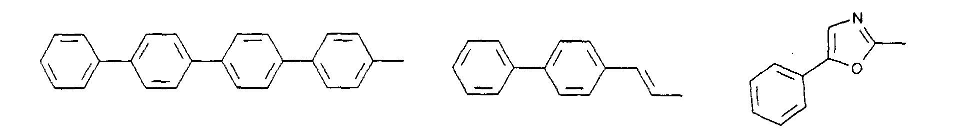 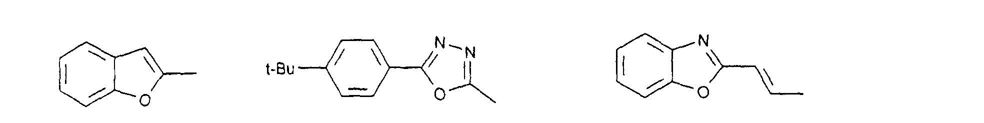 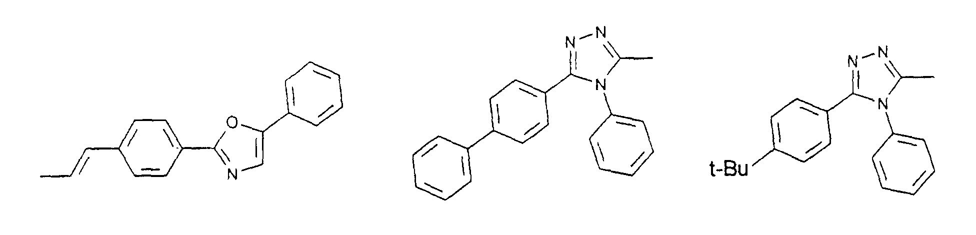 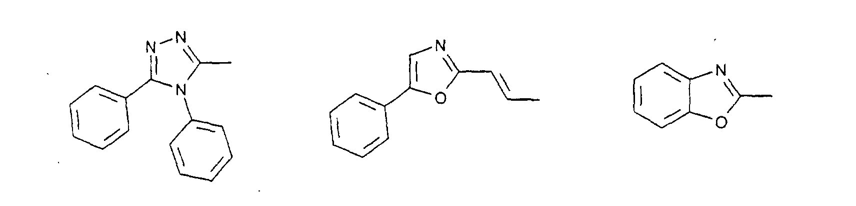 wobei die Symbole und Indizes die oben angegebenen Bedeutungen haben.

3. Spiroverbindung gemäß Anspruch 1 und/oder 2, aus der Gruppe (II)aa-(II)ac:
IIaa) K¹ = L = M = N¹ und ist aus der Gruppe: 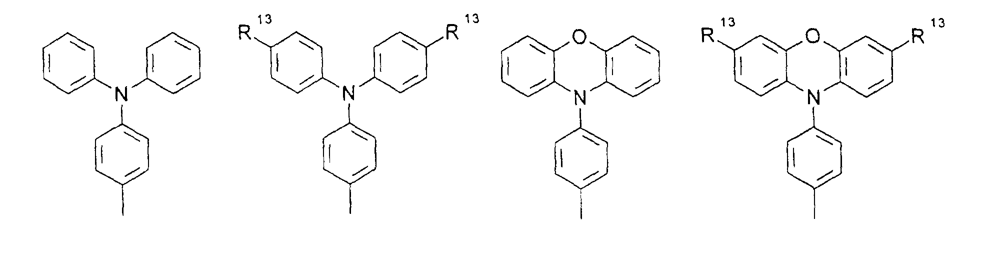 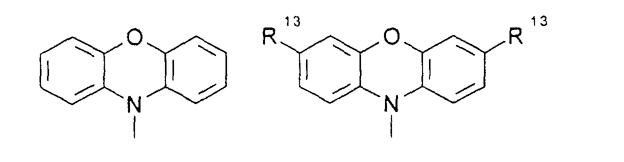 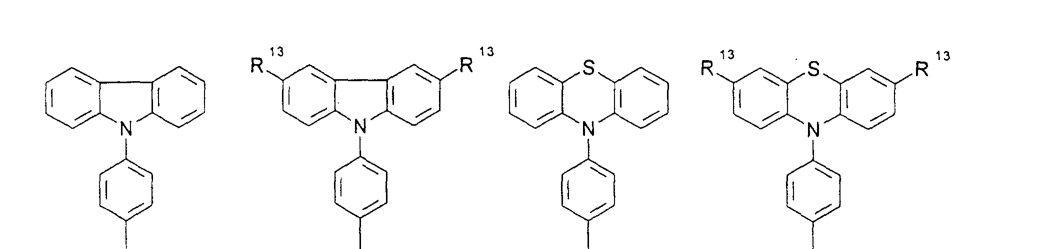 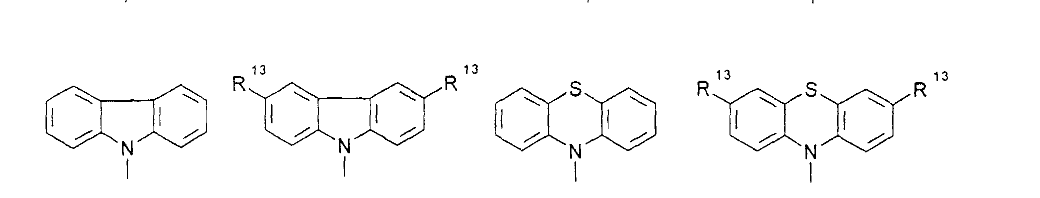 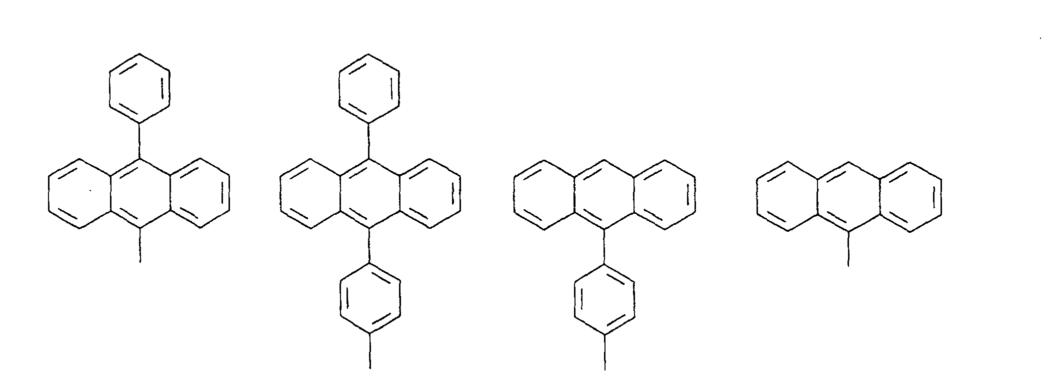 wobei R¹³ -O-CH₃, -O-C₂H₅, -S-CH₃, -S-C₂H₅ bedeutet;
und Q = P¹ und ist aus der Gruppe
H, COOR¹⁴, CH₂OR¹⁴, 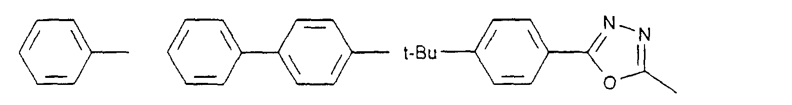 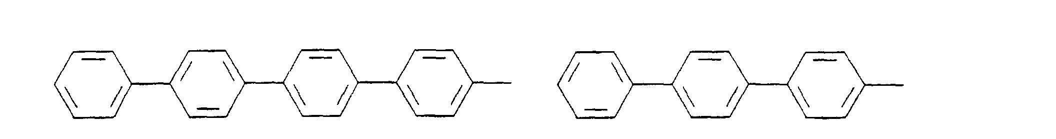 wobei R¹⁴ eine geradkettige oder verzweigte Alkylgruppe mit 1 bis 12 C-Atomen ist;
II.ba) K¹ = L = M = N¹ = Q = P¹ und ist aus der Gruppe 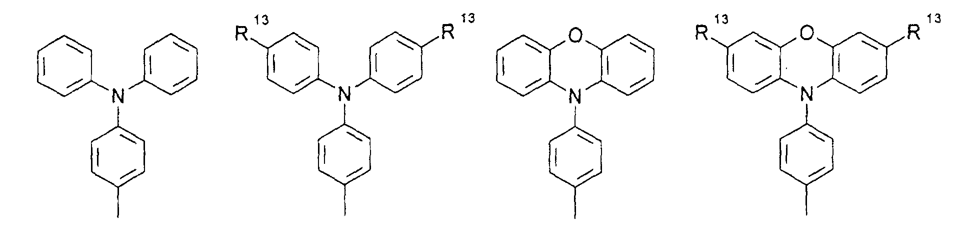 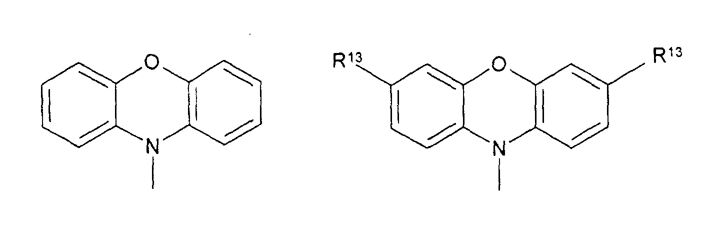 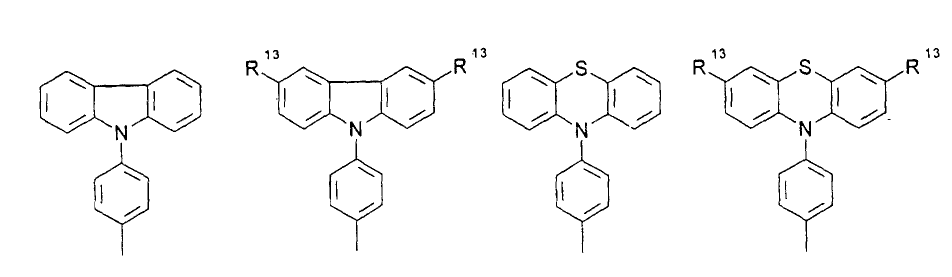 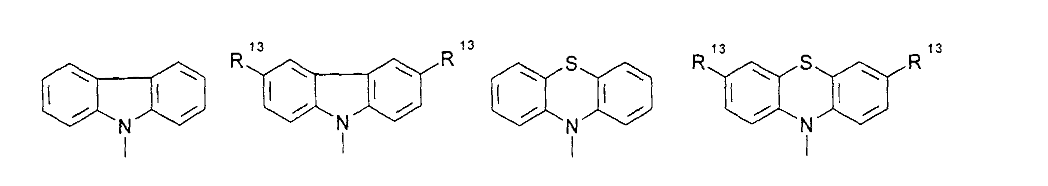 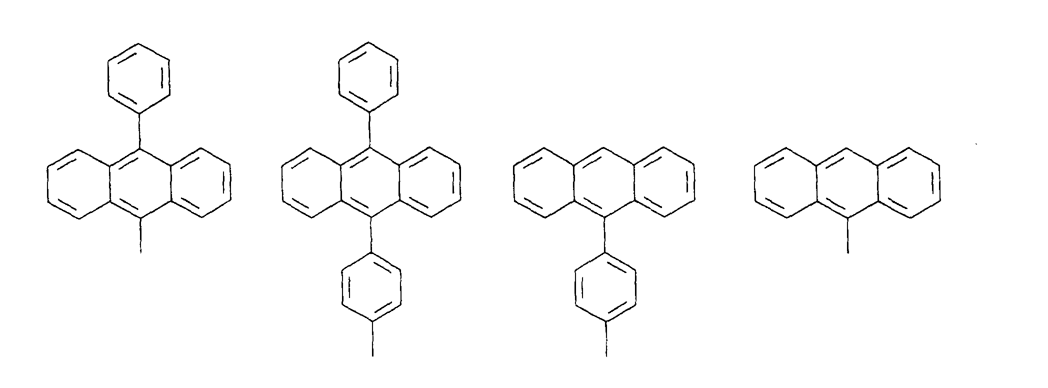 wobei R¹³ die oben angegebenen Bedeutungen hat;
II.ca) K¹ = L = M = N¹ und ist aus der Gruppe 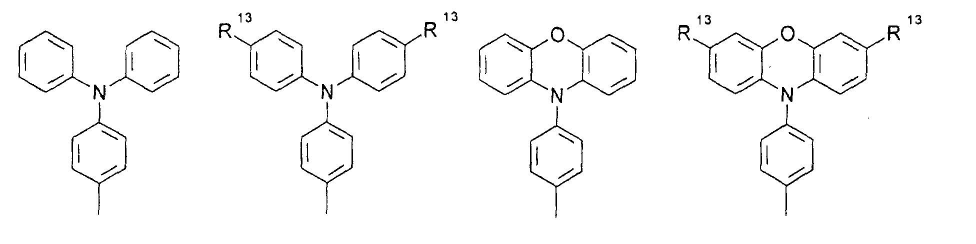 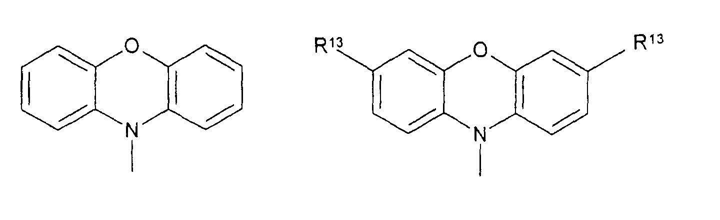 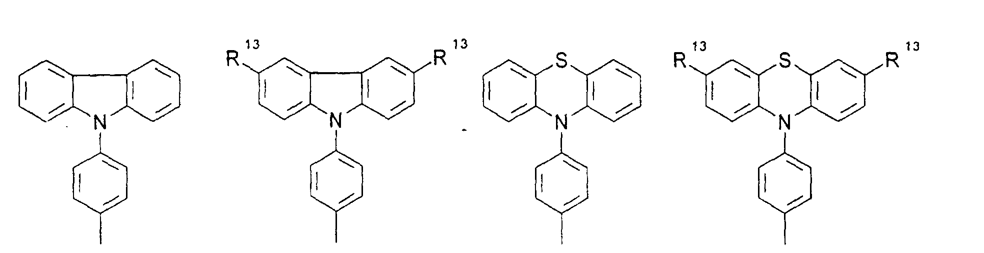 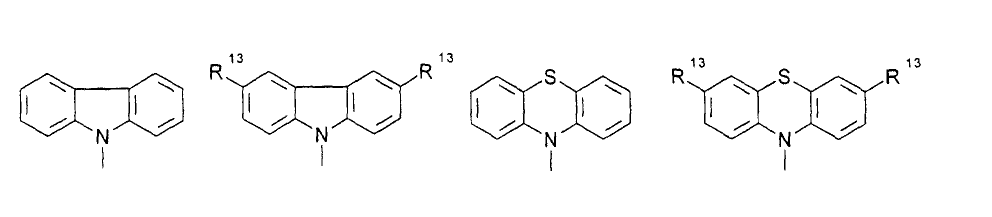 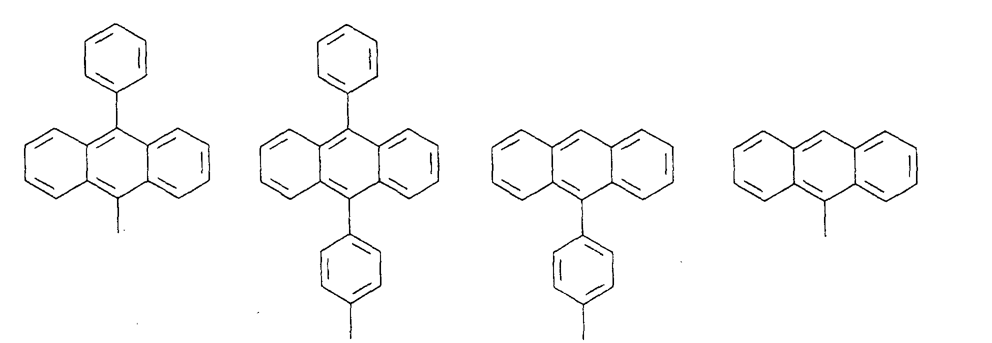 und Q = H und P¹ ist aus der Gruppe
H, COOR¹⁴, CH₂OR¹⁴, 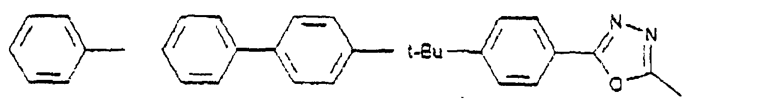 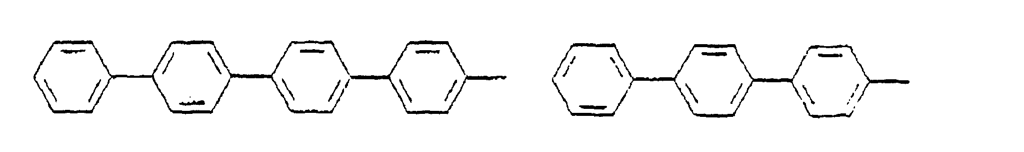 wobei R¹³, R¹⁴ die oben angegebenen Bedeutungen haben.

4. Verwendung einer amorphen Spiroverbindung gemäß einem oder mehreren der Ansprüche 1 bis 3 als Ladungstransportmaterial in photovoltaischen Zeilen.

5. Photovoltaische Zelle, enthaltend einer Ladungstransportschicht, die eine oder mehrere amorphe Spiroverbindungen gemäß einer oder mehreren der Ansprüche 1 bis 3 enthält

## Claims

1. A spiro compound of the formula (I) 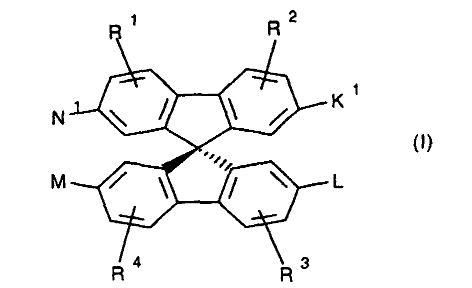 where the symbols have the following meanings:
K¹, L, M, N¹, R¹, R², R³, R⁴ are identical or different and are each
a) hydrogen, -NO₂, -CN, -F or -Cl,
b) a straight-chain or branched alkyl radical having from 1 to 20 carbon atoms, where
b1) one or more nonadjacent CH₂ groups can be replaced by -O-, -S-, -CO-O-, -O-CO-, -O-CO-O-, NR⁵ or -Si(CH₃)₂- and/or
b2) one or more CH₂ groups can be replaced by - CH=CH-, -C≡C-, 1,4-phenylene, 1,4-cyclohexylene or 1,3-cyclopentylene and/or
b3) one or more H atoms can be replaced by F and/or Cl, and/or
c) one of the following groups: 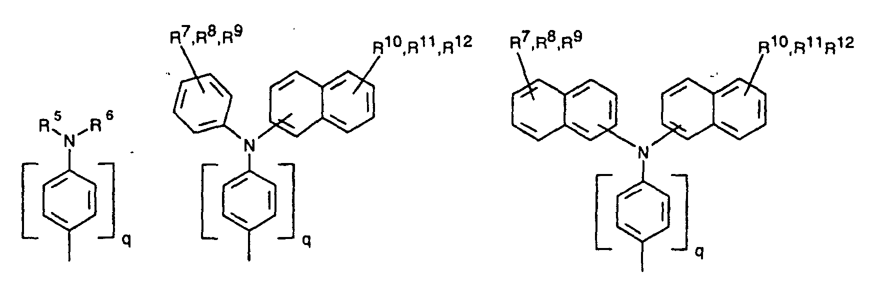 where q ≠ 0 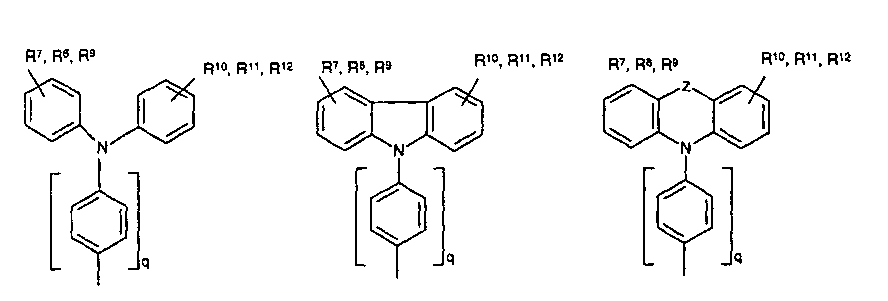 where q ≠ 0 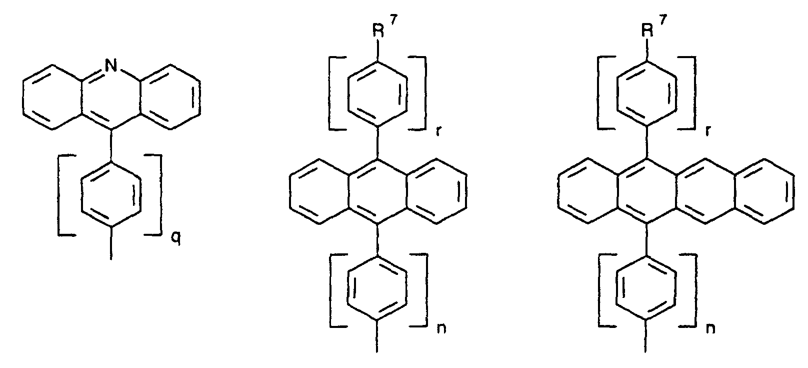 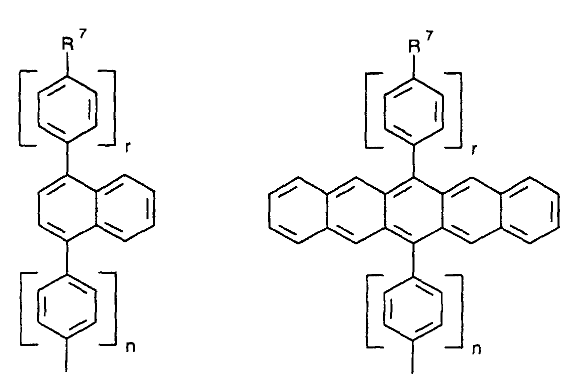 or
d) one of the following groups: 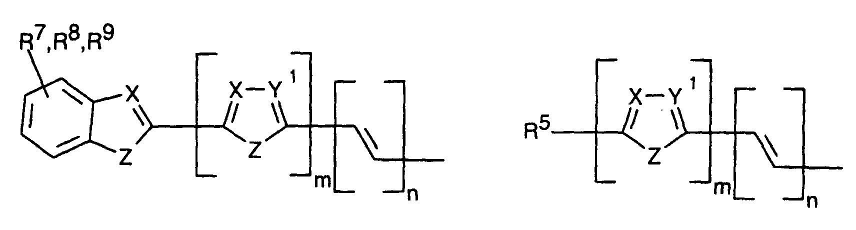 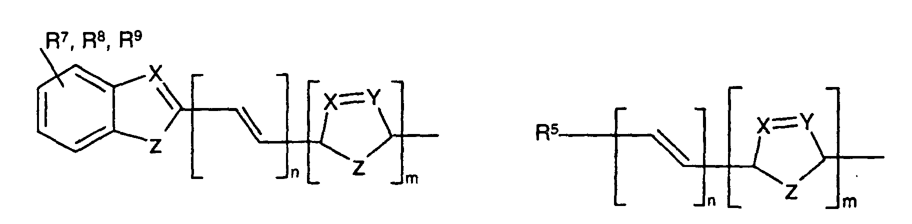 with the proviso that at least one of the radicals K¹, L, M, N¹, R¹, R², R³, R⁴ is one of the groups listed under c);
X, Y¹ are in each case identical or different and are =CR⁷- or =N-;
Z is -O-, -S-, -NR⁵-, -CRR-, -CR=CR- or -CR=N-;
R⁵,R⁶ are in each case identical or different and are each
a) hydrogen,
b) a straight-chain or branched alkyl radical having from 1 to 20 carbon atoms, where
b1) one or more nonadjacent CH₂ groups which are not bound to nitrogen can be replaced by -O-, -S-, -CO-O-, -O-CO-, -O-CO-O- or -Si(CH₃)₂- and/or
b2) one or more CH₂ groups can be replaced by -CH=CH-, -C≡C-, cyclopropane-1,2-diyl, 1,4-phenylene, 1,4-cyclohexylene or 1,3-cyclopentylene and/or
b3) one or more H atoms can be replaced by F and/or Cl and/or
b4) R⁵ and R⁶ together can also form a ring;
c) phenyl, biphenyl, 1-naphthyl, 2-naphthyl, 2-thienyl, 2-furanyl;
R⁷, R⁸, R⁹, R¹⁰, R¹¹, R¹² are identical or different and are each
a) hydrogen, -CN, -F, NO₂ or -Cl,
b) a straight-chain or branched alkyl radical having from 1 to 20 carbon atoms, where
b1) one or more nonadjacent CH₂ groups can be replaced by -O-, -S-, -CO-O-, -O-CO-, -O-CO-O-, NR⁵ or -Si(CH₃)₂- and/or
b2) one or more CH₂ groups can be replaced by -CH=CH-, -C≡C-, cyclopropane-1,2-diyl, 1,4-phenylene, 1,4-cyclohexylene or 1,3-cyclopentylene and/or
b3) one or more H atoms can be replaced by F and/or Cl;
c) phenyl, biphenyl, 1-naphthyl, 2-naphthyl, 2-thienyl, 2-furanyl, -O-phenyl, -O-biphenyl, -O-1-naphthyl, -O-2-naphthyl, -O-2-thienyl, -O-2-furanyl,
m, n, p, q, r are in each case identical or different and are 0, 1, 2, 3, 4, 5 or 6.

2. A spiro compound as claimed in claim 1 which is a spirobifluorene derivative of one of the formulae (II) a-c 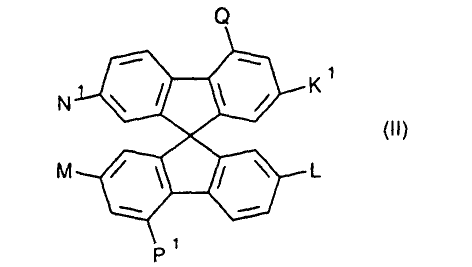 where the symbols have the following meanings:
II.a) K¹ = L = M = N¹ and is selected from the group consisting of: 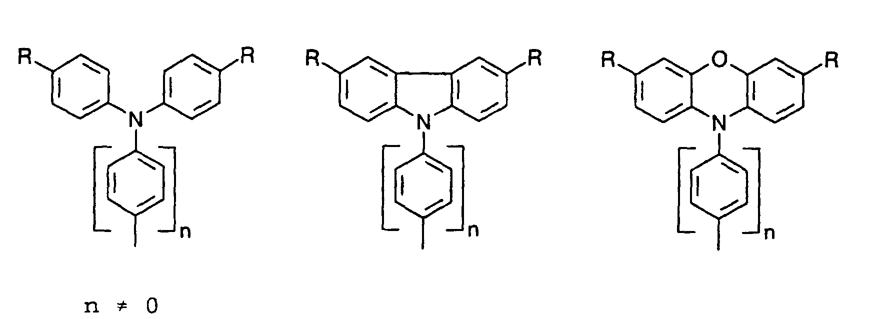 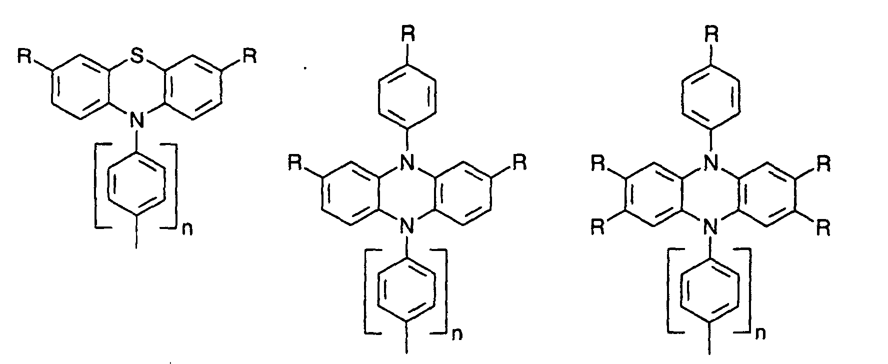 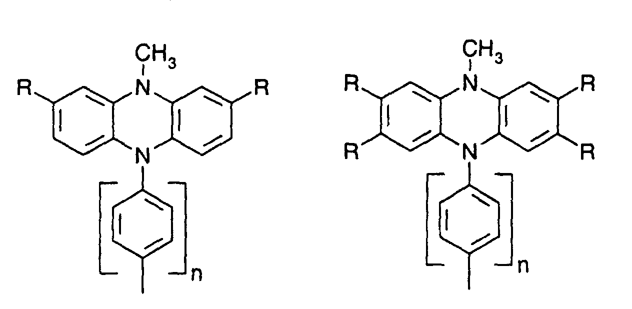 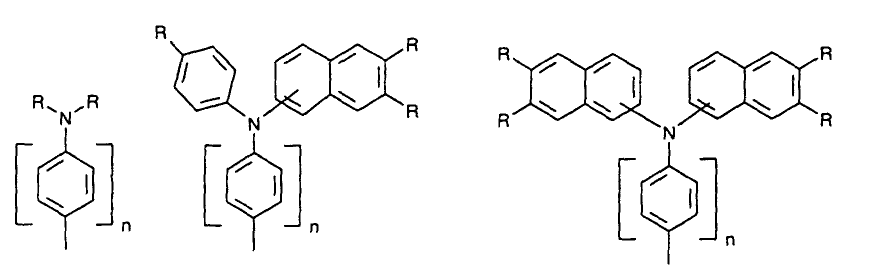 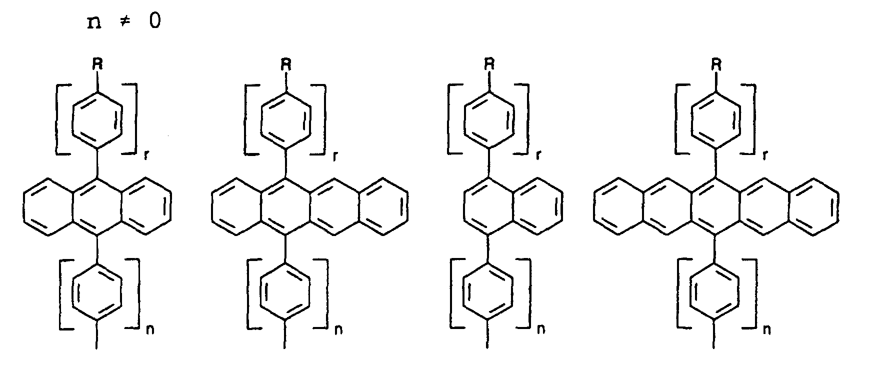
R are identical or different and are H, alkyl, -O-alkyl, -S-alkyl, each having from 1 to 20 carbon atoms, preferably from 1 to 4 carbon atoms, phenyl, biphenyl, 1-naphthyl, 2-naphthyl, 2-thienyl, 2-furanyl, -O-phenyl, -O-biphenyl, -O-1-naphthyl, -O-2-naphthyl, -O-2-thienyl, -O-2-furanyl, CN, NR₂, where -O-alkyl/aryl, -S-alkyl/aryl, CN, NR₂ must not be bound to nitrogen;
n = 0, 1, 2, 3, 4,
and Q, P¹ are identical or different and are selected from the group consisting of:
H, COOR, CH₂OR, 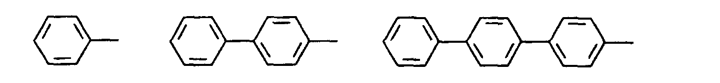 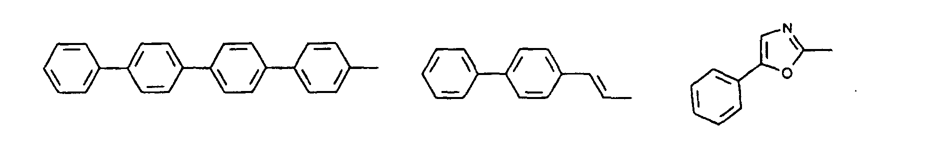 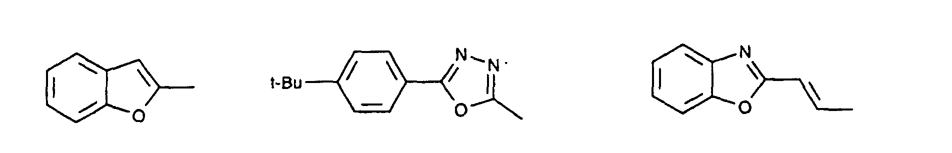 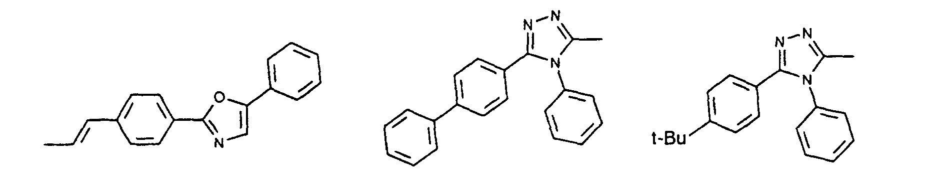 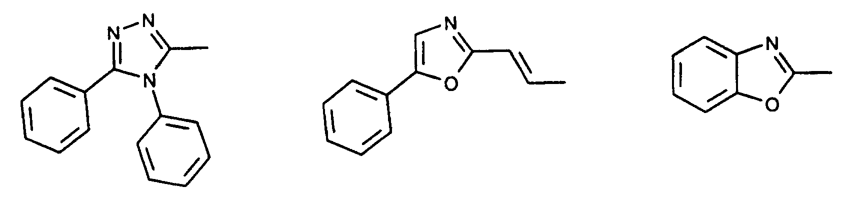 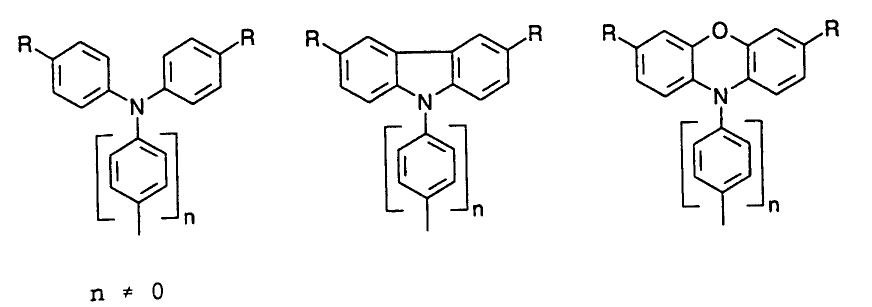 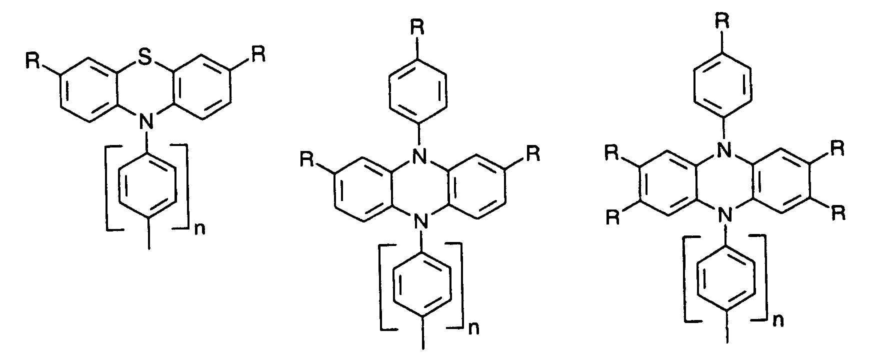 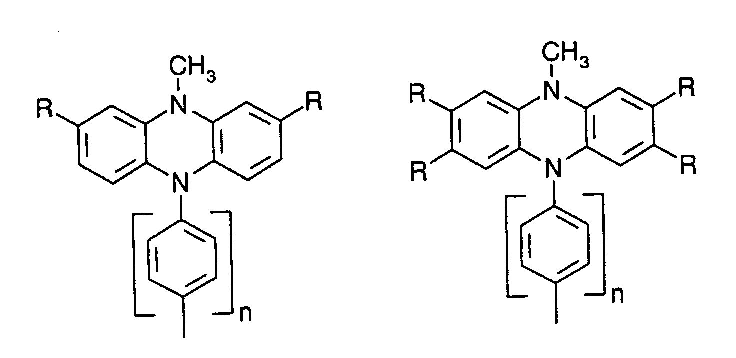 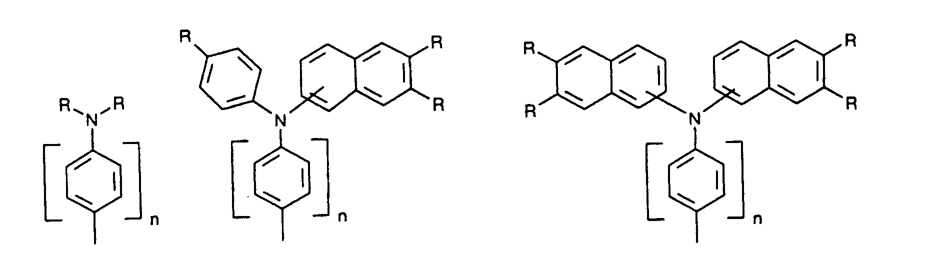 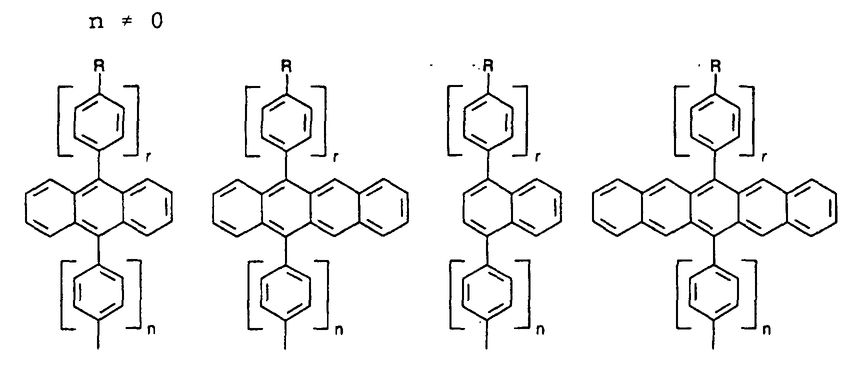 where the symbols and indices are as defined above,
II.b) K¹ = N¹ and is selected from the group consisting of: 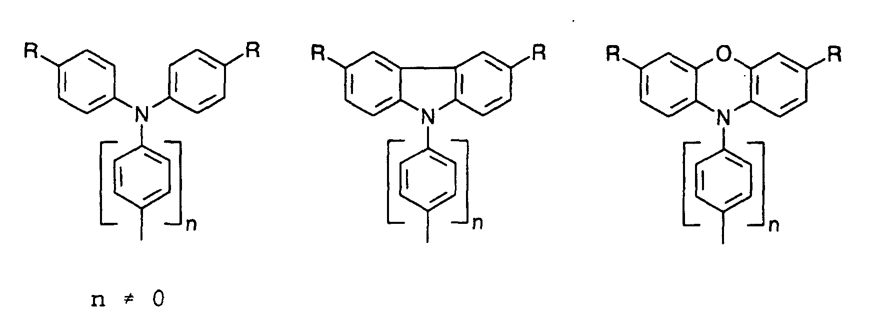 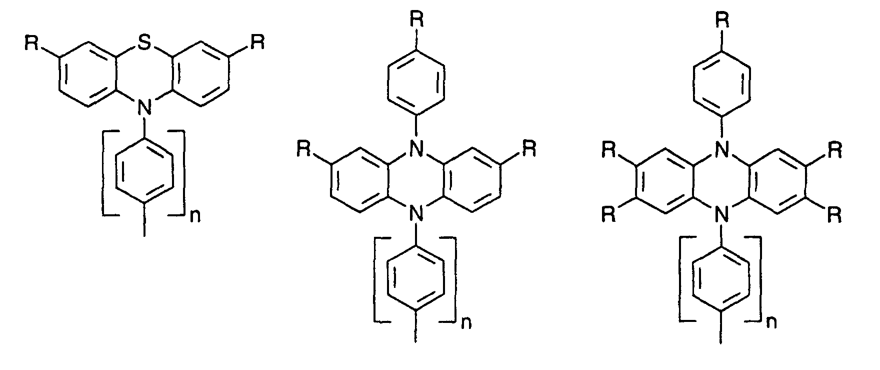 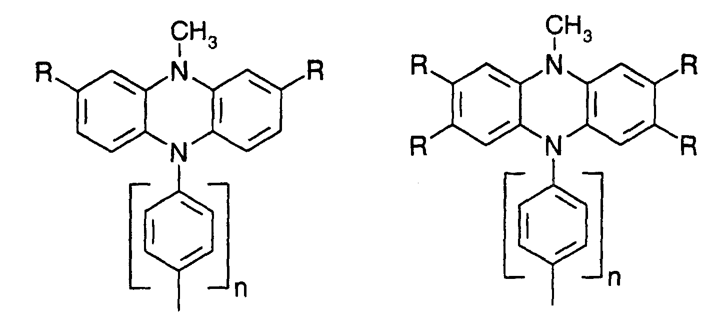 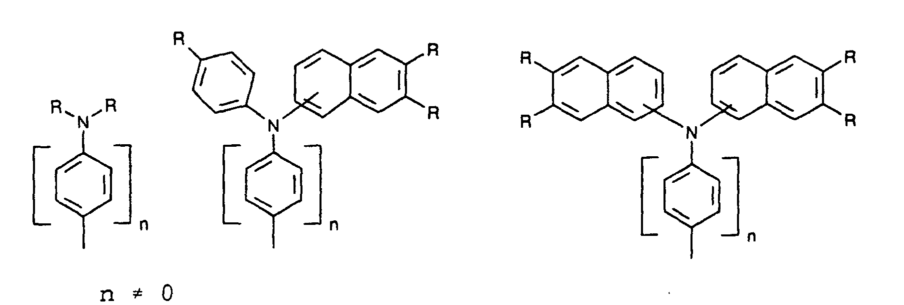 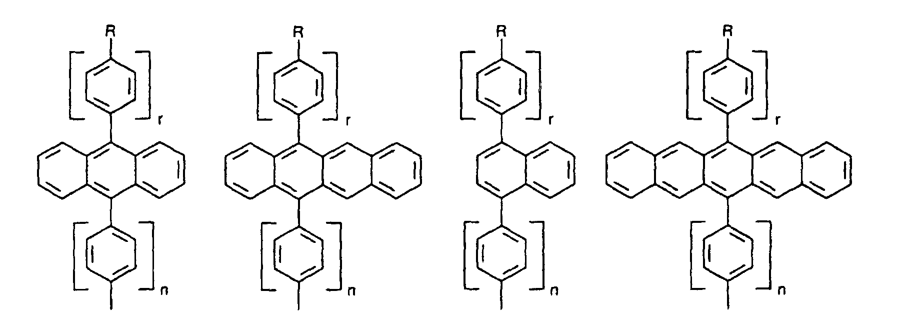 and L = M and is selected from the group consisting of:
H, COOR, CH₂OR, 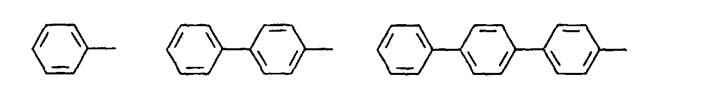 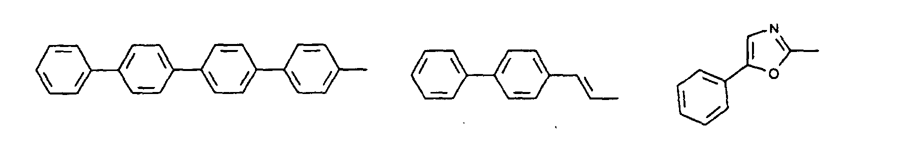 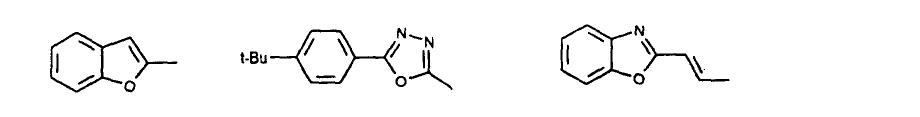 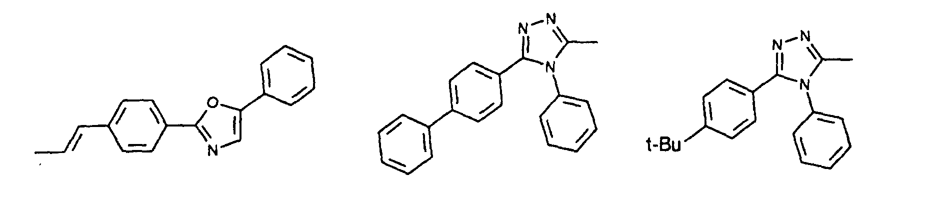 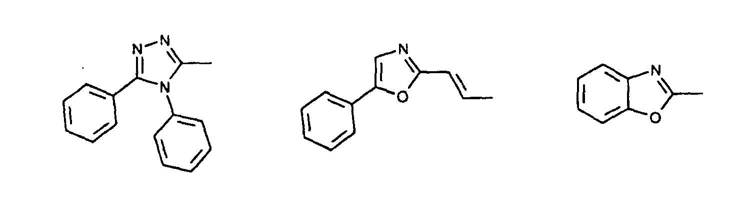 and Q, P¹ are identical or different and are selected from the group consisting of: 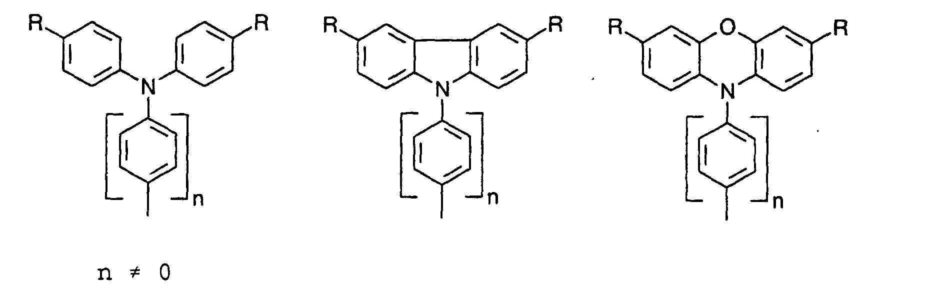 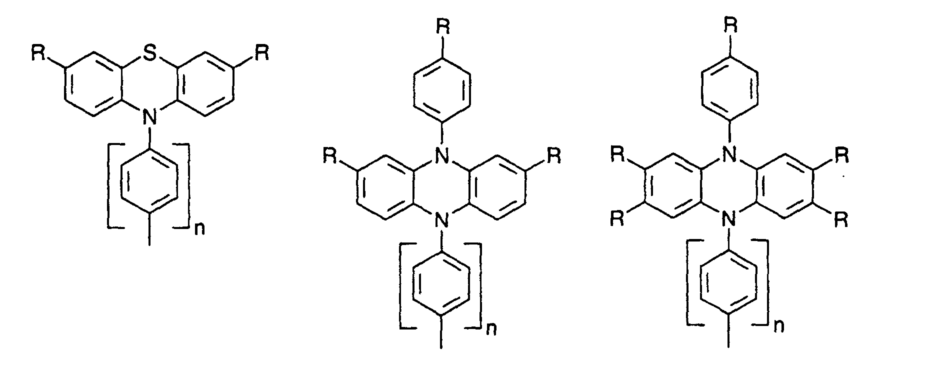 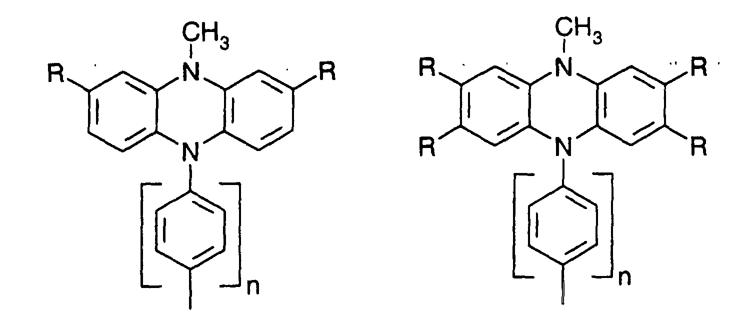 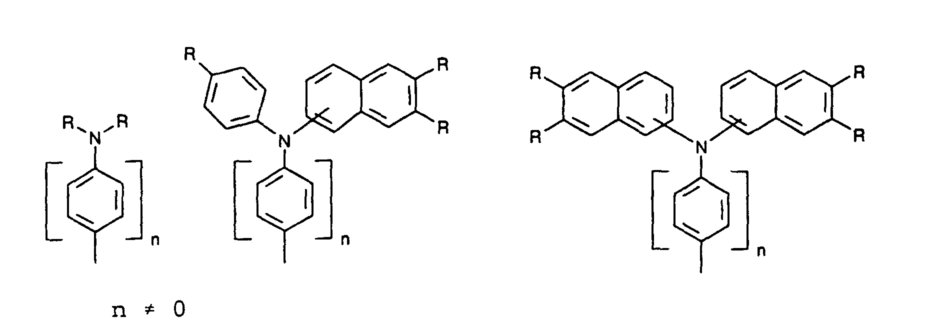 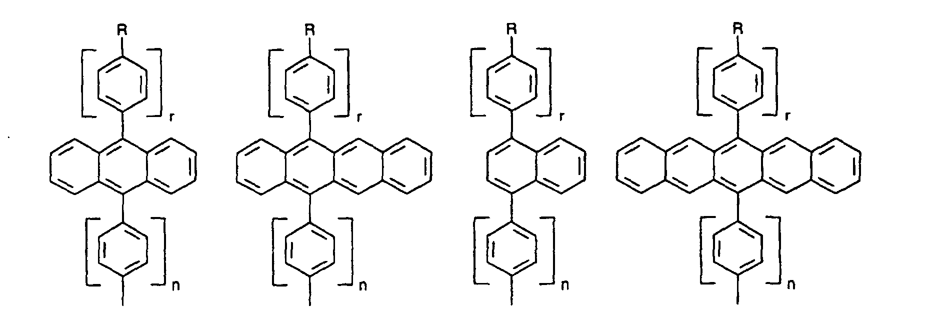 H, COOR, CH₂OR, 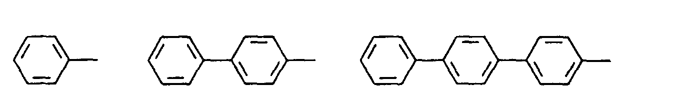 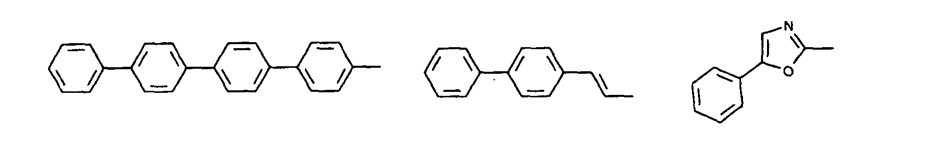 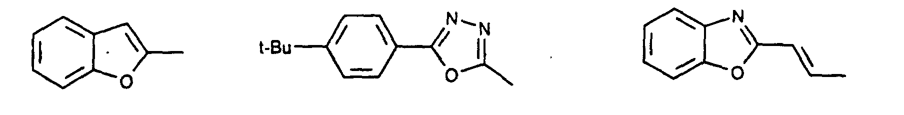 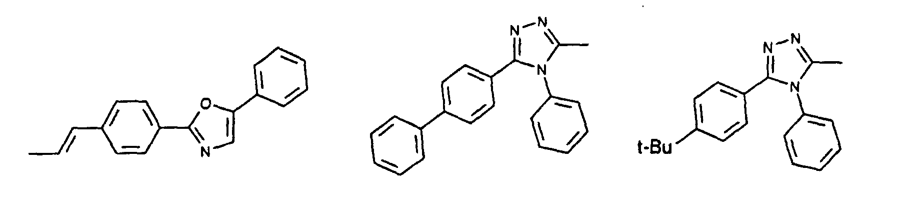 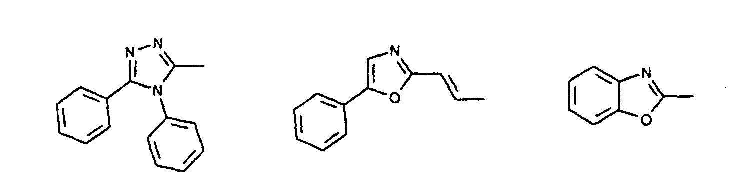 where the symbols and indices are as defined above;
IIc) K¹ = M and is selected from the group consisting of: 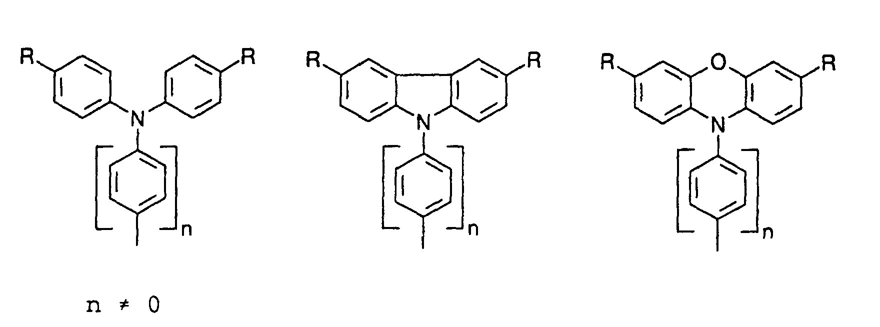 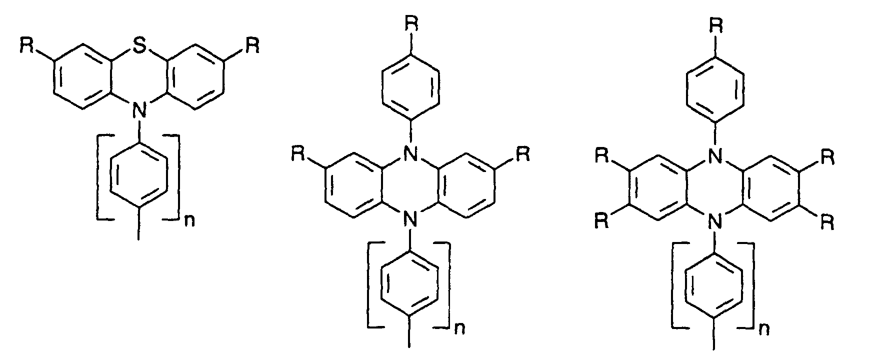 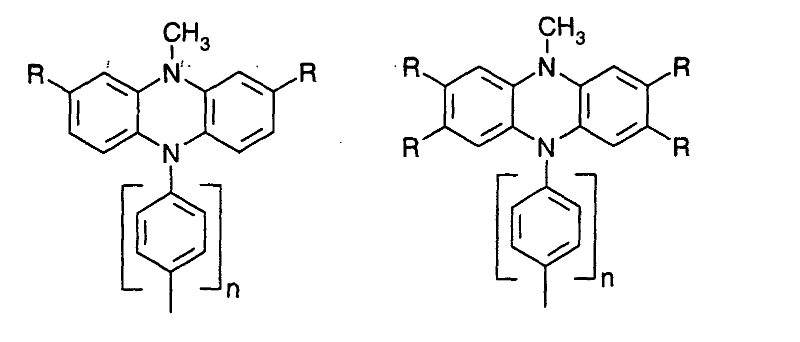 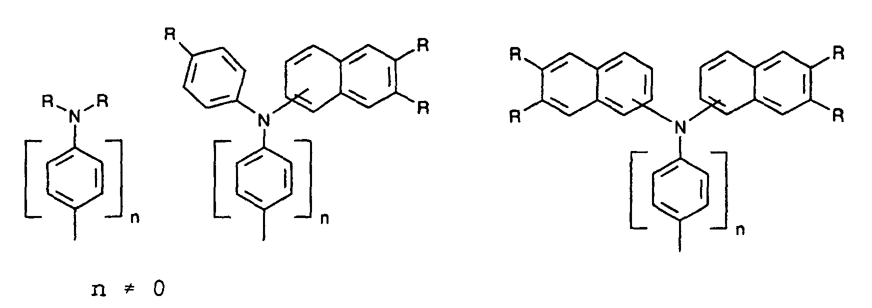 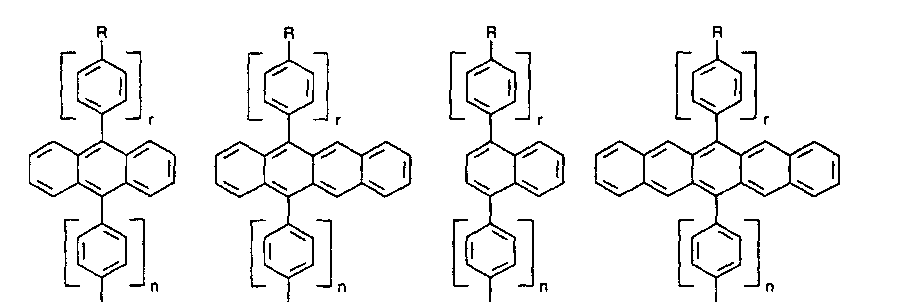 and M = N¹ and is selected from the group consisting of:
H, COOR, CH₂OR, 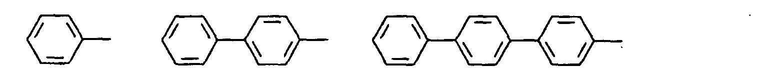 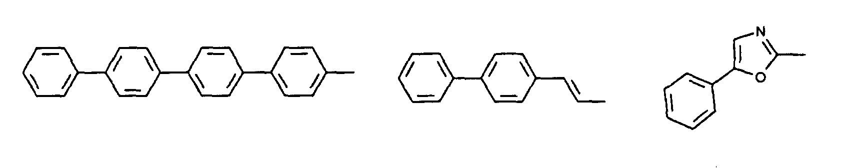 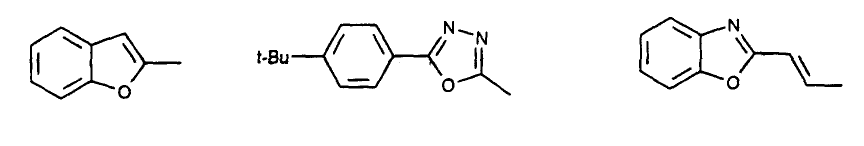 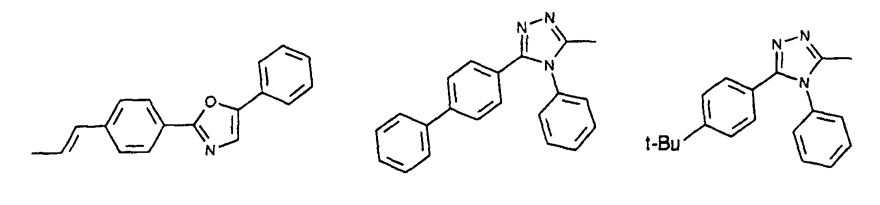 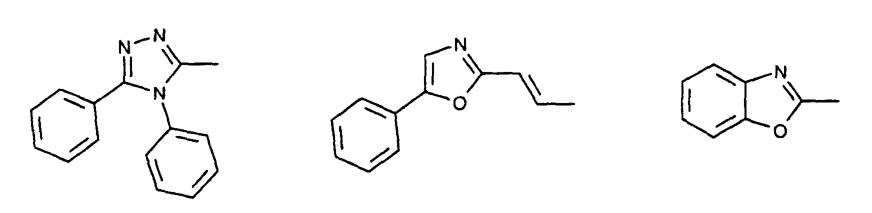 and Q, P¹ are identical or different and are selected from the group consisting of: 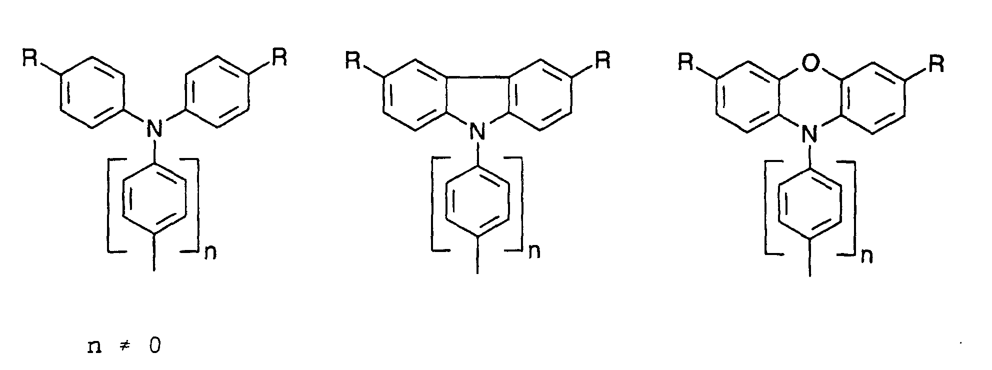 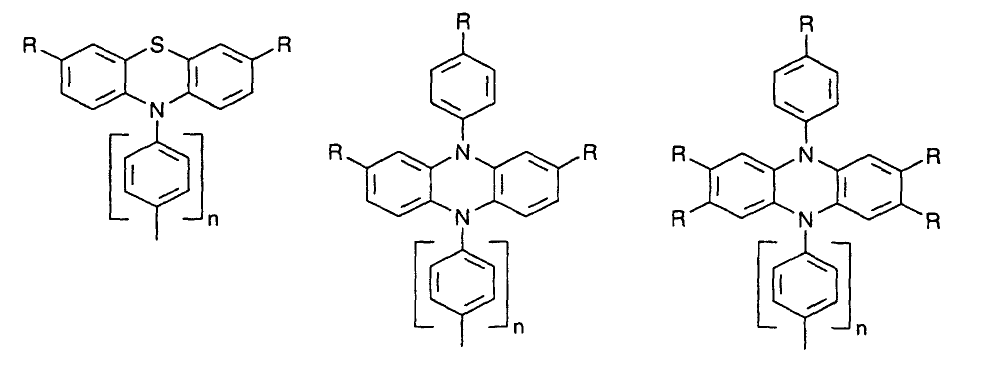 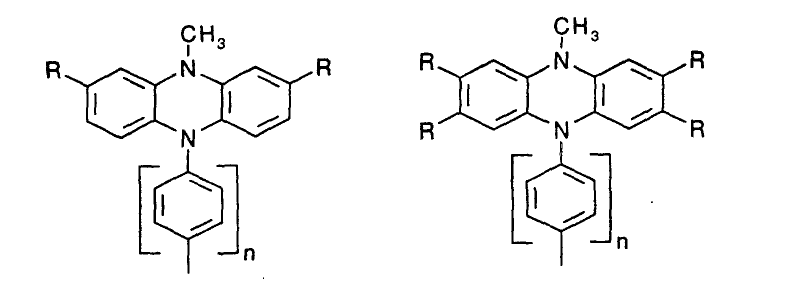 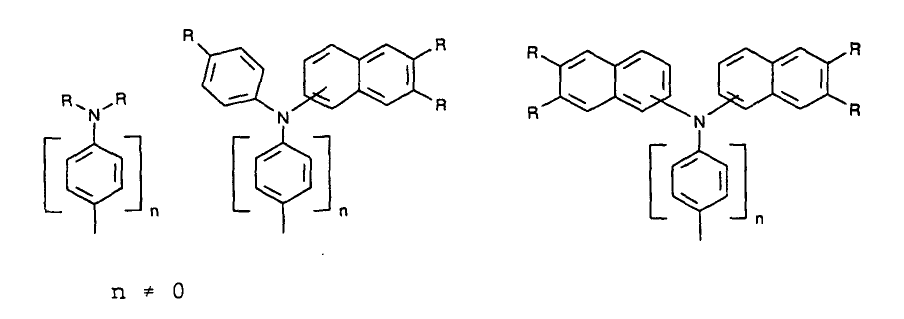 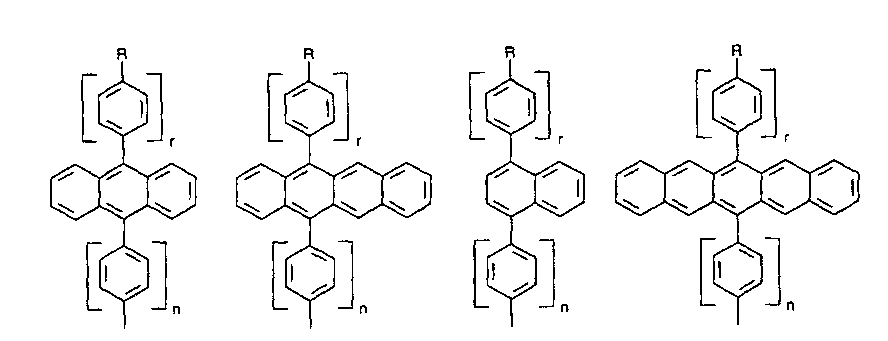 H, COOR, CH₂OR, 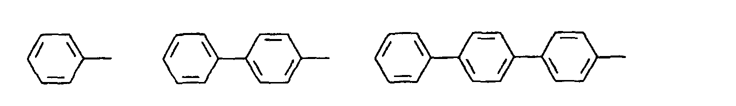 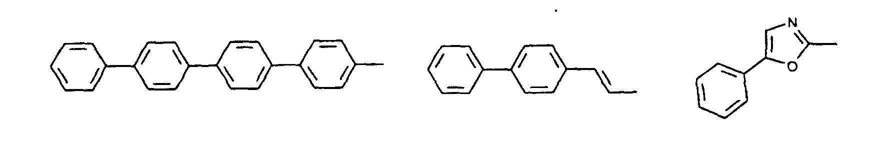 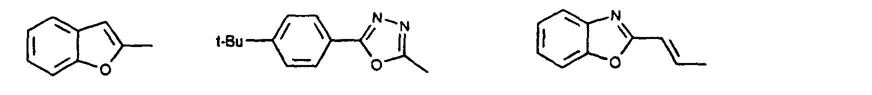 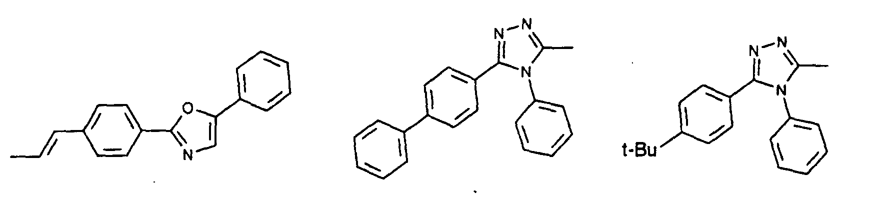 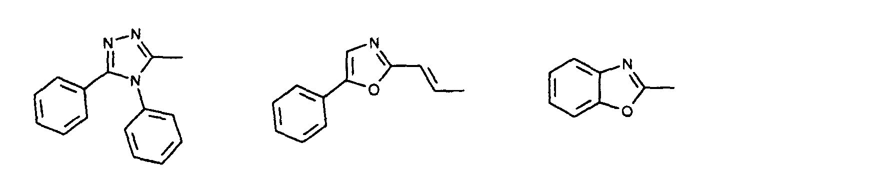 where the symbols and indices are as defined above.

3. A spiro compound as claimed in claim 1 and/or 2 selected from the group consisting of (II)aa-(II)ac:
IIaa) K¹ = L = M = N¹ and is selected from the group consisting of: 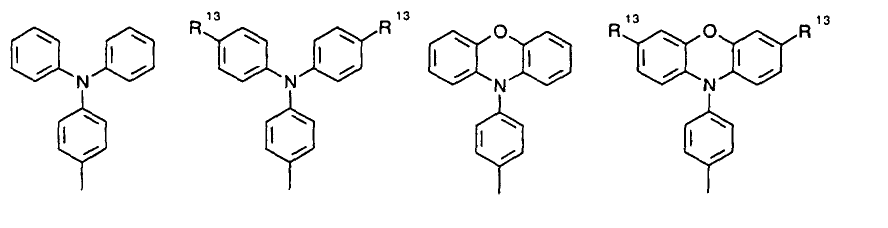 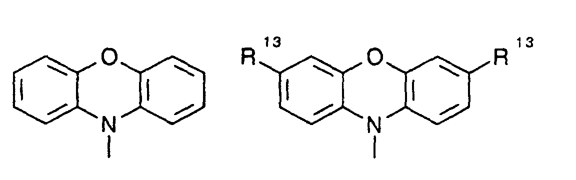 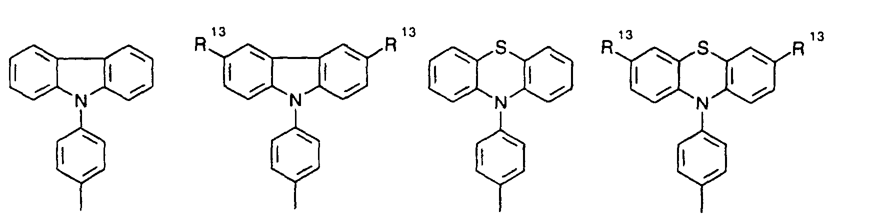 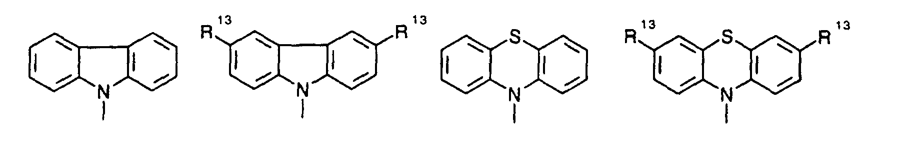 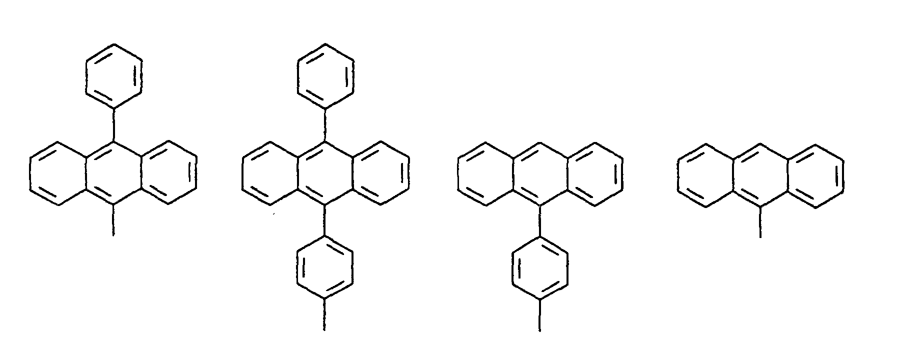 where R¹³ is -O-CH₃, -O-C₂H₅, -S-CH₃, -S-C₂H₅;
and Q = P¹ and is selected from the group consisting of:
H, COOR¹⁴, CH₂OR¹⁴, 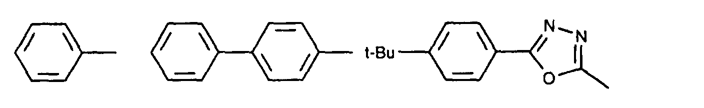 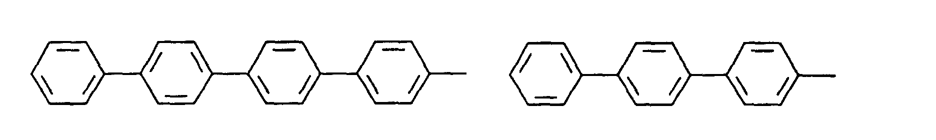 where R¹⁴ is a straight-chain or branched alkyl group having from 1 to 12 carbon atoms;
II.ba) K¹ = L = M = N¹ = Q = P¹ and is selected from the group consisting of: 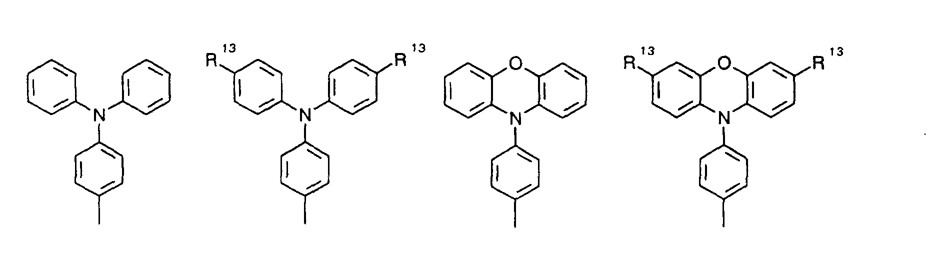 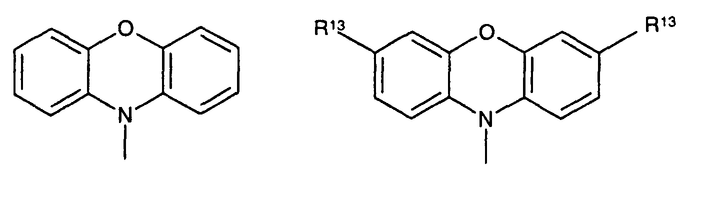 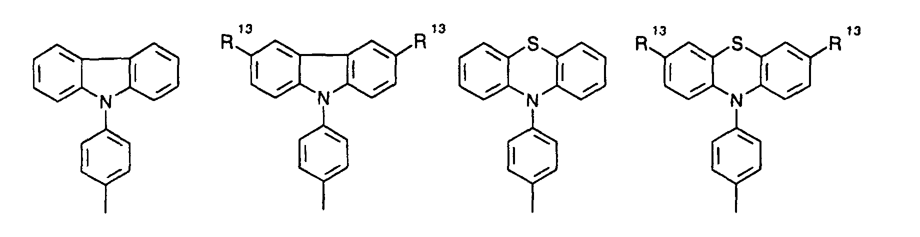 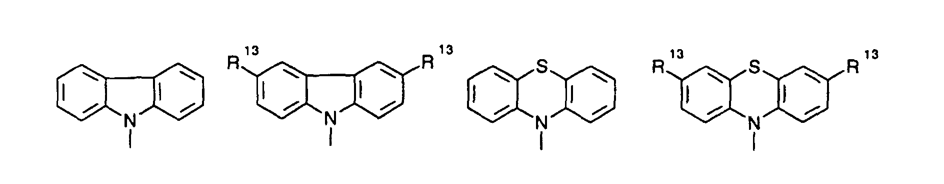 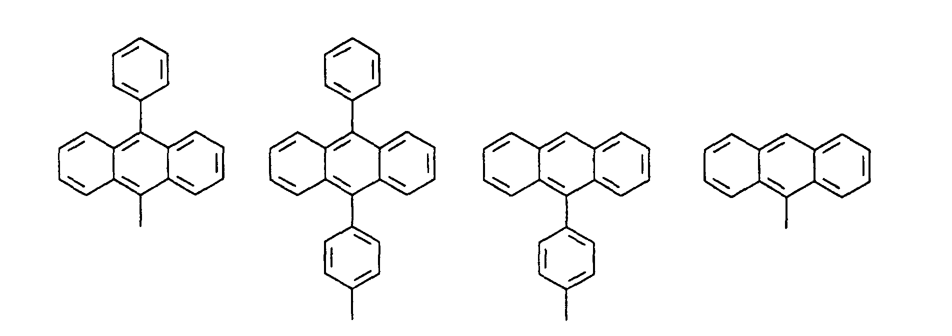 where R¹³ is as defined above;
II.ca) K¹ = L = M = N¹ and is selected from the group consisting of: 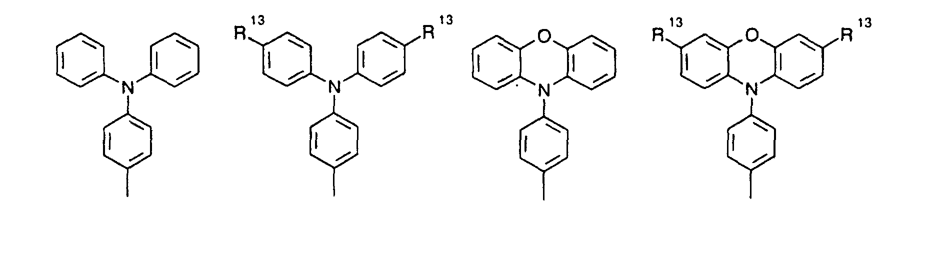 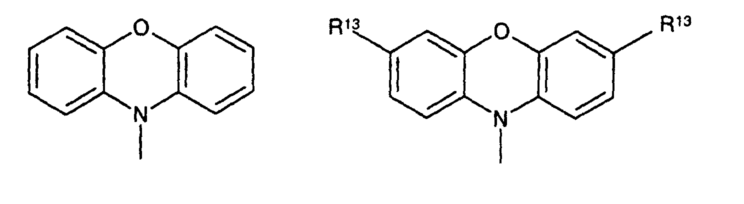 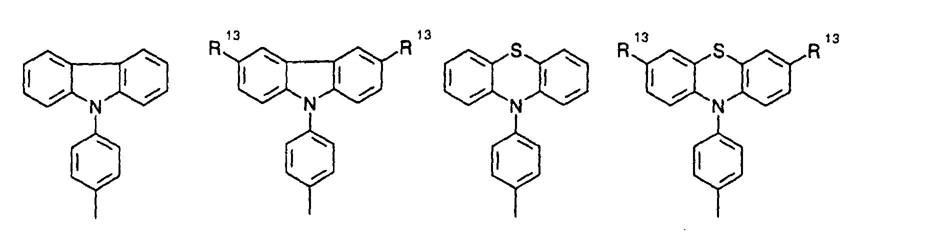 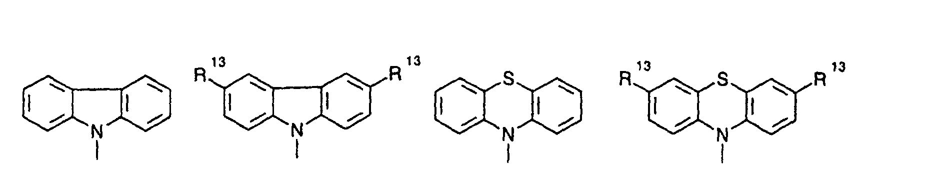 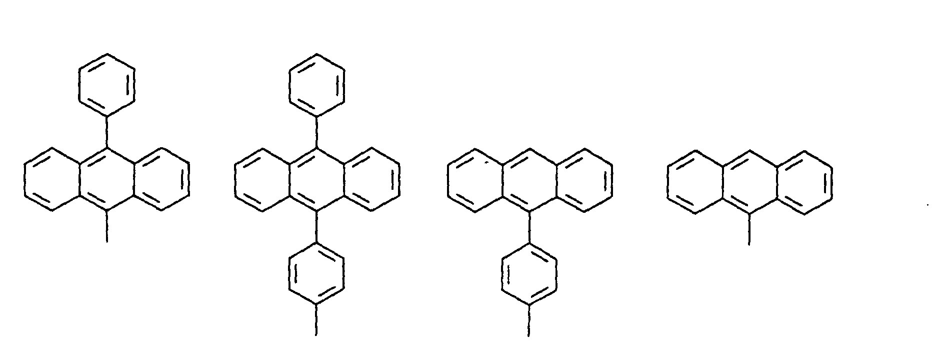 and Q = H and P¹ is selected from the group consisting of:
H, COOR¹⁴, CH₂OR¹⁴, 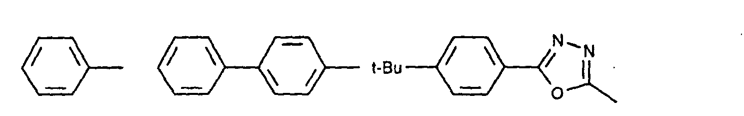 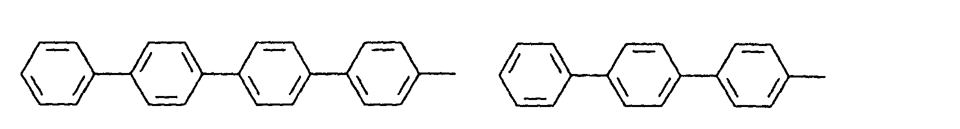 where R¹³, R¹⁴ are as defined above.

4. The use of an amorphous spiro compound as claimed in one or more of claims 1 to 3 as charge transport material in photovoltaic cells.

5. A photovoltaic cell comprising a charge transport layer which comprises one or more amorphous spiro compounds as claimed in one or more of claims 1 to 3.

## Revendications

1. Composé spirannique de formule (I) 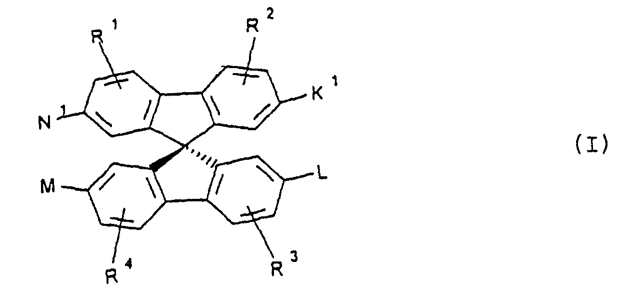 les symboles ayant les significations suivantes :
K¹, L, M, N¹, R¹, R², R³, R⁴ sont identiques ou différents et représentent
a) un atome d'hydrogène, des groupes -NO₂, -CN, -F ou -Cl,
b) un reste alkyle linéaire ou ramifié ayant 1 à 20 atomes de carbone, où
b1) un ou plusieurs groupes CH₂ non adjacents peuvent être remplacés par des groupes -O-, -S-, -CO-O-, -O-CO-, -O-CO-O-, NR⁵ ou -Si(CH₃)₂- et/ou
b2) un ou plusieurs groupes CH₂ peuvent être remplacés par des groupes -CH=CH-, -C≡C-, 1,4-phénylène, 1,4-cyclohexylène ou 1,3-cyclopentylène et/ou
b3) un ou plusieurs atomes de H peuvent être rempacés par F et/ou Cl et/ou
c) un des groupes suivants 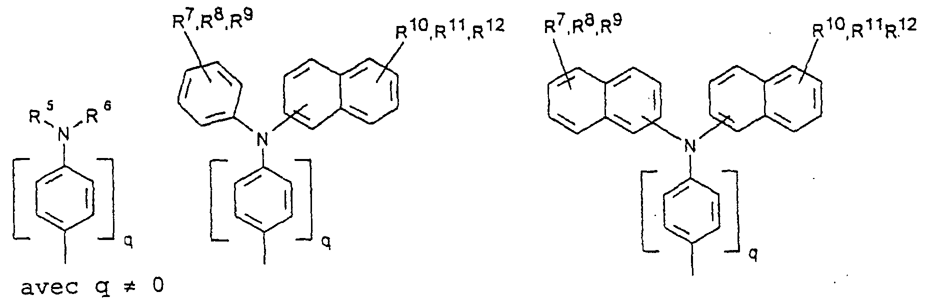 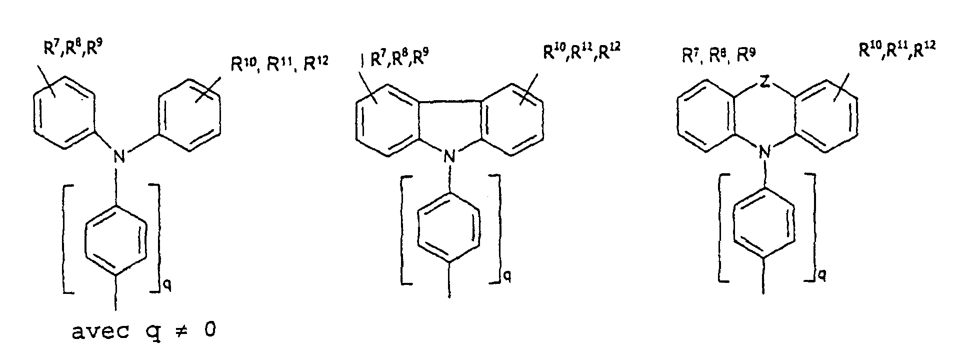 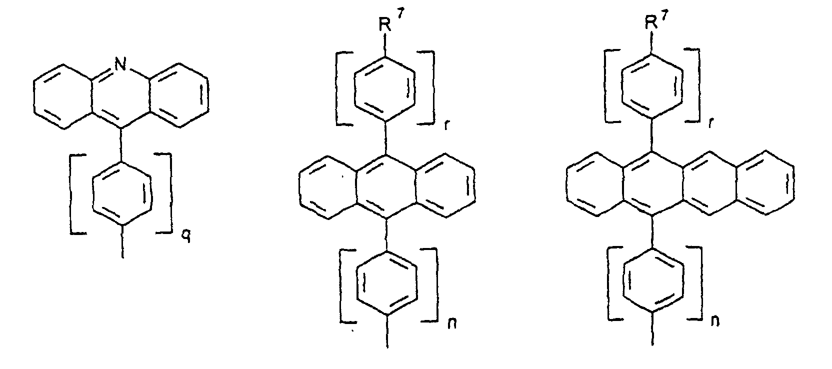 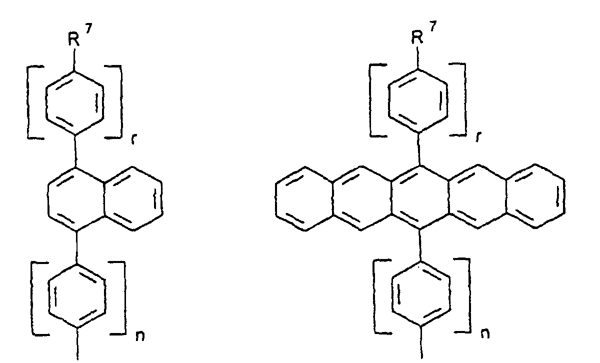 ou
d) un des groupes suivants 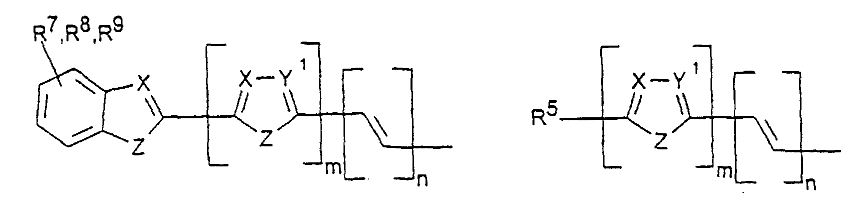 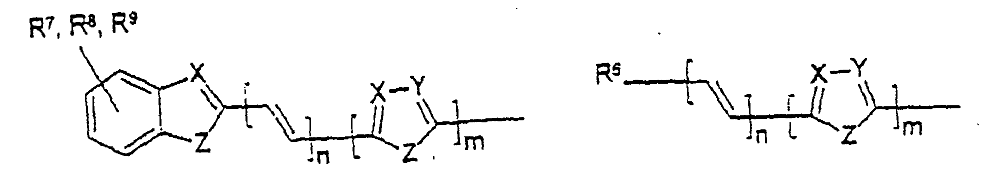 à condition qu'au moins l'un des restes K¹, L, M, N¹, R¹, R², R³, R⁴ représente un des groupes indiqués au point c) ;
X, Y¹ sont à chaque fois identiques ou différents et représentent des groupes = CR⁷ - ou = N- ;
Z représente des groupes -O-, -S-, -NR⁵-, -CRR, -CR=CR- ou -CR=N- ;
R⁵, R⁶ sont à chaque fois identiques ou différents et représentent
a) un atome d'hydrogène
b) un reste alkyle linéaire ou ramifié ayant 1 à 20 atomes de carbone,
b1) un ou plusieurs groupes CH₂ non adjacents et non liés à l'atome d'azote peuvent être remplacés par des groupes -O-, -S-, -CO-O-, -O-CO-, -O-CO-O- ou -Si(CH₃)₂ et/ou
b2) un ou plusieurs groupes CH₂ peuvent être remplacés par -CH=CH-,
-C≡C-, cyclopropane-1,2-diyle, 1,4-phénylène, 1,4-cyclohexylène ou 1,3-cyclopentylène et/ou
b3) un ou plusieurs atomes de H peuvent être remplacés par F et/ou Cl et/ou
b4) R⁵ et R⁶ ensemble peuvent former également un cycle ;
c) des grupes phényle, biphényle, 1-naphtyle, 2-naphtyle, 2-thiényle, 2-furanyle ;
R⁷, R⁸, R⁹, R¹⁰, R¹¹, R¹² sont identiques ou différents et représentent
a) un atome d'hydrogène, des groupes -CN, -F, -NO₂ ou -Cl,
b) un reste alkyle linéaire ou ramifié ayant 1 à 20 atomes de carbone, où
b1) un ou plusieurs groupes CH₂ non adjacents peuvent être remplacés par des groupes -O-, -S-, -CO-O-, -O-CO-, -O-CO-O-, NR⁵ ou -Si(CH₃)₂- et/ou
b2) un ou plusieurs groupes CH₂ peuvent être remplacés par des groupes -CH=CH-, -C≡C-, cyclopropane-1,2-diyle, 1,4-phénylène, 1,4-cyclo-hexylène ou 1,3-cyclopentylène et/ou
b3) un ou plusieurs atomes de H peuvent être remplacés par F et/ou Cl et/ou
c) des groupes phényle, biphényle, 1-naphtyle, 2-naphtyle, 2-thiényle, 2-furanyle, -O-phényle, -O-biphényle, -O-1-naphtyle, -O-2-naphtyle, -O-2-thiényle, -O-2-furanyle,
m, n, p, q, r sont à chaque fois identiques ou différents 0, 1, 2, 3, 4, 5 ou 6.

2. Composé spirannique selon la revendication 1, **caractérisé en ce qu'**il s'agit d'un dérivé de spirobifluorène de formules (II) a-c 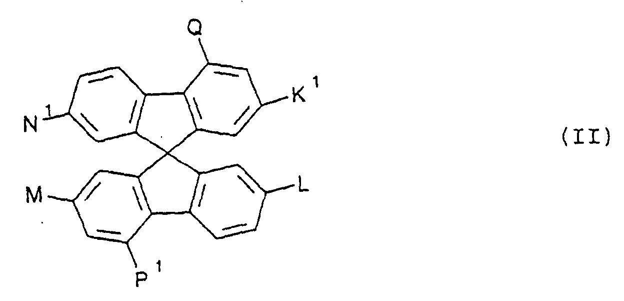 les symboles possèdent les significations suivantes :
II.a : K¹=L=M=N¹ et est pris parmi les groupes : 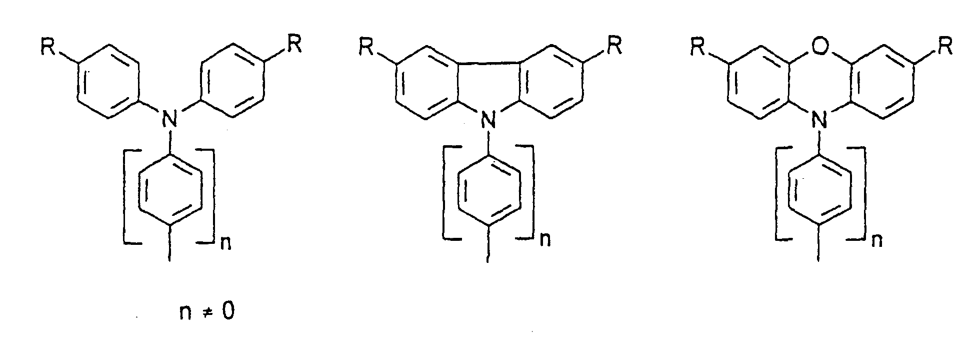 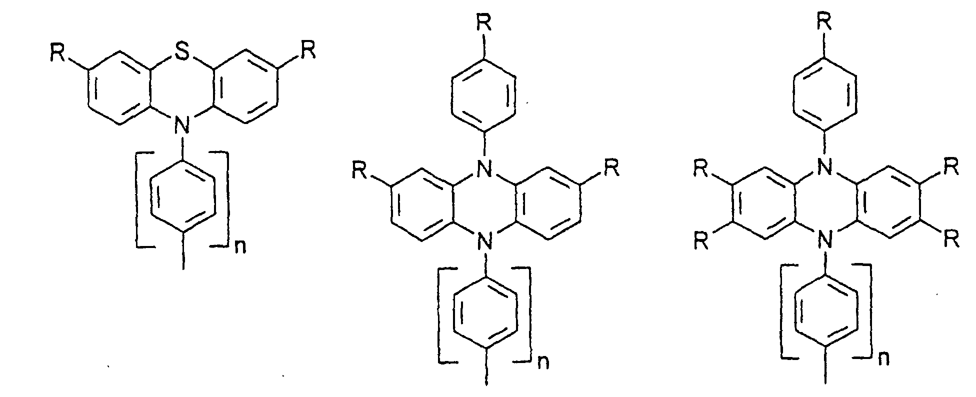 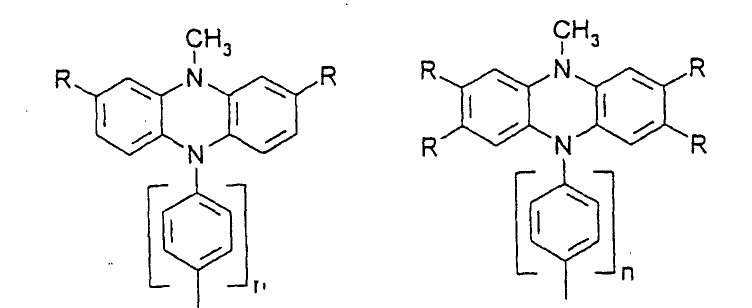 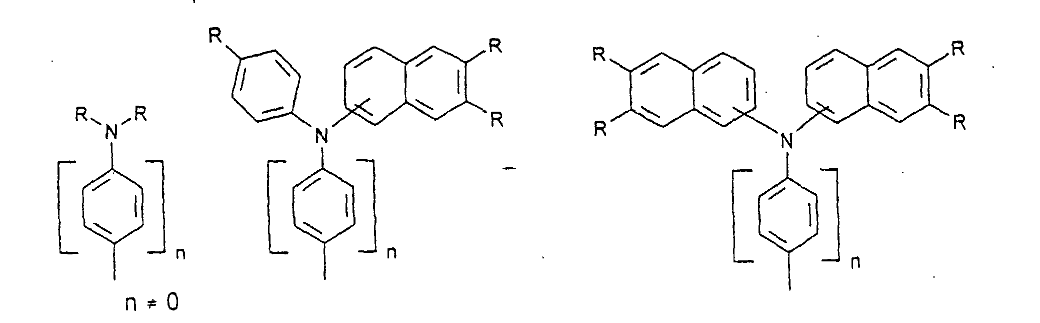 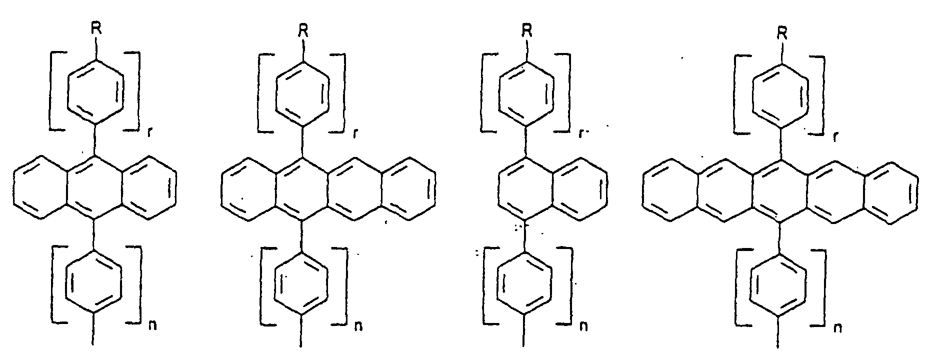
R sont identiques ou différents et représentent H, des groupes alkyle, -O-alkyle, -S-alkyle, chacun ayant 1 à 20 atomes de carbone, de préférence 1 à 4 atomes de carbone, phényle, biphényle, 1-naphtyle, 2-naphtyle,, 2-thiényle, 2-furanyle, -O-phényle, -O-biphényle, -O-1-naphtyle, -O-2-naphtyle, -O-2-thiényle, -O-2-furanyle, CN, NR², -O-alkyle/aryle, -S-alkyle/aryle, CN, NR² ne pouvant pas être liés à l'atome d'azote ;
n = 0, 1, 2, 3, 4 ;
et Q, P¹ sont identiques ou différents et sont pris parmi les groupes
H, COOR, CH₂OR, 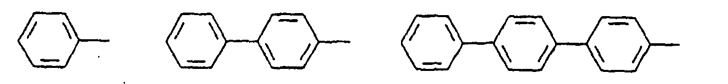 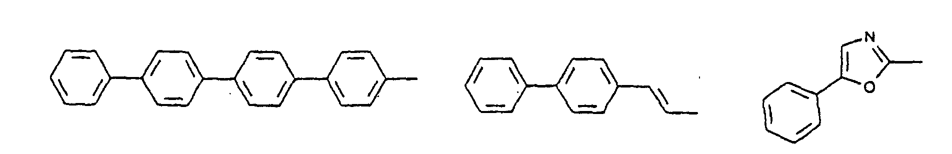 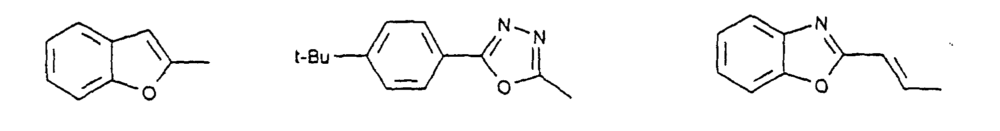 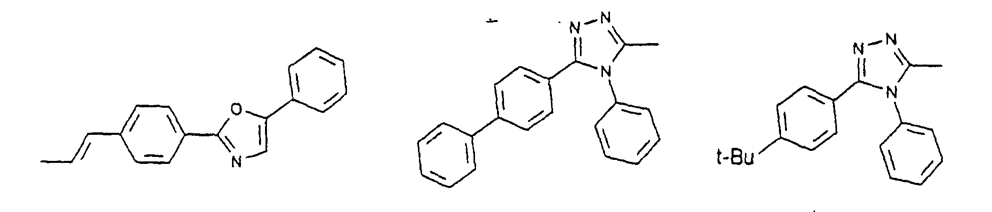 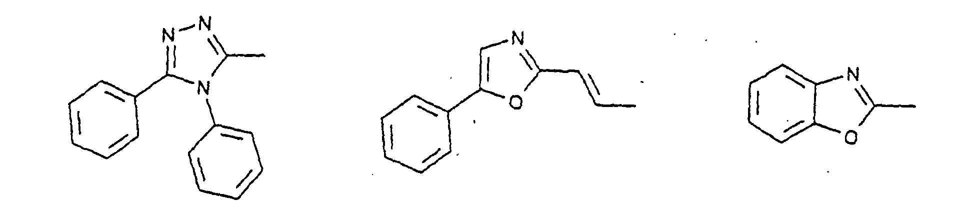 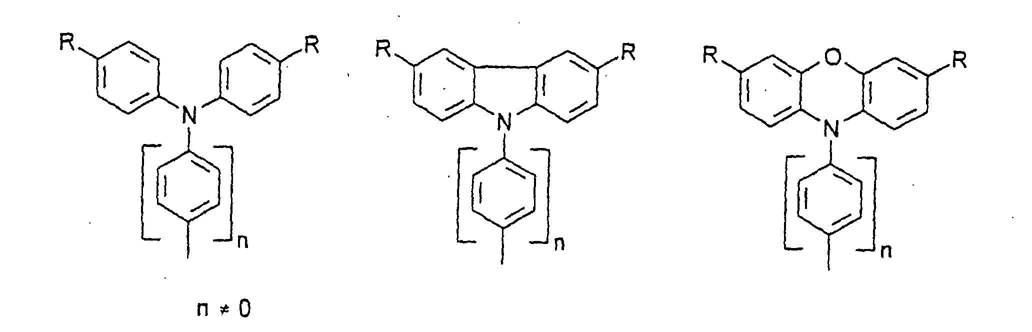 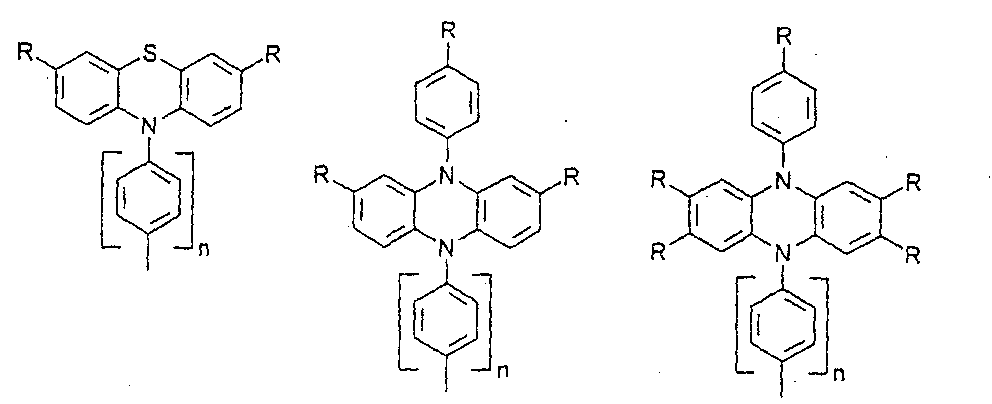 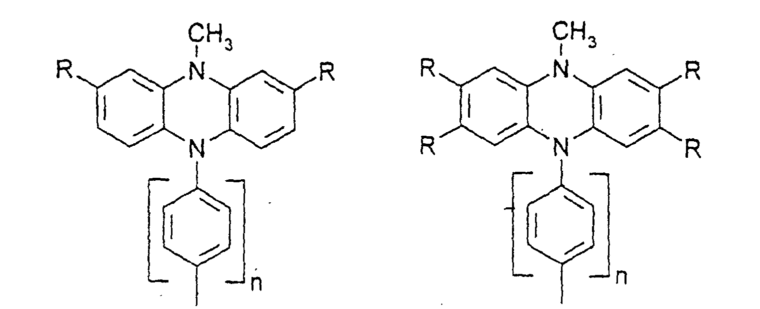 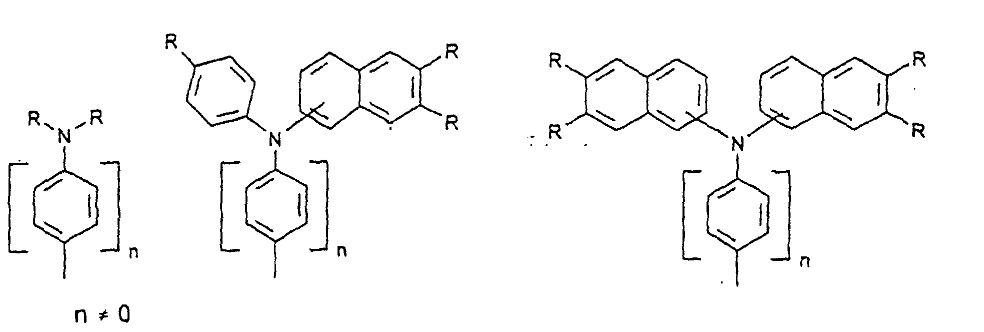 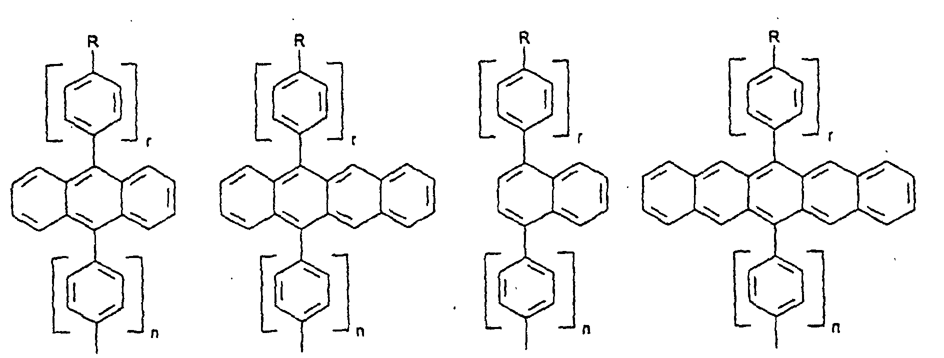 les symboles et les indices possédant les significations suivantes
II.b) K¹ =N¹ et est pris parmi les groupes 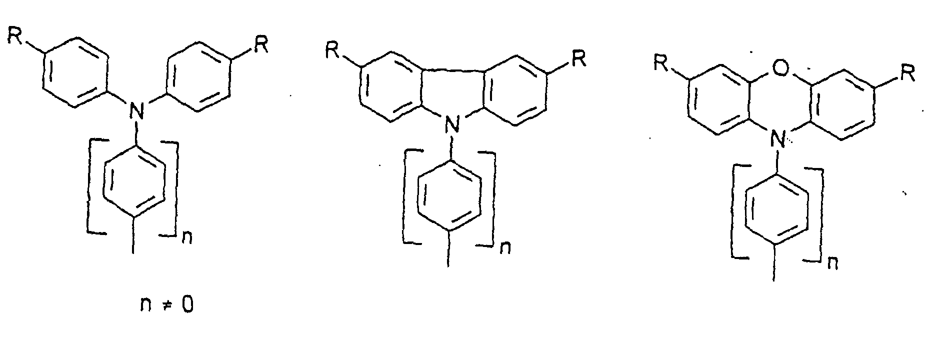 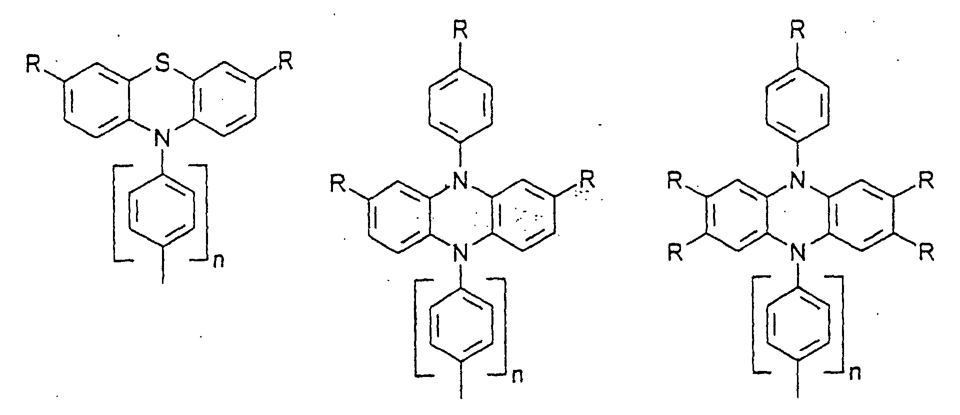 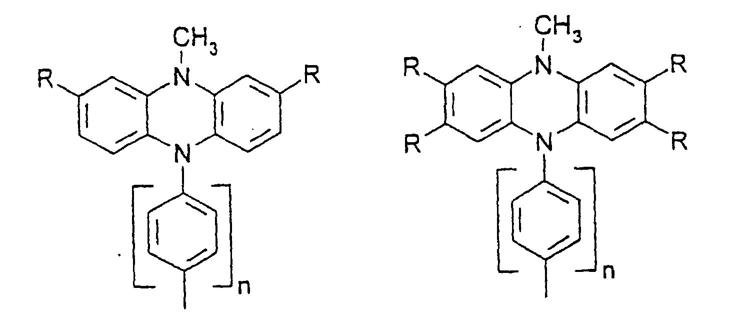 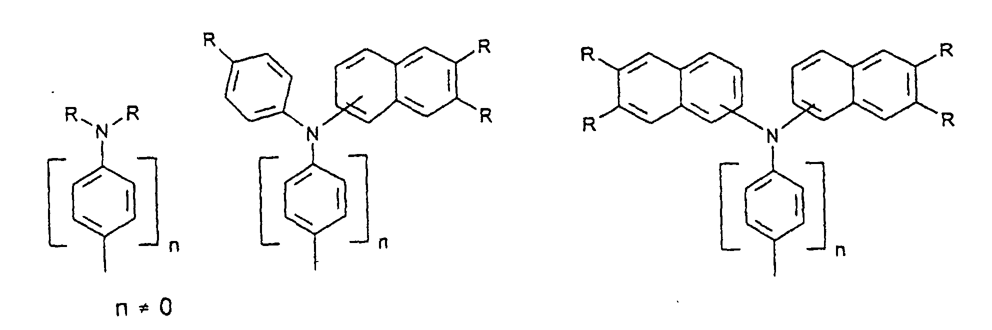 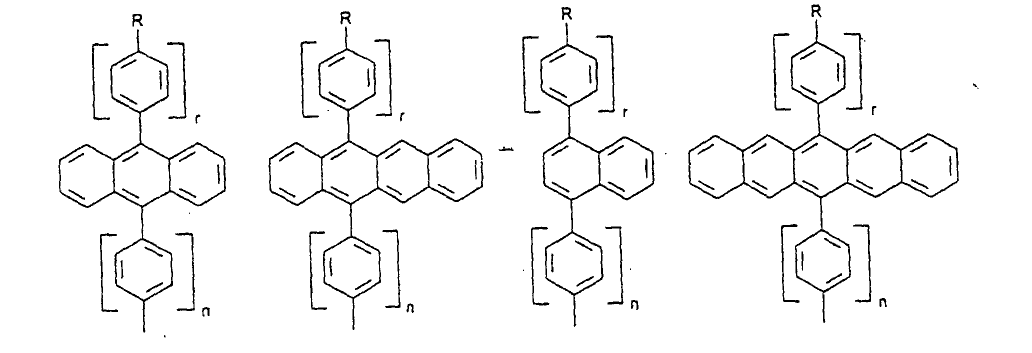 et L = M et est pris parmi les groupes
H, COOR, CH₂OR, 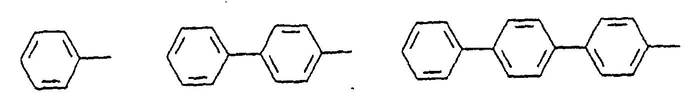 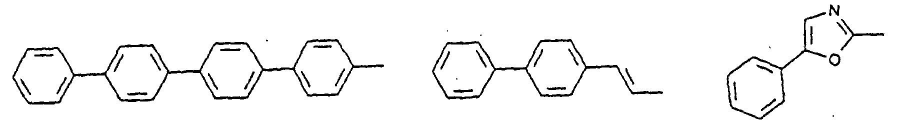 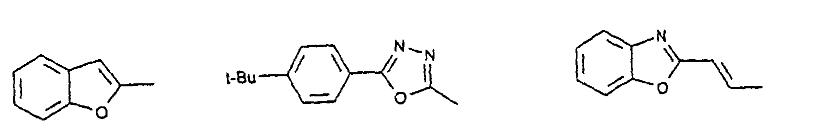 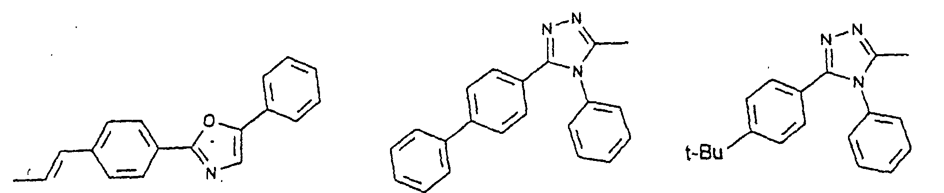 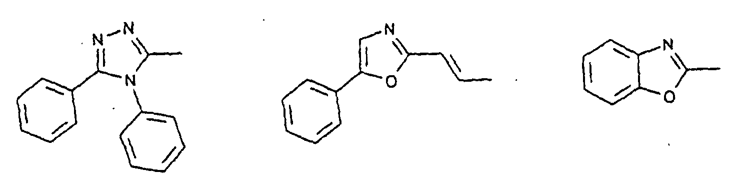 et Q, P¹ sont identiques ou différents et sont pris parmi les groupes 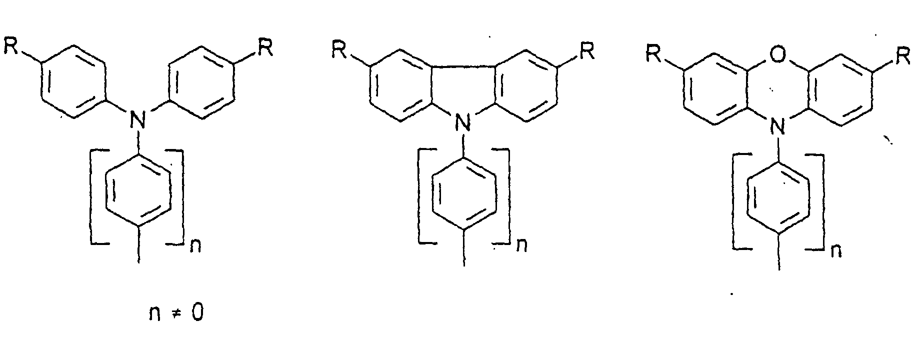 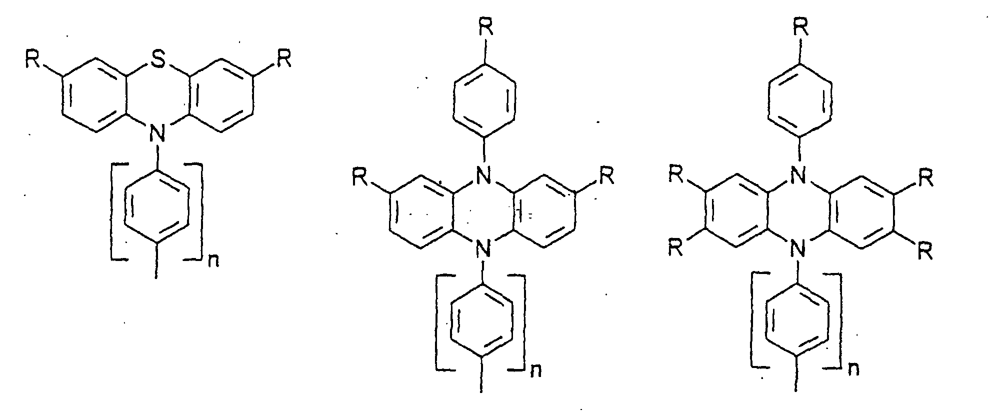 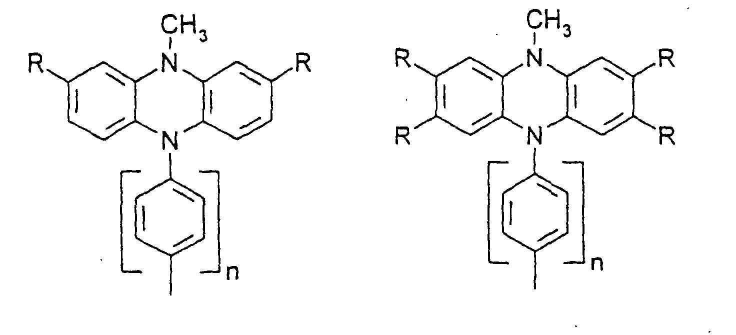 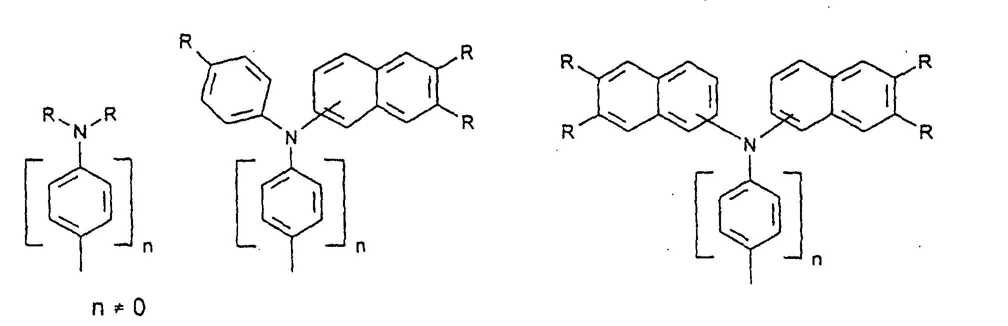 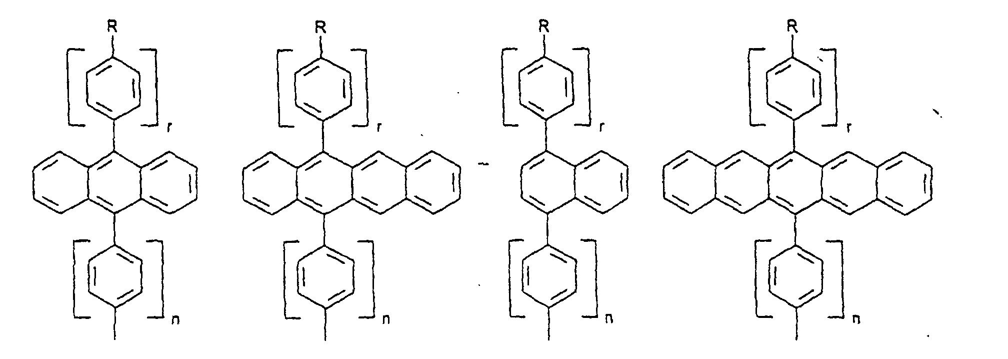 H, COOR, CH₂OR, 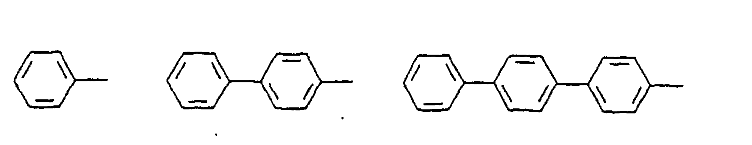 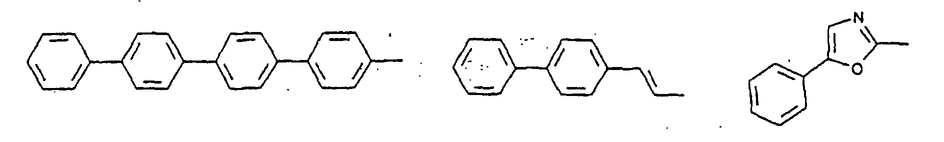 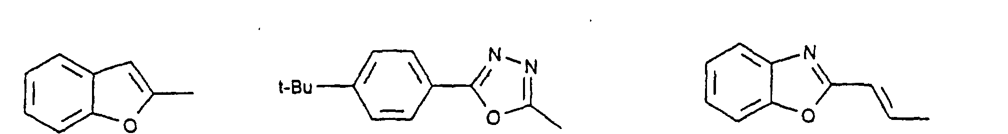 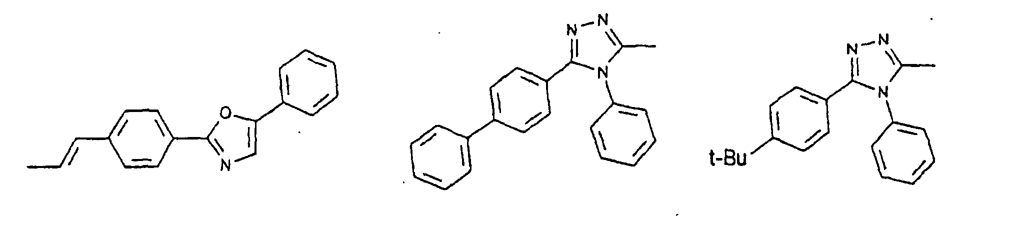 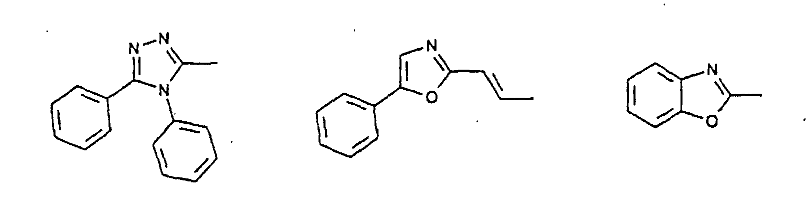 les symboles et les indices ayant les significations données ci-dessus.
II.c) K¹=M et est pris parmi les groupes 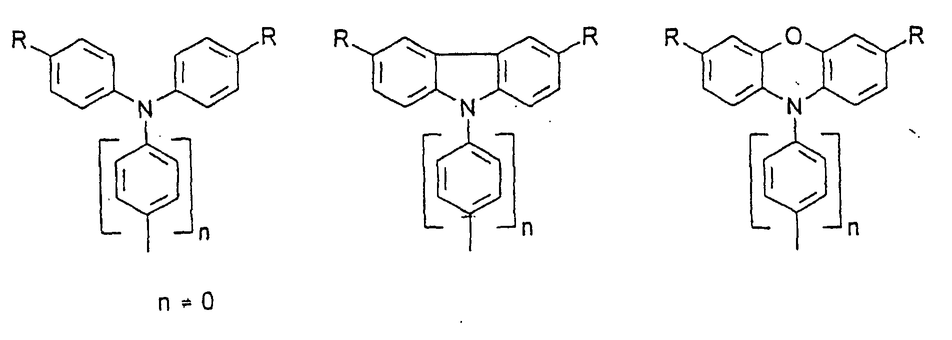 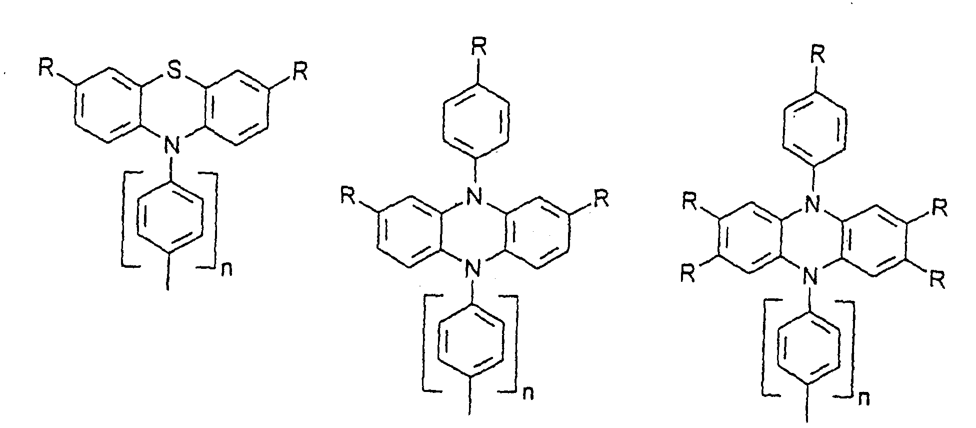 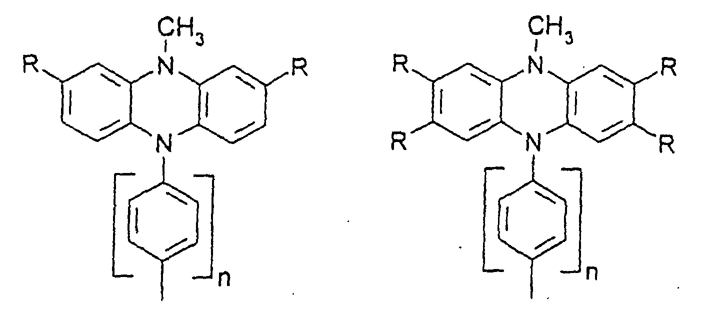 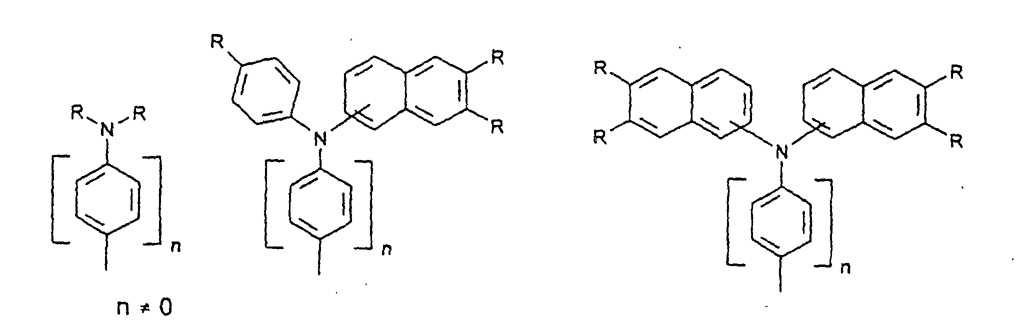 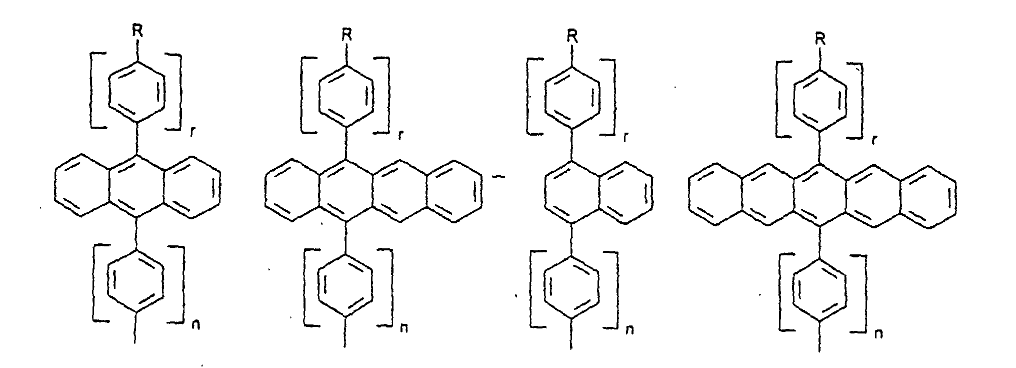 et M=N¹ et est pris parmi les groupes
H, COOR, CH₂OR, 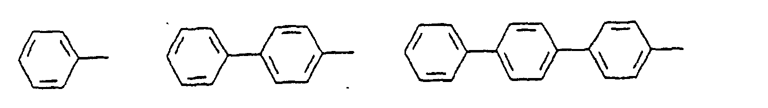 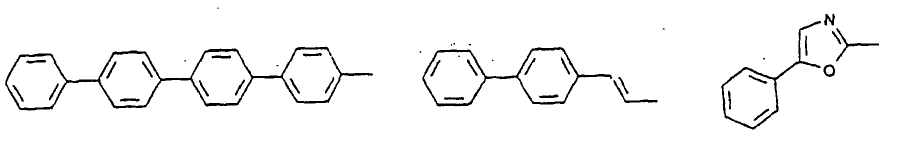 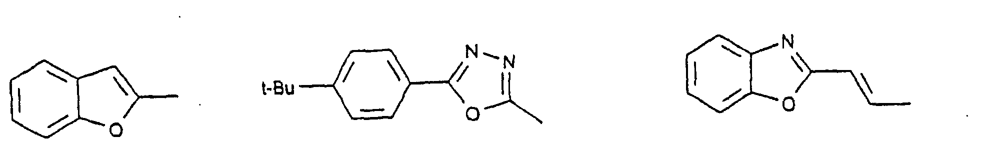 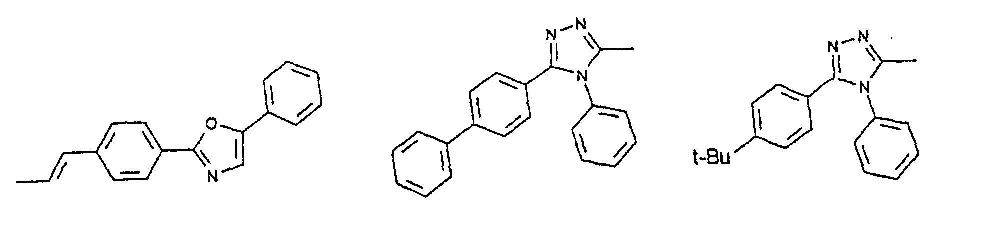 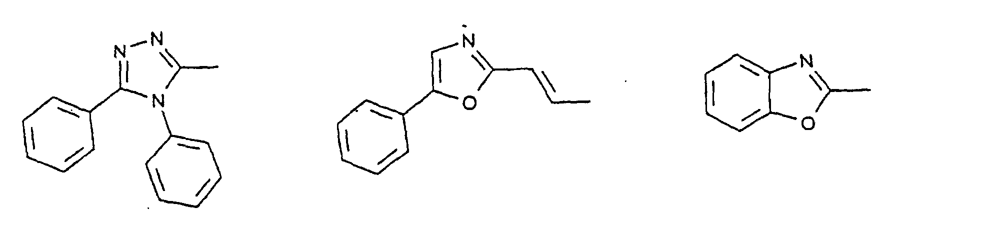 et Q, P¹ sont identiques ou différents et sont pris parmi les groupes 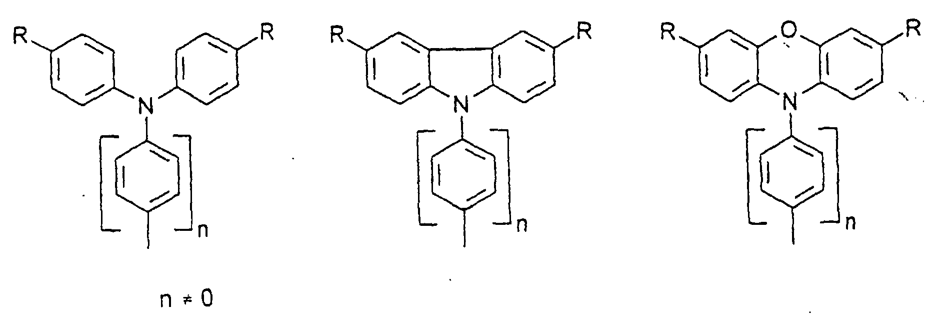 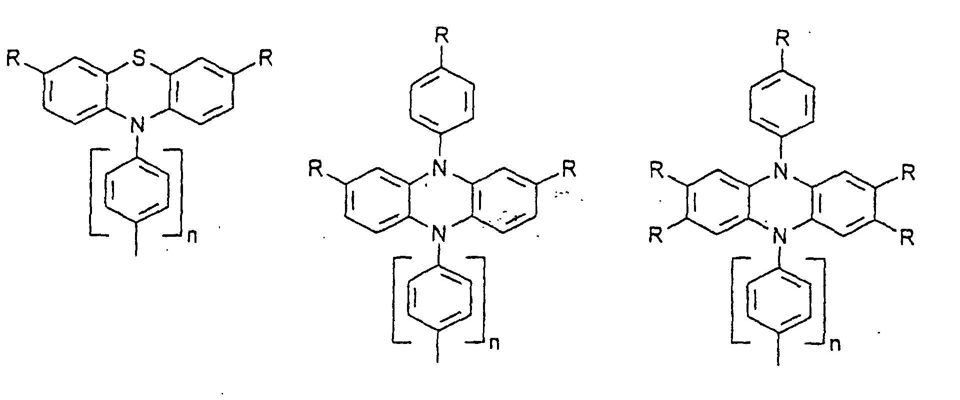 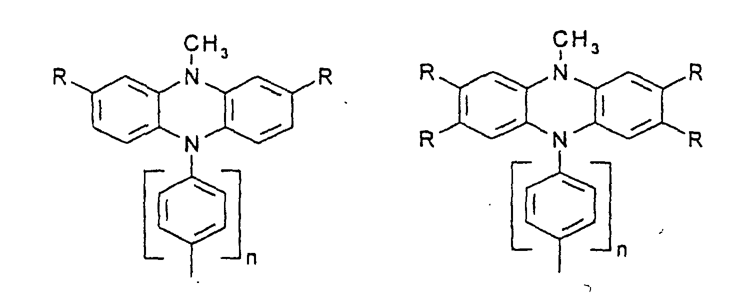 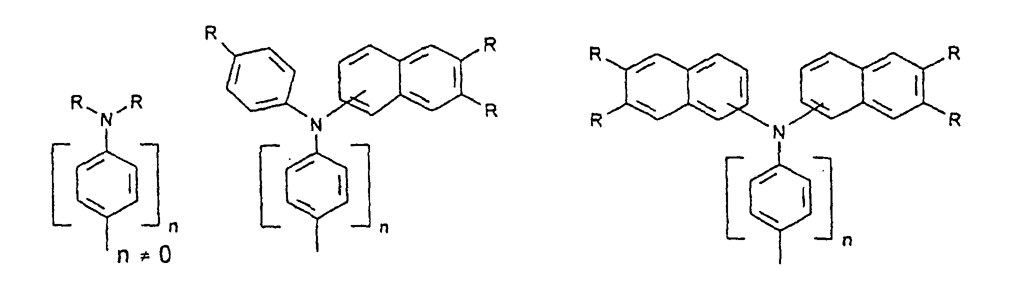 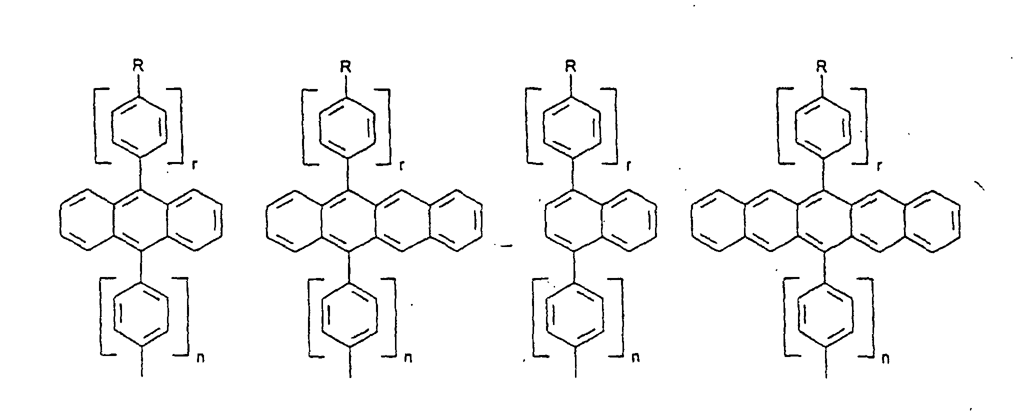 H, COOR, CH₂OR, 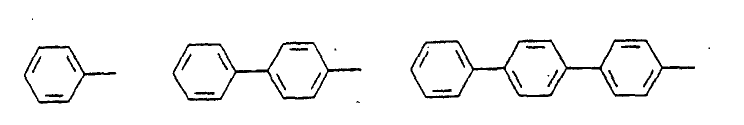 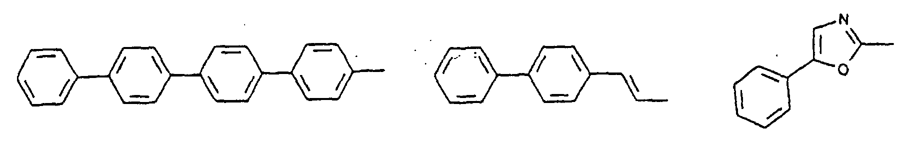 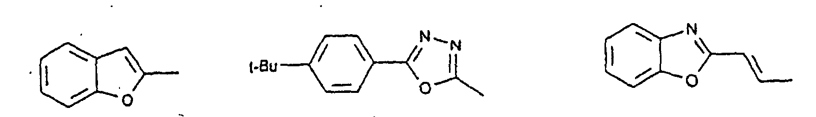 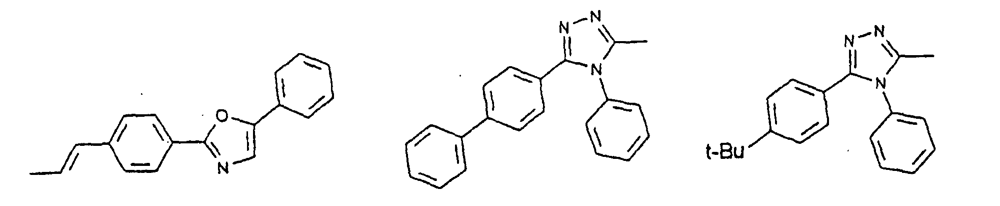 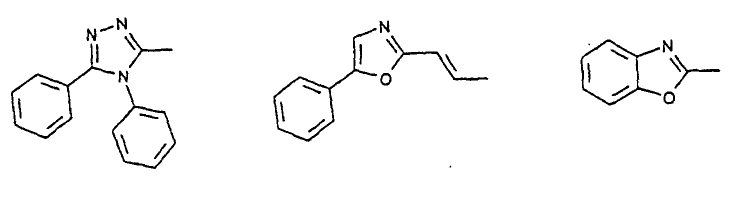 les symboles et les indices ayant les significations données ci-dessus.

3. Composé spirannique selon la revendication 1 et/ou 2 pris parmi les groupes (II)aa-(II)ac
IIaa) K¹=L=M=N¹ et est pris parmi les groupes 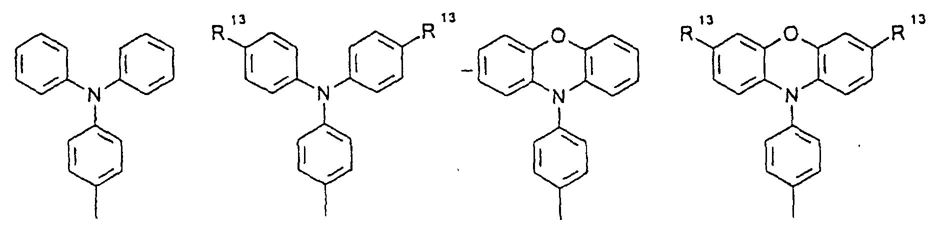 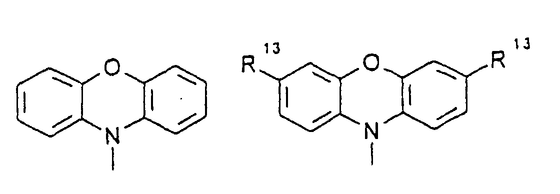 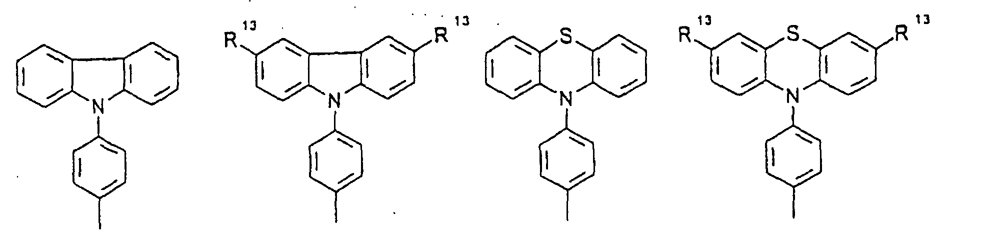 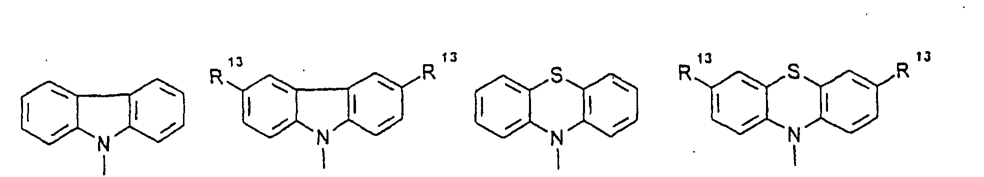 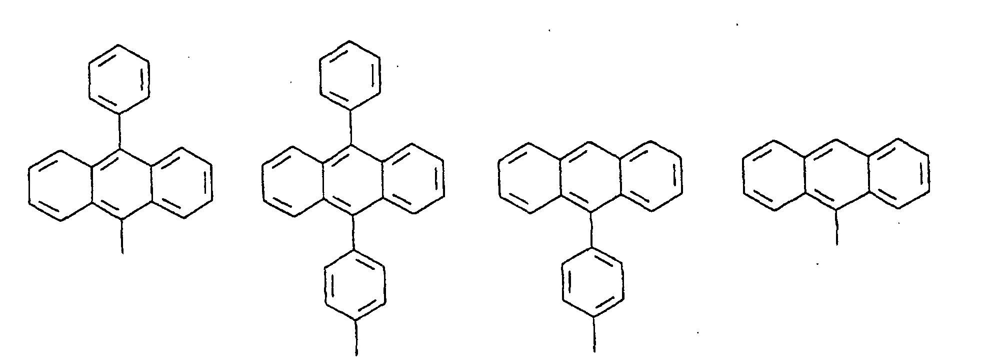 où R¹³ représente les groupes -O-CH₃, -O-C₂H₅, -S-CH₃, -S-C₂H₅ ;
et Q = P¹ et est pris parmi les groupes
H. COOR¹⁴, CH₂OR¹⁴, 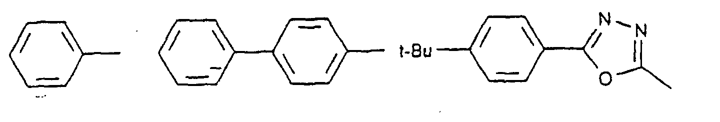 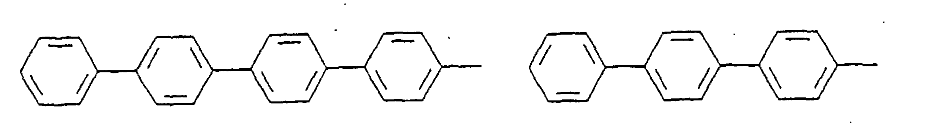 où R¹⁴ représente un groupe alkyle linéaire ou ramifié ayant 1 à 12 atomes de carbone ;
II.ba) K¹=L=M=N¹=Q=P¹ et est pris parmi les groupes 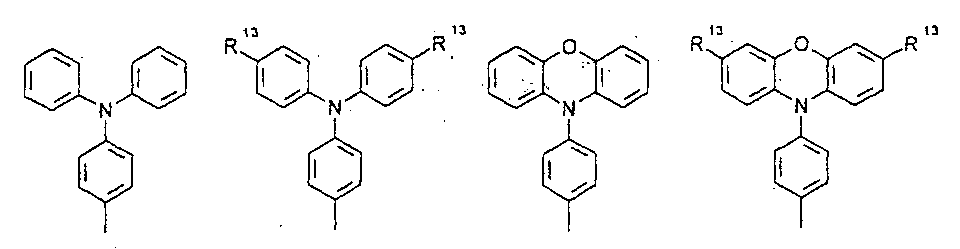 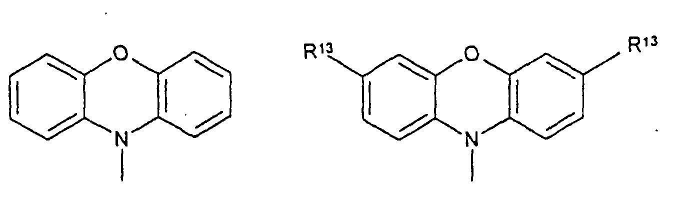 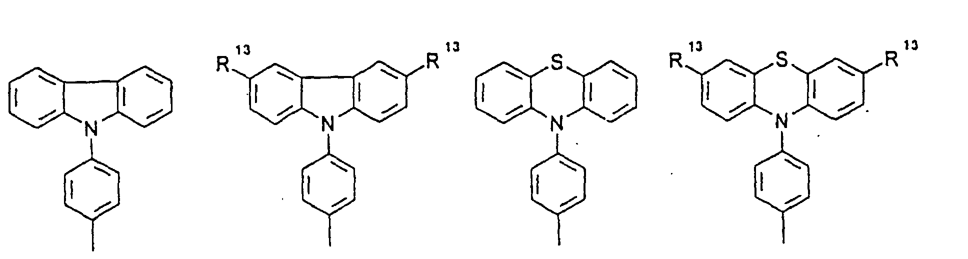 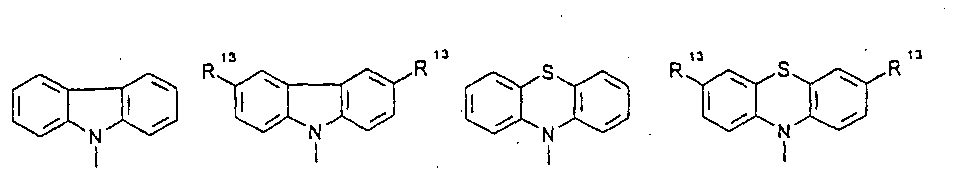 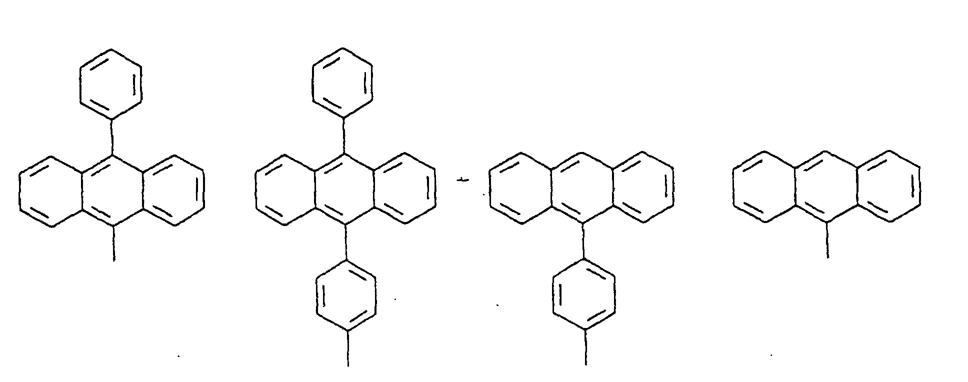 où R¹³ possède les significations données ci-dessus ; II.ca) K¹=L=M=N¹ et pris parmi les groupes 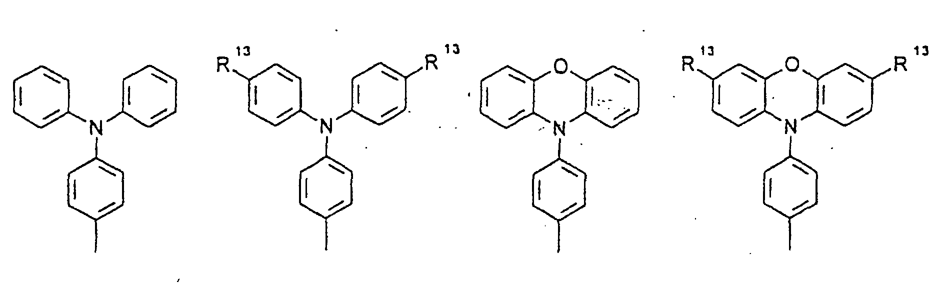 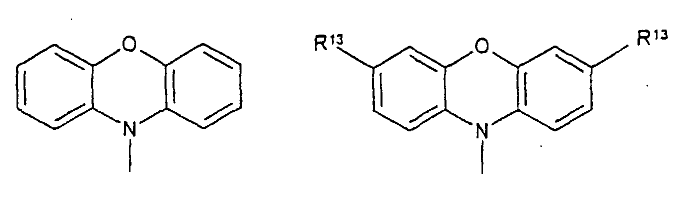 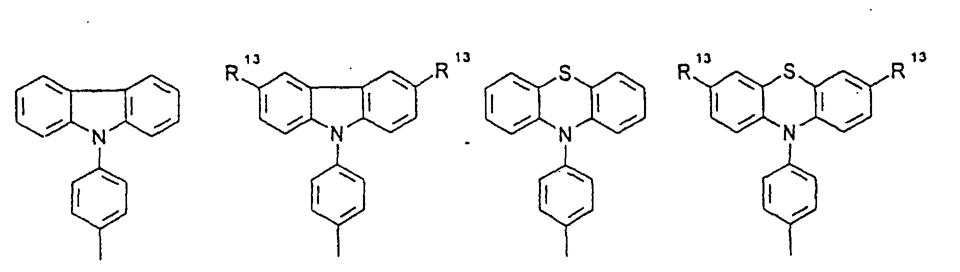 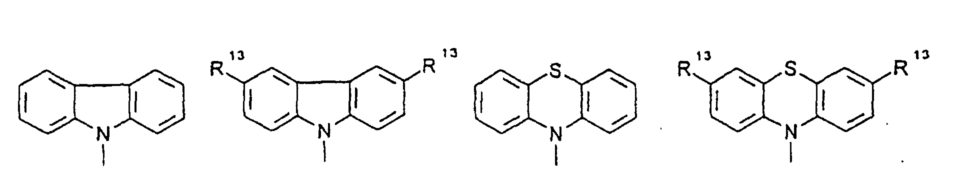 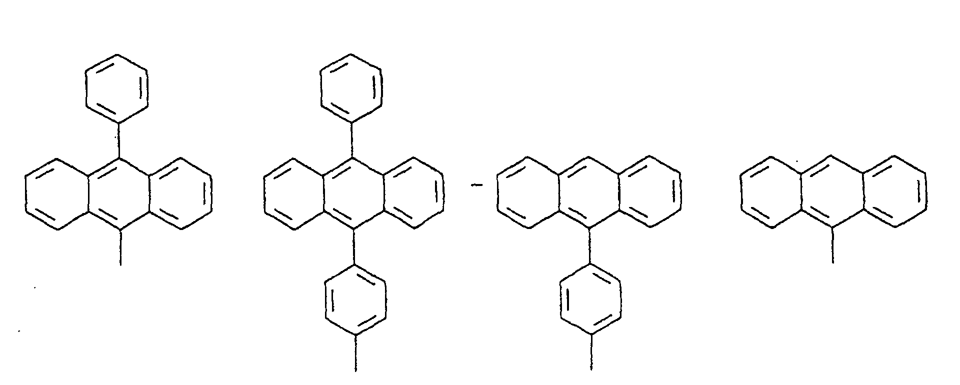 et Q=H et P¹ est pris parmi les groupes
H, COOR¹⁴, CH₂OR¹⁴, 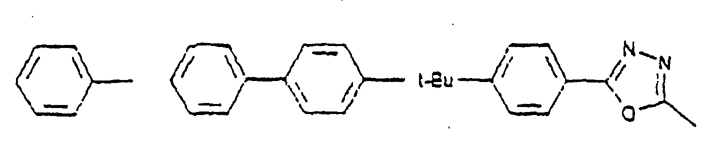 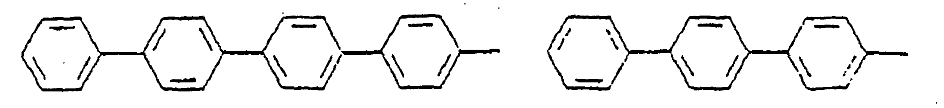 où R¹³, R¹⁴ possèdent les significations données ci-dessus.

4. Utilisation d'un composé spiro amorphe selon une ou plusieurs des revendications 1 à 3 en tant que matière de transport de charge dans des cellules photovoltaïques.

5. Cellule photovoltaïque contenant une couche de transport de charge, qui contient un ou plusieurs composés spiro amorphes selon une ou plusieurs des revendications 1 à 3.
